(19) **Europäisches Patentamt**
**European Patent Office**
**Office européen des brevets**

(11) **EP 4 257 607 A1**

(12) **EUROPEAN PATENT APPLICATION**
published in accordance with Art. 153(4) EPC

(43) Date of publication:
**11.10.2023 Bulletin 2023/41**

(21) Application number: **21900071.8**

(22) Date of filing: **02.12.2021**

(51) International Patent Classification (IPC):
**C07K 16/28** (2006.01)        **A61K 39/395** (2006.01)
**A61P 35/02** (2006.01)        **A61P 37/00** (2006.01)

(52) Cooperative Patent Classification (CPC):
**A61K 39/395; A61P 35/00; A61P 35/02;**
**A61P 37/00; C07K 16/00; C07K 16/28; C07K 16/46**

(86) International application number:
**PCT/CN2021/135121**

(87) International publication number:
**WO 2022/117045 (09.06.2022 Gazette 2022/23)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **02.12.2020  CN 202011402180**

(71) Applicant: **Keymed Biosciences Co.,Ltd.**
**Chengdu, Sichuan 610219 (CN)**

(72) Inventors:
• **CHEN, Bo**
**Chengdu, Sichuan 610219 (CN)**

• **XU, Gang**
**Chengdu, Sichuan 610219 (CN)**
• **WANG, Ying**
**Chengdu, Sichuan 610219 (CN)**

(74) Representative: **Elzaburu S.L.P.**
**Edificio Torre de Cristal**
**Paseo de la Castellana 259 C, planta 28**
**28046 Madrid (ES)**

Remarks:
The complete document including Reference Table(s) and the Sequence Listing(s) can be downloaded from the EPO website

(54) **DEVELOPMENT AND APPLICATION OF T-CELL ENGAGER THERAPEUTIC AGENT**

(57) The present disclosure relates to the development and application of a T-cell engager therapeutic agent. A T-cell engager is a T-cell bispecific antibody, and the bispecific antibody contains a first binding domain bound to BCMA on the surface of a target cell and a second binding domain bound to CD3 on the surface of a T cell. The bispecific antibody has good tolerance, can effectively block the binding of the BCMA to a receptor APRIL, and has no adverse reaction, such as weight loss.

FIG. 1

Processed by Luminess, 75001 PARIS (FR)

## Description

## Technical Field

The present disclosure relates to the development and application of a T-cell engager therapeutic agent, particularly a bispecific antibody bound to BCMA and CD3, and applications thereof.

## Background Art

B-cell maturation antigen (BCMA), as a member of the tumor necrosis factor receptor superfamily, is widely expressed on the surface of myeloma cells, only expressed on plasma cells in normal tissues, but not on naive B cells, CD34+ hematopoietic stem cells, and other normal tissues. BCMA-deficient mice have normal B cell development and humoral immune response. In multiple myeloma patient samples, after the ligand APRIL (A Proliferation Inducing Ligand) binds to BCMA, it may stimulate myeloma cell growth and proliferation, and prevent tumor cell apoptosis simultaneously. Since the expression of BCMA in malignant plasma cells is significantly higher than that in normal plasma cells, BCMA has become an important target for immunotherapy.

In hematological malignancies, multiple myeloma is the second most common disease, frequently occurring in the elderly. Accumulation of malignantly proliferating plasma cells in the patient's bone marrow may lead to bone marrow failure and loss of immune function, and is often accompanied by multiple osteolytic lesions, hypercalcemia, anemia, and renal damage simultaneously. Although new drugs, including proteasome inhibitors, immunomodulators, and targeted antibodies, have significantly prolonged patient survival in recent years, nearly all patients relapse. New therapies that are being developed such as CAR-T cells (chimeric antigen receptor T-cells) have a complicated preparation process, high treatment cost, and high recurrence rate; antibody drug conjugates (ADC) are limited to low doses due to the safety of conjugated toxins; although BCMA bispecific antibodies previously developed are clinically shown to be quite effective, increased doses are accompanied with increased side effects, which may lead to a strong cytokine storm syndrome and endanger patient life. Thus, there remains a need in the art to develop new, safer, and more effective drugs.

A T-cell bispecific antibody (or referred to as T-cell engager) is a specially constructed antibody molecule that recognizes a target cell surface antigen (antigen arm) at one end and binds to a T-cell CD3 receptor (CD3 arm) at the other end. Using antibodies that target the $\varepsilon$ chain of CD3, the CD3 on T cells is aggregated in a manner like TCR/peptide/HLA, thereby activating T cells and killing tumors. The BCMA×CD3 bispecific molecule is a T-cell TCB antibody that targets BCMA expressed on myeloma cells and the CD3$\varepsilon$ chain (CD3e) present on T cells. The action mechanism of the BCMA×CD3 bispecific molecule involves binding to both BCMA+ myeloma cells and CD3+ T cells, resulting in T cell activation and T cell-mediated cell killing.

During a normal immune response, TCR binds with low affinity (about 1-100 $\mu$M) to an exogenous peptide-human leukocyte antigen complex (HLA) on infected or mutated cells, transduces an activation signal into the nucleus via CD3, activates the expression of transcription factors and their downstream proteins (cytokines, granzymes, perforin, etc.), wherein the signal intensity produced by the TCR complex will determine the fate of T cells. The $\varepsilon$, $\gamma$, $\delta$, and $\zeta$ subunits of the signaling complex associate with each other to form CD3$\varepsilon$-$\gamma$ heterodimers, CD3$\varepsilon$-$\delta$ heterodimers, and CD3$\zeta$-$\zeta$ homodimers. The early developed CD3 bispecific antibodies were mostly based on a few murine antibodies such as OKT3, L2K, UCHT1, and TR66, and have high affinity. In clinical studies, it was found that excessively strong affinity of CD3 antibody would lead to excessive activation of T cells and release of a large number of cytokines, forming cytokine storm syndrome; at the same time, high affinity also leads to the enrichment of bispecific antibodies in secondary lymphoid organs, reducing the exposure to tumor tissue. The binding ability of the Fc portion of the antibody to the Fc$\gamma$ receptor is another important factor affecting drug safety. Since the Fc$\gamma$ receptor is expressed in various normal tissues, after bispecific antibody binds to the Fc$\gamma$ receptor on cell membrane through Fc, it may result in cross-linking activation of CD3 receptor bound at the other end due to Fc$\gamma$ receptor aggregation, thus resulting in severe off-target toxicity, for example early marketed catumaxomab triggered rapid cytokine release due to Fc fragment binding to Fc$\gamma$ receptors expressed by liver Kupffer cells. By using a human IgG2 subtype or IgG4 subtype which has a weak Fc $\gamma$ receptor binding ability, or further performing amino acid substitutions at the corresponding position in CH2, for example, Armour et al. substituted positions 233-236 (EU sequence numbering) of IgG1 and IgG4 with the corresponding sequence of IgG2, reduced binding to Fc $\gamma$ receptors (Armour KL et al., Recombinant human IgG molecules lacking Fc gamma receptor I binding and monocyte triggering activities, Eur J Immunol. 1999 Aug;29(8):2613-24), Newman et al. introduced Ser228Pro and Leu235Glu mutations in IgG4 to stabilize the IgG4 structure while reducing binding to Fc $\gamma$ receptors (Newman R. et al., Modification of the Fc region of a primatized IgG antibody to human CD4 retains its ability to modulate CD4 receptors but does not deplete CD4(+) T cells in chimpanzees, Clin Immunol. 2001 Feb;98(2):164-74,), Idusogie et al. found that alanine substitution at positions Asp270, Lys322, Pro329, or Pro331 significantly reduced the binding ability of IgG1 to complement component C1q (Idusogie EE et al., Mapping of the C1q Binding Site on Rituxan, a Chimeric Antibody with a Human IgG1 Fc, J Immunol April 15, 2000, 164 (8) 4178-4184), etc.

## Summary of the Invention

In order to solve the problems in the prior art, the present disclosure provides a novel BCMA antibody having a high affinity to the BCMA protein. Also, the present disclosure provides novel BCMA-CD3 κλ bispecific antibodies formed by BCMA antibodies and CD3 antibodies. By adopting a full-length IgG configuration, introducing charge, and optimizing affinity, BCMA-CD3 κλ bispecific antibody allows the κλ bispecific antibody preferentially to localize in BCMA+ tumor tissues, can recruit activated T cells at a low concentration, and thus causes effective killing to target cells, but does not activate T cells in the absence of target cells. At the same time, κλ bispecific antibodies do not bind to receptors such as FcγRI, FcγRIIA, FcγRIIIA, etc. thereby reducing the risk of cytokine storm occurring. It is proved in a mouse model with reconstituted human immune system that, this novel BCMA-CD3 κλ bispecific antibody can effectively inhibit tumor growth, and the efficacy was superior to the same class of antibodies.

In one aspect, the present disclosure provides a BCMA-binding antibody or antigen-binding portion thereof.

In one aspect, the present disclosure provides a bispecific antibody or antigen-binding portion thereof.

In one aspect, the present disclosure provides a nucleic acid encoding bispecific antibodies or antigen-binding portions thereof as previously described.

In one aspect, the present disclosure provides a vector comprising a nucleic acid as previously described.

In one aspect, the present disclosure provides a cell comprising a nucleic acid or vector as previously described.

The antibody or antigen-binding portion thereof according to the foregoing aspects, wherein the antibody or antigen-binding portion thereof as previously described is humanized.

In one aspect, the present disclosure provides a pharmaceutical composition or kit comprising an antibody or antigen-binding portion thereof, or nucleic acid encoding the same as previously described, and a pharmaceutically acceptable carrier.

In one aspect, the present disclosure provides antibody-drug conjugates comprising the foregoing antibodies or antigen-binding portions thereof as previously described, bispecific or multispecific molecules covalently attached to a therapeutic moiety.

In one aspect, the present disclosure provides a method of treating a disorder associated with BCMA, comprising the steps of administering to a mammal a therapeutically effective amount of an antibody or antigen-binding fragment thereof, nucleic acid, vector, cell, and/or pharmaceutical composition as previously described.

In one aspect, the present disclosure provides a use of the foregoing antibodies or antigen-binding fragments thereof, nucleic acids, vectors, cells, and/or pharmaceutical compositions in the manufacture of a drug or kit for treating a BCMA-associated disorder in a mammal.

The antibodies of the present disclosure can be used in a variety of applications, including the detection of BCMA proteins, diagnosis, treatment, or prevention of diseases associated with BCMA.

## Brief Description of the Drawings

FIG. 1 shows SDS-PAGE results of human, cynomolgus monkey BCMA-mFc bivalent recombinant protein and BCMA-hFc kih monovalent recombinant protein.

FIG. 2 shows that, both human and cynomolgus monkey BCMA stably transfected cells are detected by flow cytometry, both human BCMA stably transfected cells (CHO-hBCMA-2C2) and cynomolgus monkey BCMA stably transfected cells (CHO-cynoBCMA-1A2) express high levels of BCMA.

FIG. 3 shows SDS-PAGE results for the CD3εγ-Fc recombinant protein.

FIG. 4 shows binding of humanized CD3 antibody to CD3εγ-Fc recombinant protein.

FIG. 5 shows binding of humanized CD3 antibodies to Jurkat cells.

FIG. 6 shows binding of BCMA×CD3 κλ bispecific antibodies to BCMA stably transfected cells.

FIG. 7 shows high-affinity binding of BCMA×CD3 κλ bispecific antibodies to BCMA+ tumor cells.

FIG. 8 shows binding of BCMA×CD3 κλ bispecific antibodies to Jurkat cells.

FIG. 9 shows binding of BCMA×CD3 κλ bispecific antibodies to human peripheral blood T cells.

FIG. 10 shows killing of NCI-H929 cells and activation of T cells by the BCMA×CD3 κλ bispecific antibody, wherein FIG. 10A shows killing of NCI-H929 cells, and FIG. 10B shows activation of T cells.

FIG. 11 shows killing of RPMI-8226 cells and activation of T cells by the BCMA×CD3 κλ bispecific antibody, wherein FIG. 11A shows killing of RPMI-8226 cells, and FIG. 11B shows activation of T cells.

FIG. 12 shows activation of the T cell signaling pathway by the BCMA×CD3 κλ bispecific antibody.

FIG. 13 shows activation of peripheral blood T cells by the BCMA×CD3 κλ bispecific antibody.

FIG. 14 shows binding of BCMA×CD3 κλ bispecific antibodies to activated FcγR receptors.
FIG. 15 shows blocking of BCMA binding to the receptor APRIL by a BCMA×CD3 κλ bispecific antibody.
FIG. 16 shows inhibitory effect of BCMA×CD3 κλ bispecific antibodies on the subcutaneous NCI-H929 xenograft tumor model in immunodeficient mice.

**Detailed Description of Embodiments**

**I. Definitions**

In the present disclosure, the scientific and technical terms used herein have the meanings commonly understood by those skilled in the art, unless otherwise specified. Also, as used herein, protein and nucleic acid chemistry, molecular biology, cell and tissue culture, microbiology, immunology, and laboratory procedures used herein are terms and routine procedures widely used in the corresponding fields. Meanwhile, to better understand the present disclosure, definitions and explanations of related terms are provided below.

As used herein, the term "BCMA" may refer to the concept of BCMA itself and any variant, isotype, and paralog thereof, which are present together in animals and preferably in humans.

The term "human BCMA" refers to BCMA of human origin and may preferably have, but is not limited to, the amino acid sequence of Genbank accession number AB052772.1.

The terms "anti-BCMA antibody" and "BCMA-binding antibody" refer to an antibody that is capable of binding to BCMA with sufficient affinity such that the antibody is useful as a diagnostic and/or therapeutic agent in targeting BCMA. In one embodiment, the degree of binding of an anti-BCMA antibody to an unrelated non-BCMA protein is less than about 10% of the binding of the antibody to BCMA, as measured, for example, by a radioimmunoassay (RIA). In certain embodiments, a BCMA-binding antibody has a dissociation constant ($K_d$) of $\leq 1\ \mu M$, $\leq 100\ nM$, $\leq 10\ nM$, $\leq 1\ nM$, $\leq 0.1\ nM$, $\leq 0.01\ nM$, or $\leq 0.001\ nM$ (e.g. $10^{-8}$ M or less, e.g. from $10^{-8}$ M to $10^{-13}$ M, e.g. from $10^{-9}$ M to $10^{-13}$ M). In certain embodiments, anti-BCMA antibodies bind BCMA epitopes conserved among BCMA from different species.

"CD3" refers to any native CD3 from any vertebrate source, including mammals, such as primates (e.g. humans), non-human primates (e.g. cynomolgus monkey), and rodents (e.g. mice and rats), unless otherwise specified. The term encompasses "full-length" unprocessed CD3 as well as any form of CD3 derived from processing in cells. The term also encompasses naturally occurring variants of CD3, such as splicing variants or allelic variants. In one embodiment, the CD3 is human CD3, in particular, the ε subunit (CD3ε) of human CD3. The amino acid sequence of human CD3ε is shown under UniProt (www.uniprot.org) accession number P07766 (version 144), or NCBI (www.ncbi.nlm.nih.gov/) RefSeq NP_000724.1. The amino acid sequence of cynomolgus monkey CD3ε is shown under NCBI GenBank no. BAB71849.1.

The term "cell surface" is used in accordance with its normal meaning in the art and thus includes cell exterior accessible by binding to proteins and other molecules.

As used herein, unless otherwise specified, the term "about" or "approximately" means within plus or minus 10% of a given value or range. Where an integer is required, the term refers to an integer rounding up or down to the nearest integer within plus or minus 10% of the given value or range.

The phrase "substantially identical" with respect to polypeptide chain sequences of an antibody is understood to mean an antibody that exhibits at least 60%, 65%, 70%, 75%, 80%, 85%, 90%, 95%, 96%, 97%, 98%, 99% or more sequence identity to a reference polypeptide chain sequence. With respect to a nucleic acid sequence, the term is understood to mean a nucleotide sequence that exhibits at least greater than 60%, 65%, 70%, 75%, 80%, 85%, 90%, 95%, 96%, 97%, 98%, 99% or more sequence identity to a reference nucleic acid sequence.

Sequence "similarity" or "identity" has well-known meanings in the field, and the percentage of sequence identity between two nucleic acid or polypeptide molecules or regions can be calculated using published techniques. Sequence identity can be measured along the full-length polynucleotide or polypeptide, or along a region of the molecule. Although there are many methods of measuring the identity between two polynucleotides or polypeptides, the term "identity" is well known to technicians(Carrillo, H. & Lipman, D. SIAM J Applied Math 48: 1073 (1988)).

A "substituted" variant is a variant in which at least one amino acid residue in the native sequence has been removed and a different amino acid is inserted at its same position. The substitution may be single, wherein only one amino acid in the molecule is substituted; or maybe multiple amino acids are substituted, wherein two or more amino acids are substituted in the same molecule. Multiple substitutions may be at consecutive sites. Likewise, an amino acid may be substituted with multiple residues, wherein such variants include both substitutions and insertions. An "insertion" variant is a variant in which one or more amino acids are inserted immediately adjacent to an amino acid at a particular position in a native sequence. The immediately adjacent amino acids is meant attached to the α-carboxyl or α-amine functional groups of the amino acid. A "deletion" variant is a variant in which one or more amino acids in the native amino acid sequence have been removed. Typically, deletion variants have one or two amino acids deleted in a particular region of the molecule.

With respect to the variable domains of antibodies, the term "variable" refers to certain portions of related molecules that differ widely in sequence among antibodies and are used for specific recognition and binding of a particular antibody to its specific target. However, the variability is not uniformly distributed throughout the variable domain of the antibody. The variability is concentrated in what is known as the complementarity determining regions (CDRs; i.e. CDR1, CDR2, and CDR3) or three segments of the hypervariable region, all of which are located within the variable domains of the light chains and heavy chains. The highly conserved portions within the variable domains are referred to as framework (FR) regions or framework sequences. Each variable domain of a native heavy chain and light chain comprises four FR regions, which predominantly adopt a β-folded configuration, they are linked by three CDRs, the CDRs form loops, the loops are linked to the β-folded configuration and in some cases the loops form part of the β-folded configuration. The CDRs of each chain are typically linked together in close proximity by FR regions and are helpful for the formation of target binding sites (epitopes or determinants) of antibodies by use of CDR from other chains. As used herein, the numbering of amino acid residue of immunoglobulin is performed in accordance with the amino acid residue numbering system of immunoglobulin edited by Kabat et al., unless otherwise indicated. One CDR may have the ability to specifically bind to an associated epitope.

As used herein, an "antibody fragment" or "antigen-binding fragment" of an antibody refers to any portion of a full-length antibody that is less than full-length, but comprises at least a portion of the variable region (e.g. one or more CDR and/or one or more antibody binding sites) of the antibody that binds antigen, and thus the antibody fragment" or "antigen-binding fragment" retains binding specificity as well as at least a portion of the specific binding capacity of the full-length antibody. Therefore, an antigen-binding fragment refers to an antibody fragment that comprises an antigen-binding portion that binds to the same antigen as the antibody from which the antibody fragment is derived. Antibody fragments include antibody derivatives produced by enzymatic treatment of full-length antibodies, as well as synthetically produced derivatives, e.g. recombinantly produced derivatives. Antibodies include antibody fragments. Examples of antibody fragments include, but are not limited to, Fab, Fab', $F(ab')_2$, single chain Fv (scFv), Fv, dsFv, diabodies, Fd and Fd' fragments, and other fragments, including modified fragments. The fragments may comprise multiple chains linked together, for example by disulfide bonds and/or by peptide linkers. Antibody fragments generally comprise at least or about 50 amino acids, and typically at least or about 200 amino acids. An antigen-binding fragment includes any antibody fragment that, when the antibody fragment is inserted into an antibody framework (e.g. by displacement of the corresponding region), it results in an antibody that immunospecifically binds (i.e. exhibits a Ka of at least or at least about $10^7$-$10^8$M$^{-1}$) to an antigen. A "functional fragment" or "analog of an anti-BCMA antibody" is a fragment or analog that prevents or substantially reduces the binding ability of the receptor to a ligand or initiate signal transduction. As used herein, functional fragments generally have the same meaning as "antibody fragments", and in the case of antibodies, fragments that prevent or substantially reduce the binding ability of the receptor to a ligand or initiate signal transduction, e.g. Fv, Fab, $F(ab')_2$, and the like. A "Fv" fragment consists of a dimer ($V_H$-$V_L$ dimer) formed by a variable domain of a heavy chain and a variable domain of a light chain in a non-covalent association. In this configuration, the three CDRs of each variable domain interact with each other to determine a target binding site on the surface of the $V_H$-$V_L$ dimer, as is the case with intact antibodies. The six CDRs jointly confer target binding specificity to the intact antibody. However, even a single variable domain (or half of an Fv comprising only three target-specific CDRs) may still have the ability to recognize and bind targets.

As used herein, the term "bispecific antibody (BsAb)" refers to antibodies and/or antigen-binding molecules capable of specifically binding to two different antigene determinants. Generally, a bispecific antibody and/or antigen-binding molecule comprises two antigen-binding sites, each of which is specific for a different antigene determinant. In certain embodiments, the bispecific antibody and/or antigen-binding molecule is capable of binding two antigene determinants simultaneously, particularly two antigene determinants expressed on two different cells.

As used herein, "monoclonal antibody" refers to a population of identical antibodies, meaning that each individual antibody molecule in the population of monoclonal antibodies is identical to another antibody molecule. This property is in contrast to the property of a polyclonal population of antibodies, the polyclonal population of antibodies comprises antibodies having a plurality of different sequences. Monoclonal antibodies can be prepared by many well-known methods (Smith et al. (2004) J. Clin. Pathol.57, 912-917; and Nelson et al., J Clin Pathol (2000), 53, 111-117). For example, monoclonal antibodies can be made by immortalizing B cells, for example, by fusion with myeloma cells to produce hybridoma cell lines, or by infecting B cells with a virus such as EBV Recombinant techniques can also be used to prepare antibodies from clonal populations of host cells in vitro by transforming host cells with plasmids carrying artificial sequences encoding the nucleotides of the antibodies.

As used herein, the term "hybridoma" or "hybridoma cell" refers to a cell or cell line (typically a myeloma or lymphoma cell) resulting from the fusion of antibody-producing lymphocytes and non-antibody-producing cancer cells. As is known to those skilled in the art, hybridomas can be propagated and continuously supplied to produce a particular monoclonal antibody. Methods for producing hybridomas are known in the art. When referring to the term "hybridoma" or "hybridoma cell", it also includes subclones and progeny cells of hybridomas.

As used herein, a full-length antibody is an antibody having two full-length heavy chains (e.g. VH-CH1-CH2-CH3 or VH-CH1-CH2-CH3-CH4), two full-length light chains (VL-CL) and a hinge region, e.g. an antibody naturally produced by an antibody-secreting B cell as well as a synthetically produced antibody having the same domain.

The term "chimeric antibody" refers to an antibody in which the variable region sequences are derived from one species and the constant region sequences are derived from another species, such as an antibody in which the variable region sequences are derived from a mouse antibody and the constant region sequences are derived from a human antibody.

A "humanized" antibody refers to a form of non-human (e.g. mouse) antibody that is a chimeric immunoglobulin, immunoglobulin chain or fragment thereof (e.g. Fv, Fab, Fab', $F(ab')_2$ or other antigen-binding subsequences of an antibody) that contains a minimal sequence derived from non-human immunoglobulin. Preferably, the humanized antibody is a human immunoglobulin (recipient antibody) in which residues of the complementarity-determining region (CDR) of the recipient antibody are replaced by CDR residues from a non-human species (donor antibody) such as mouse, rat, or rabbit having the desired specificity, affinity, and capacity.

Furthermore, in humanization it is also possible to mutate amino acid residues within the CDR1, CDR2, and/or CDR3 regions of VH and/or VL, thereby improving one or more binding properties (e.g. affinity) of the antibody. Mutations can be introduced, e.g. by PCR-mediated mutagenesis, and their effect on antibody binding or other functional properties can be assessed using the in vitro or in vivo assays described herein. Typically, conservative mutations are introduced. Such mutations may be amino acid substitutions, additions, or deletions. In addition, mutations within CDR typically do not exceed one or two. Thus, the humanized antibodies of the present disclosure also encompass antibodies comprising one or two amino acid mutations within CDR.

As used herein, the term "CDR" refers to a complementarity-determining region, as is known that, each heavy chain and light chain of an antibody molecule has three CDRs. CDR is also known as a hypervariable region and is present in the variable region of each of the heavy chain and light chains of an antibody, and has sites with very high variability in the primary structure of CDR. In the present specification, the heavy chain CDR of is represented by CDR1, CDR2, and CDR3 from the amino terminus of the amino-terminal sequence of the heavy chain, and the light chain CDR is represented by CDR1, CDR2, CDR3 from the amino terminus of the amino-terminal sequence of the light chain. These sites are adjacent to each other in the tertiary structure and determine the specificity of the antigen to which the antibody binds.

As used herein, the term "epitope" refers to any antigene determinant on an antigen bound to the paratope of an antibody. Epitope determinants usually comprise chemically active surface types of molecules, such as amino acids or sugar side chains, and usually have specific three-dimensional structural characteristics as well as specific charge characteristics.

As used herein, "specific binding" or "immunospecifically binding" with respect to an antibody or antigen-binding fragment thereof is used interchangeably herein and refers to the ability of an antibody or antigen-binding fragment to form one or more non-covalent bonds with the same antigen through non-covalent interactions between the antibody and the antibody binding site of the antigen. The antigen may be an isolated antigen or present in a tumor cell. Generally, an antibody that immunospecifically binds (or specifically binds) to an antigen binds the antigen with an affinity constant Ka of about or $1\times10^7 M^{-1}$ or $1\times10^8 M^{-1}$ or more (or a dissociation constant (Kd) of $1\times10^{-7}M$ or $1\times10^{-8}M$ or less). Affinity constants can be determined by standard kinetic methods for antibody reactions, e.g. immunoassays, surface plasmon resonance (SPR) (Rich and Myszka (2000) Curr. Opin. Biotechnol 11:54; Englebienne (1998) Analyst. 123:1599), isothermal titration calorimetry (ITC), or other kinetic interaction assays known in the art; see also U.S. Patent No. 7,229,619 which describes an exemplary SPR and ITC method for calculating the binding affinity of an antibody). Instruments and methods for detecting and monitoring the rate of binding in real-time are known and commercially available (see, BiaCore 2000, Biacore AB, Upsala, Sweden and GE Healthcare Life Sciences; Malmqvist (2000) Biochem.Soc.Trans. 27:335).

As used herein, the terms "polynucleotide" and "nucleic acid molecule" refer to an oligomer or polymer comprising at least two linked nucleotides or nucleotide derivatives, including deoxyribonucleic acid (DNA) and ribonucleic acid (RNA), typically linked together by phosphodiester bonds. As used herein, the term "nucleic acid molecule" is intended to include DNA molecules and RNA molecules. The nucleic acid molecule may be single-stranded or double-stranded and may be cDNA.

As used herein, an isolated nucleic acid molecule is a nucleic acid molecule isolated from other nucleic acid molecules present in the natural source of the nucleic acid molecule. An "isolated" nucleic acid molecule, such as a cDNA molecule, can be substantially free of other cellular material or culture medium when prepared by recombinant techniques, or substantially free of chemical precursors or other chemical ingredients when chemically synthesized. Exemplary isolated nucleic acid molecules provided herein include isolated nucleic acid molecules encoding a provided antibody or antigen-binding fragment.

As used herein, "operably linked" with respect to a nucleic acid sequence, region, element or domain means that the nucleic acid regions are functionally related to one another. For example, a promoter may be operably linked to a nucleic acid encoding a polypeptide such that the promoter regulates or mediates transcription of the nucleic acid.

Also provided are "conservative sequence modifications" with respect to the sequences set forth in the sequence listings described herein, i.e. nucleotide sequence and amino acid sequence modifications that do not eliminate binding of an antibody encoded by a nucleic acid or containing an amino acid sequence to an antigen. These conservative sequence modifications include conservative nucleotide substitutions and amino acid substitutions, and nucleotide additions and amino acid additions, and nucleotide deletions and amino acid deletions. For example, modifications can be introduced into the sequence listings described herein by standard techniques known in the art, such as site-directed mutagenesis and PCR-mediated mutagenesis. Conservative sequence modifications include conservative amino acid substitutions, wherein an amino acid residue is replaced with an amino acid residue having a similar side chain. Families of amino acid residues having similar side chains have been defined in the art. These families include amino acids with basic side chains (e.g. lysine, arginine, histidine), amino acids with acidic side chains (e.g. aspartic acid, glutamic acid), amino acids with uncharged polar side chains (e.g. glycine, asparagine, glutamine, serine, threonine, tyrosine, cysteine, tryptophan), amino acids with nonpolar side chains (e.g. alanine, valine, leucine, isoleucine, proline, phenylalanine, methionine), amino acids with $\beta$-branched side chains (e.g. threonine, valine, isoleucine) and amino acids with aromatic side chains (e.g. tyrosine, phenylalanine, tryptophan, histidine). Thus, a predicted nonessential amino acid residue in an anti-BCMA antibody is preferably replaced with another amino acid residue from the same side chain family. Methods for identifying conservative substitutions of nucleotides and amino acids that do not eliminate antigen binding are well known in the art (For example, see Brummell et al., Biochem. 32: 1180-1187 (1993); Kobayashi et al., Protein Eng. 12(10): 879-884 (1999); Burks et al., Proc. Natl. Acad. Sci. USA 94: 412-417 (1997)). Alternatively, in another embodiment, mutations can be randomly introduced along all or a portion of the anti-BCMA antibody coding sequence, e.g. by saturation mutagenesis, and the resulting modified anti-BCMA antibodies can be screened for improved binding activity.

As used herein, "expression" refers to a process of producing a polypeptide by transcription and translation of a polynucleotide. The expression level of a polypeptide can be assessed using any method known in the art, including, for example, methods of determining the amount of polypeptide produced from a host cell. Such methods may include, but are not limited to, quantitation of polypeptides in cell lysates by ELISA, gel electrophoresis followed by Coomassie blue staining, Lowry protein assay, and Bradford protein assay.

As used herein, a "host cell" is a cell for receiving, maintaining, replicating, and amplifying a vector. Host cells may also be used to express a polypeptide encoded by the vector. As the host cell divides, the nucleic acid contained in the vector replicates, thereby amplifying the nucleic acids. The host cell may be a eukaryotic cell or a prokaryotic cell. Suitable host cells include, but are not limited to, CHO cells, various COS cells, HeLa cells, and HEK cells such as HEK 293 cells.

As used herein, a "vector" is a replicable nucleic acid from which one or more heterologous proteins can be expressed when the vector is transformed into an appropriate host cell. With respect to vectors, they include vectors into which a nucleic acid encoding a polypeptide or fragment thereof may be introduced, typically by restriction enzyme digestion and ligation. with respect to vectors, they also include vectors comprising a nucleic acid encoding a polypeptide. Vectors are used to introduce a nucleic acid encoding a polypeptide into a host cell, to amplify the nucleic acid, or to express/display the polypeptide encoded by the nucleic acid. The vectors usually remain episomal, but can be designed such that the gene or part thereof is integrated into the chromosome of the genome. Also contemplated are vectors for artificial chromosomes, such as yeast artificial chromosome vectors and mammalian artificial chromosomes. The selection and use of such vehicles are well-known to those skilled in the art.

As used herein, a vector also includes a "virus vector" or a "viral vector". A viral vector is an engineered virus operably linked to an exogenous gene to transfer the exogenous gene (as a vehicle or shuttle) into a cell.

As used herein, an "expression vector" includes a vector capable of expressing a DNA, the DNA is operably linked to regulatory sequences, such as a promoter region, capable of affecting the expression of such a DNA fragment. Such additional fragments may include promoter and terminator sequences and optionally may include one or more origins of replication, one or more selectable markers, enhancers, polyadenylation signals, and the like. Expression vectors are typically derived from plasmid or viral DNA, or may contain elements of both. Thus, an expression vector refers to a recombinant DNA or RNA construct, such as a plasmid, phage, recombinant virus, or other vector. When introduced into an appropriate host cell, it results in the expression of a cloned DNA. Suitable expression vectors are well known to those skilled in the art and include expression vectors that are replicable in eukaryotic and/or prokaryotic cells and expression vectors that remain episomal or that are integrated into the host cell genome.

As used herein, the "treatment" of an individual with a disease or disease condition indicates that the symptoms of the individual are partially or completely relieved, or remain unchanged after treatments. Therefore, treatment includes prevention, treatment, and/or cure. Prevention refers to preventing the underlying disease and/or preventing the worsening of symptoms or the progression of the disease. Treatment also includes any pharmaceutical use of any antibody or antigen-binding fragment thereof provided and the compositions provided herein.

As used herein, "therapeutic effect" refers to an effect resulting from the treatment of a subject that alters, typically ameliorates, or ameliorates a symptom of a disease or condition, or cures a disease or condition.

As used herein, a "therapeutically effective amount" or "therapeutically effective dose" refers to an amount of a substance, compound, material, or composition comprising a compound that is at least sufficient to produce a therapeutic effect after administration to a subject. Thus, it is an amount necessary to prevent, cure, ameliorate, block, or partially block the symptoms of a disease or disorder.

As used herein, a "prophylactically effective amount" or "prophylactically effective dose" refers to an amount of a substance, compound, material, or composition comprising a compound that, when administered to a subject, has the desired prophylactic effect, e.g. prevents or delays the occurrence or recurrence of a disease or condition, reduces the likelihood of the occurrence or recurrence of a disease or condition. A fully prophylactically effective dose does not need to occur by administration of one dose, and may occur only after administration of a series of doses. Thus, a prophylactically effective amount can be administered in one or more administrations.

As used herein, the term "patient" refers to a mammal, such as a human.

## II. Detailed Description of Specific Embodiments

In one aspect, the present disclosure provides a bispecific antibody or antigen-binding portion thereof, comprising:

(a) a first antigen-binding portion or an antigen-binding fragment thereof bound to a BCMA antigen on the surface of a target cell, the first antigen-binding portion comprises a first heavy chain and a first light chain, the first antigen-binding portion comprises a first binding domain that binds to the first antigen, wherein the first binding domain comprises a heavy chain CDR selected from the amino acid sequences of SEQ ID NOs: 12, 13, 14, 26, 27, 28, 36, 37, 43, 44, 50, 55, 56, 57, 65, 66, 71, 72, 73 and 147, or any variant thereof, and/or a light chain CDR selected from the amino acid sequences of SEQ ID NOs: 17, 18, 19, 22, 23, 31, 32, 33, 40, 47, 60, 61, 62, 76, 77, 78, 83, 84, 87, 88, 89, 92 and 144 or any variant; and
(b) a second antigen-binding portion or antigen-binding fragment thereof bound to a CD3 antigen on the surface of a T cell, the second antigen-binding portion comprises a second heavy chain and a second light chain, the second antigen-binding portion comprises a second binding domain that binds to a second antigen.

The antibody or antigen-binding portion thereof according to the preceding aspect, the first binding domain comprises a heavy chain CDR1 selected from the amino acid sequences of SEQ ID NOs: 12, 26, 55, 65, 71 or any variant thereof, a heavy chain CDR2 selected from the amino acid sequences of SEQ ID NOs: 13, 27, 36, 43, 50, 56, 66, 72, 147 or any variant thereof, a heavy chain CDR3 selected from the amino acid sequences of SEQ ID NOs: 14, 28, 37, 44, 57, 73 or any variant thereof; and/or the first binding domain comprises a light chain CDR1 selected from the amino acid sequences of SEQ ID NOs: 17, 22, 31, 47, 60, 76, 83, 87, 92, 144 or any variant thereof, a light chain CDR2 selected from the amino acid sequences of SEQ ID NOs: 18, 23, 32, 40, 61, 77, 84, 88 or any variant thereof, a light chain CDR3 selected from the amino acid sequences of SEQ ID NOs: 19, 33, 62, 78, 89 or any variant thereof.

The antibody or antigen-binding portion thereof according to any of the preceding aspects, the heavy chain CDR1, CDR2, and CDR3 sequences of the first binding domain are selected from: the first heavy chain CDR1, CDR2, and CDR3 sequences of the amino acid sequences of SEQ ID NOs: 12, 13 and 14, the first heavy chain CDR1, CDR2, and CDR3 sequences of the amino acid sequences of SEQ ID NOs: 26, 27 and 28, the first heavy chain CDR1, CDR2, and CDR3 sequences of the amino acid sequences of SEQ ID NOs: 12, 36 and 37, the first heavy chain CDR1, CDR2, and CDR3 sequences of the amino acid sequences of SEQ ID NOs: 12, 43 and 44, the first heavy chain CDR1, CDR2, and CDR3 sequences of the amino acid sequences of SEQ ID NOs: 12, 50 and 14, the first heavy chain CDR1, CDR2, and CDR3 sequences of the amino acid sequences of SEQ ID NOs: 55, 56 and 57, the first heavy chain CDR1, CDR2, and CDR3 sequences of the amino acid sequences of SEQ ID NOs: 65, 66 and 14, the first heavy chain CDR1, CDR2, and CDR3 sequences of the amino acid sequences of SEQ ID NOs: 71, 72 and 73; the first heavy chain CDR1, CDR2, and CDR3 sequences of the amino acid sequences of SEQ ID NOs: 71, 147 and 73; and the light chain CDR1, CDR2, and CDR3 sequences of the first binding domain are selected from: the sequences of the first light chain CDR1, CDR2 and CDR3 of the amino acid sequences of SEQ ID NOs: 17, 18 and 19, the sequences of the first light chain CDR1, CDR2 and CDR3 of the amino acid sequences of SEQ ID NOs: 22, 23 and 19, the sequences of the first light chain CDR1, CDR2 and CDR3 of the amino acid sequences of SEQ ID NOs: 31, 32 and 33, the sequences of the first light chain CDR1, CDR2 and CDR3 of the amino acid sequences of SEQ ID NOs: 17, 40 and 19, the sequences of the first light chain CDR1, CDR2 and CDR3 of the amino acid sequences of SEQ ID NOs: 47, 18 and 19, the sequences of the first light chain CDR1, CDR2 and CDR3 of the amino acid sequences of SEQ ID NOs: 60, 61 and 62, the sequences of the first light chain CDR1, CDR2 and CDR3 of the amino acid sequences of SEQ ID NOs: 76, 77 and 78, and the sequences of the first light chain CDR1, CDR2 and CDR3 of the amino acid sequences of SEQ ID NOs: 83, 84 and 19, the first light chain CDR1, CDR2, and CDR3 sequences of the amino acid sequences of SEQ ID NOs: 87, 88 and 89, the first light chain CDR1, CDR2, and CDR3 sequences of the amino acid sequences of SEQ ID NOs: 92, 23 and 19, the first light chain CDR1, CDR2, and CDR3 sequences of the amino acid sequences of SEQ ID NOs: 144, 77 and 78.

In some embodiments, the first binding domain comprises the first heavy chain CDR1, CDR2, and CDR3 sequences of the amino acid sequences of SEQ ID NOs: 12, 13 and 14 and the first light chain CDR1, CDR2, and CDR3 sequences of the amino acid sequences of SEQ ID NOs: 17, 18 and 19.

In some embodiments, the first binding domain comprises the first heavy chain CDR1, CDR2, and CDR3 sequences of the amino acid sequences of SEQ ID NOs: 12, 13 and 14 and the first light chain CDR1, CDR2, and CDR3 sequences of the amino acid sequences of SEQ ID NOs: 22, 23 and 19.

In some embodiments, the first binding domain comprises the first heavy chain CDR1, CDR2, and CDR3 sequences of the amino acid sequences of SEQ ID NOs: 26, 27 and 28 and the first light chain CDR1, CDR2, and CDR3 sequences of the amino acid sequences of SEQ ID NOs: 31, 32 and 33.

In some embodiments, the first binding domain comprises the first heavy chain CDR1, CDR2, and CDR3 sequences of the amino acid sequences of SEQ ID NOs: 12, 36 and 37 and the first light chain CDR1, CDR2, and CDR3 sequences of the amino acid sequences of SEQ ID NOs: 17, 40 and 19.

In some embodiments, the first binding domain comprises the first heavy chain CDR1, CDR2, and CDR3 sequences of the amino acid sequences of SEQ ID NOs: 12, 43 and 44 and the first light chain CDR1, CDR2, and CDR3 sequences of the amino acid sequences of SEQ ID NOs: 47, 18 and 19.

In some embodiments, the first binding domain comprises the first heavy chain CDR1, CDR2, and CDR3 sequences of the amino acid sequences of SEQ ID NOs: 12, 50 and 14 and the first light chain CDR1, CDR2, and CDR3 sequences of the amino acid sequences of SEQ ID NOs: 17, 40 and 19.

In some embodiments, the first binding domain comprises the first heavy chain CDR1, CDR2, and CDR3 sequences of the amino acid sequences of SEQ ID NOs: 55, 56 and 57 and the first light chain CDR1, CDR2, and CDR3 sequences of the amino acid sequences of SEQ ID NOs: 60, 61 and 62.

In some embodiments, the first binding domain comprises the first heavy chain CDR1, CDR2, and CDR3 sequences of the amino acid sequences of SEQ ID NOs: 12, 13 and 14 and the first light chain CDR1, CDR2, and CDR3 sequences of the amino acid sequences of SEQ ID NOs: 17, 40 and 19.

In some embodiments, the first binding domain comprises the first heavy chain CDR1, CDR2, and CDR3 sequences of the amino acid sequences of SEQ ID NOs: 65, 66 and 14 and the first light chain CDR1, CDR2, and CDR3 sequences of the amino acid sequences of SEQ ID NOs: 17, 40 and 19.

In some embodiments, the first binding domain comprises the first heavy chain CDR1, CDR2, and CDR3 sequences of the amino acid sequences of SEQ ID NOs: 71, 72 and 73 and the first light chain CDR1, CDR2, and CDR3 sequences of the amino acid sequences of SEQ ID NOs: 76, 77 and 78.

In some embodiments, the first binding domain comprises the first heavy chain CDR1, CDR2, and CDR3 sequences of the amino acid sequences of SEQ ID NOs: 12, 13 and 14 and the first light chain CDR1, CDR2, and CDR3 sequences of the amino acid sequences of SEQ ID NOs: 83, 84 and 19.

In some embodiments, the first binding domain comprises the first heavy chain CDR1, CDR2, and CDR3 sequences of the amino acid sequences of SEQ ID NOs: 12, 13, and 14 and the first light chain CDR1, CDR2, and CDR3 sequences of the amino acid sequences of SEQ ID NOs: 87, 88 and 89.

In some embodiments, the first binding domain comprises the first heavy chain CDR1, CDR2, and CDR3 sequences of the amino acid sequences of SEQ ID NOs: 12, 13 and 14 and the first light chain CDR1, CDR2, and CDR3 sequences of the amino acid sequences of SEQ ID NOs: 92, 23 and 19.

In some embodiments, the first binding domain comprises the first heavy chain CDR1, CDR2, and CDR3 sequences of the amino acid sequences of SEQ ID NOs: 71, 147 and 73 and the first light chain CDR1, CDR2, and CDR3 sequences of the amino acid sequences of SEQ ID NOs: 144, 77 and 78.

The antibody or antigen-binding portion thereof according to any of the preceding aspects, the first binding domain comprises a first heavy chain variable region selected from the amino acid sequences of SEQ ID NOs: 10, 24, 34, 41, 48, 53, 63, 69, 79, 145, 150, 154, 158, 162, 166 and 170 or any variant thereof, and/or comprises a first light chain variable region selected from the amino acid sequences of SEQ ID NOs: 15, 20, 29, 38, 45, 51, 58, 67, 74, 81, 85, 90, 142, 148, 152, 156, 160, 164 and 168 or any variant thereof.

In some embodiments, the first binding domain comprises a first heavy chain variable region of the amino acid sequence of SEQ ID NO: 10 or any variant thereof, and a first light chain variable region of the amino acid sequence of SEQ ID NO: 15 or any variant thereof.

In some embodiments, the first binding domain comprises a first heavy chain variable region of the amino acid sequence of SEQ ID NO: 10 or any variant thereof, and a first light chain variable region of the amino acid sequence of SEQ ID NO: 20 or any variant thereof.

In some embodiments, the first binding domain comprises a first heavy chain variable region of the amino acid sequence of SEQ ID NO: 24 or any variant thereof, and a first light chain variable region of the amino acid sequence of SEQ ID NO: 29 or any variant thereof.

In some embodiments, the first binding domain comprises a first heavy chain variable region of the amino acid sequence of SEQ ID NO: 34 or any variant thereof, and a first light chain variable region of the amino acid sequence of SEQ ID NO: 38 or any variant thereof.

In some embodiments, the first binding domain comprises a first heavy chain variable region of the amino acid sequence of SEQ ID NO: 41 or any variant thereof, and a first light chain variable region of the amino acid sequence of SEQ ID NO: 45 or any variant thereof.

In some embodiments, the first binding domain comprises a first heavy chain variable region of the amino acid sequence of SEQ ID NO: 48 or any variant thereof, and a first light chain variable region of the amino acid sequence of SEQ ID NO: 51 or any variant thereof.

In some embodiments, the first binding domain comprises a first heavy chain variable region of the amino acid sequence of SEQ ID NO: 53 or any variant thereof, and a first light chain variable region of the amino acid sequence of SEQ ID NO: 58 or any variant thereof.

In some embodiments, the first binding domain comprises a first heavy chain variable region of the amino acid sequence of SEQ ID NO: 10 or any variant thereof, and a first light chain variable region of the amino acid sequence of SEQ ID NO: 38 or any variant thereof.

In some embodiments, the first binding domain comprises a first heavy chain variable region of the amino acid sequence of SEQ ID NO: 63 or any variant thereof, and a first light chain variable region of the amino acid sequence of SEQ ID NO: 67 or any variant thereof.

In some embodiments, the first binding domain comprises a first heavy chain variable region of the amino acid sequence of SEQ ID NO: 69 or any variant thereof, and a first light chain variable region of the amino acid sequence of SEQ ID NO: 74 or any variant thereof.

In some embodiments, the first binding domain comprises a first heavy chain variable region of the amino acid sequence of SEQ ID NO: 79 or any variant thereof, and a first light chain variable region of the amino acid sequence of SEQ ID NO: 81 or any variant thereof.

In some embodiments, the first binding domain comprises a first heavy chain variable region of the amino acid sequence of SEQ ID NO: 10 or any variant thereof, and a first light chain variable region of the amino acid sequence of SEQ ID NO: 85 or any variant thereof.

In some embodiments, the first binding domain comprises a first heavy chain variable region of the amino acid sequence of SEQ ID NO: 10 or any variant thereof, and a first light chain variable region of the amino acid sequence of SEQ ID NO: 90 or any variant thereof.

In some embodiments, the first binding domain comprises a first heavy chain variable region of the amino acid sequence of SEQ ID NO: 145 or any variant thereof, and a first light chain variable region of the amino acid sequence of SEQ ID NO: 142 or any variant thereof.

In some embodiments, the first binding domain comprises a first heavy chain variable region of the amino acid sequence of SEQ ID NO: 150 or any variant thereof, and a first light chain variable region of the amino acid sequence of SEQ ID NO: 148 or any variant thereof.

In some embodiments, the first binding domain comprises a first heavy chain variable region of the amino acid sequence of SEQ ID NO: 154 or any variant thereof, and a first light chain variable region of the amino acid sequence of SEQ ID NO: 152 or any variant thereof.

In some embodiments, the first binding domain comprises a first heavy chain variable region of the amino acid sequence of SEQ ID NO: 158 or any variant thereof, and a first light chain variable region of the amino acid sequence of SEQ ID NO: 156 or any variant thereof.

In some embodiments, the first binding domain comprises a first heavy chain variable region of the amino acid sequence of SEQ ID NO: 162 or any variant thereof, and a first light chain variable region of the amino acid sequence of SEQ ID NO: 160 or any variant thereof.

In some embodiments, the first binding domain comprises a first heavy chain variable region of the amino acid sequence of SEQ ID NO: 166 or any variant thereof, and a first light chain variable region of the amino acid sequence of SEQ ID NO: 164 or any variant thereof.

In some embodiments, the first binding domain comprises a first heavy chain variable region of the amino acid sequence of SEQ ID NO: 170 or any variant thereof, and a first light chain variable region of the amino acid sequence of SEQ ID NO: 168 or any variant thereof.

The antibody or antigen-binding portion thereof according to any of the preceding aspects, the second binding domain comprises a heavy chain CDR selected from the amino acid sequences of SEQ ID NOs: 114, 115, 116, 119, 122, 125, 128, 131, 134, 135 or any variant thereof; and/or a light chain CDR selected from the amino acid sequences of SEQ ID NOs: 95, 96, 97, 102, 103, 108, 111 or any variant thereof.

In some embodiments, the second binding domain comprises a second heavy chain CDR1 selected from the amino acid sequences of SEQ ID NOs: 114, 119, 134 or any variant thereof, a second heavy chain CDR2 selected from the amino acid sequences of SEQ ID NOs: 115, 135 or any variant thereof, a second heavy chain CDR3 selected from the amino acid sequences of SEQ ID NOs: 116, 122, 125, 128, 131 or any variant thereof; and/or a second light chain CDR1 selected from the amino acid sequences of SEQ ID NOs: 95, 102 or any variant thereof, a second light chain CDR2 selected from the amino acid sequences of SEQ ID NOs: 96, 103, 111 or any variant thereof, a second light chain CDR3 selected from the amino acid sequences of SEQ ID NOs: 91, 108 or any variant thereof.

In some embodiments, the heavy chain CDR1, CDR2, and CDR3 sequences of the second binding domain are selected from: second heavy chain CDR1, CDR2, and CDR3 sequences of the amino acid sequences of SEQ ID NOs: 114, 115 and 116, second heavy chain CDR1, CDR2, and CDR3 sequences of the amino acid sequences of SEQ ID NOs: 119, 115 and 116, second heavy chain CDR1, CDR2, and CDR3 sequences of the amino acid sequences of SEQ ID NOs: 119, 115 and 122, second heavy chain CDR1, CDR2, and CDR3 sequences of the amino acid sequences of SEQ ID NOs: 119, 115 and 125, second heavy chain CDR1, CDR2, and CDR3 sequences of the amino acid sequences of SEQ ID NOs: 119, 115 and 128, second heavy chain CDR1, CDR2, and CDR3 sequences of the amino acid sequences of SEQ ID NOs: 119, 115 and 131, second heavy chain CDR1, CDR2, and CDR3 sequences of the amino acid sequences of SEQ ID NOs: 134, 135 and 116; and the light chain CDR1, CDR2, and CDR3 sequences of the second binding domain are selected from: second light chain CDR1, CDR2, and CDR3 sequences of the amino acid sequences of SEQ ID NOs: 95, 96 and 97, second light chain CDR1, CDR2, and CDR3 sequences of the amino acid sequences of SEQ ID NOs: 102, 103 and 97, second light chain CDR1, CDR2, and CDR3 sequences of the amino acid sequences of SEQ ID NOs: 95, 96 and 108, second light chain CDR1, CDR2, and CDR3 sequences of the amino acid sequences of SEQ ID NOs: 95, 111 and 108.

In some embodiments, the second binding domain comprises the second heavy chain CDR1, CDR2, and CDR3 sequences of the amino acid sequences of SEQ ID NOs: 114, 115 and 116, and the second light chain CDR1, CDR2, and CDR3 sequences of the amino acid sequences of SEQ ID NOs: 95, 96 and 97.

In some embodiments, the second binding domain comprises the second heavy chain CDR1, CDR2, and CDR3 sequences of the amino acid sequences of SEQ ID NOs: 114, 115 and 116, and the second light chain CDR1, CDR2, and CDR3 sequences of the amino acid sequences of SEQ ID NOs: 102, 103 and 97.

In some embodiments, the second binding domain comprises the second heavy chain CDR1, CDR2, and CDR3 sequences of the amino acid sequences of SEQ ID NOs: 114, 115 and 116, and the second light chain CDR1, CDR2, and CDR3 sequences of the amino acid sequences of SEQ ID NOs: 95, 96 and 108.

In some embodiments, the second binding domain comprises the second heavy chain CDR1, CDR2, and CDR3 sequences of the amino acid sequences of SEQ ID NOs: 114, 115 and 116, and the second light chain CDR1, CDR2, and CDR3 sequences of the amino acid sequences of SEQ ID NOs: 95, 111 and 108.

In some embodiments, the second binding domain comprises a second heavy chain variable region selected from the amino acid sequences of SEQ ID NOs: 112, 117, 120, 123, 126, 129, 132, 136, 138, 140, or any variant thereof, and/or comprises a second light chain variable region selected from the amino acid sequences of SEQ ID NOs: 93, 98, 100, 104, 106, 109 or any variant thereof.

In some embodiments, the second binding domain comprises a second heavy chain variable region of the amino acid sequence of SEQ ID NO: 136 or any variant thereof, and a second light chain variable region of the amino acid sequence of SEQ ID NO: 93 or any variant thereof.

In some embodiments, the second binding domain comprises a second heavy chain variable region of the amino acid sequence of SEQ ID NO: 138 or any variant thereof, and a second light chain variable region of the amino acid sequence of SEQ ID NO: 93 or any variant thereof.

In some embodiments, the second binding domain comprises a second heavy chain variable region of the amino acid sequence of SEQ ID NO: 138 or any variant thereof, and a second light chain variable region of the amino acid sequence of SEQ ID NO: 98 or any variant thereof.

In some embodiments, the second binding domain comprises a second heavy chain variable region of the amino acid sequence of SEQ ID NO: 138 or any variant thereof, and a second light chain variable region of the amino acid sequence of SEQ ID NO: 100 or any variant thereof.

In some embodiments, the second binding domain comprises a second heavy chain variable region of the amino acid sequence of SEQ ID NO: 112 or any variant thereof, and a second light chain variable region of the amino acid sequence of SEQ ID NO: 106 or any variant thereof.

In some embodiments, the second binding domain comprises a second heavy chain variable region of the amino acid sequence of SEQ ID NO: 136 or any variant thereof, and a second light chain variable region of the amino acid sequence of SEQ ID NO: 106 or any variant thereof.

In some embodiments, the second binding domain comprises a second heavy chain variable region of the amino acid sequence of SEQ ID NO: 138 or any variant thereof, and a second light chain variable region of the amino acid sequence of SEQ ID NO: 106 or any variant thereof.

The antibody or antigen-binding portion thereof according to any of the preceding aspects, the first light chain of the first antigen-binding portion is a κ light chain and the second light chain of the second antigen-binding portion is a λ light chain,

in some embodiments, the second light chain variable region of the second antigen-binding portion has a $Gln_{40}Glu$ mutation ($V\lambda_{CD3}$:$Gln_{40}Glu$); the second heavy chain variable region of the second antigen-binding portion has a $Gln_{39}Lys$ mutation ($VH_{CD3}$:$Gln_{39}Lys$).

In some embodiments, the first light chain variable region of the first antigen-binding portion has a $Gln_{42}Lys$ mutation ($V\kappa_{BCMA}$: $Gln_{42}Lys$) In some embodiments, the first heavy chain variable region of the first antigen-binding portion has a $Gln_{39}Glu$ mutation ($VH_{BCMA}$: $Gln_{39}Glu$).

In some embodiments, the Fc portion of the first antigen-binding portion and the second antigen-binding portion of the bispecific antibody adopts a knob-into-hole structure. In some preferred embodiments, the Fc portion adopts a knob-into-hole structure of human IgG4.

In some embodiments, the first heavy chain comprises a heavy chain selected from SEQ ID NOs: 174, 178, or any variant thereof, the first light chain comprises a light chain selected from SEQ ID Nos: 172, 176, or any variant thereof.

In some preferred embodiments, the first heavy chain comprises a heavy chain selected from SEQ ID NO: 174 or any variant thereof, the first light chain comprises a light chain selected from SEQ ID NO: 172 or any variant thereof.

In some preferred embodiments, the first heavy chain comprises a heavy chain selected from SEQ ID NO: 174 or any variant thereof, the first light chain comprises a light chain selected from SEQ ID NO: 176 or any variant thereof.

In some preferred embodiments, the first heavy chain comprises a heavy chain selected from SEQ ID NO: 178 or any variant thereof, the first light chain comprises a light chain selected from SEQ ID NO: 172 or any variant thereof.

In some preferred embodiments, the first heavy chain comprises a heavy chain selected from SEQ ID NO: 178 or any variant thereof, the first light chain comprises a light chain selected from SEQ ID NO: 176 or any variant thereof.

In some embodiments, the second heavy chain comprises a heavy chain of SEQ ID NO: 182 or any variant thereof, the second light chain comprises a light chain of SEQ ID NO: 180 or any variant thereof.

In some preferred embodiments, the first binding domain of the bispecific antibody comprises a first heavy chain variable region of the amino acid sequence of SEQ ID NO: 145 or any variant thereof, and a first light chain variable region of the amino acid sequence of SEQ ID NO: 142 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof.

In some preferred embodiments, the first binding domain of the bispecific antibody comprises a first heavy chain variable region of the amino acid sequence of SEQ ID NO: 150 or any variant thereof, and a first light chain variable region of the amino acid sequence of SEQ ID NO: 148 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof.

In some preferred embodiments, the first binding domain of the bispecific antibody comprises a first heavy chain variable region of the amino acid sequence of SEQ ID NO: 154 or any variant thereof, and a first light chain variable region of the amino acid sequence of SEQ ID NO: 152 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof.

In some preferred embodiments, the first binding domain of the bispecific antibody comprises a first heavy chain variable region of the amino acid sequence of SEQ ID NO: 158 or any variant thereof, and a first light chain variable region of the amino acid sequence of SEQ ID NO: 156 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof.

In some preferred embodiments, the first binding domain of the bispecific antibody comprises a first heavy chain variable region of the amino acid sequence of SEQ ID NO: 162 or any variant thereof, and a first light chain variable region of the amino acid sequence of SEQ ID NO: 160 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof.

In some preferred embodiments, the first binding domain of the bispecific antibody comprises a first heavy chain variable region of the amino acid sequence of SEQ ID NO: 166 or any variant thereof, and a first light chain variable region of the amino acid sequence of SEQ ID NO: 164 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof.

In some preferred embodiments, the first binding domain of the bispecific antibody comprises a first heavy chain variable region of the amino acid sequence of SEQ ID NO: 170 or any variant thereof, and a first light chain variable region of the amino acid sequence of SEQ ID NO: 168 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof;

In a specific embodiment, the first heavy chain comprises SEQ ID NO: 174, the first light chain of the bispecific antibody comprises SEQ ID NO: 172; the second heavy chain comprises SEQ ID NO: 182, the second light chain comprises SEQ ID NO: 180.

In a specific embodiment, the first heavy chain comprises SEQ ID NO: 174, the first light chain of the bispecific antibody comprises SEQ ID NO: 176; the second heavy chain comprises SEQ ID NO: 182, the second light chain comprises SEQ ID NO: 180.

In a specific embodiment, the first heavy chain comprises SEQ ID NO: 178, the first light chain of the bispecific antibody comprises SEQ ID NO: 172; the second heavy chain comprises SEQ ID NO: 182, the second light chain comprises SEQ ID NO: 180.

In a specific embodiment, the first heavy chain comprises SEQ ID NO: 178, the first light chain of the bispecific antibody comprises SEQ ID NO: 176; the second heavy chain comprises SEQ ID NO: 182, the second light chain comprises SEQ ID NO: 180.

In one aspect, the present disclosure provides a BCMA-binding antibody or antigen-binding portion thereof, the antibody comprises a first antigen-binding portion as previously described.

In some embodiments, the BCMA-binding antibody or antigen-binding portion thereof has at least greater than 60%, 65%, 70%, 75%, 80%, 85%, 90%, 95%, 96%, 97%, 98%, 99% or more sequence identity to the antibody or antigen-binding portion thereof of any of the preceding aspects.

In one aspect, the present disclosure provides a nucleic acid encoding the antibody or antigen-binding portion thereof according to any of the preceding aspects, or a nucleic acid molecule having at least greater than 60%, 65%, 70%, 75%, 80%, 85%, 90%, 95%, 96%, 97%, 98%, 99% or more sequence identity thereto.

In some embodiments, the nucleic acid encoding the first heavy chain variable region of the first antigen-binding portion is selected from the nucleotide sequences of SEQ ID NOs: 11, 25, 35, 42, 49, 54, 64, 70, 80, 146, 151, 155, 159, 163, 167, 171 or any variant thereof; and/or the nucleic acid encoding the first light chain variable region of the first antigen-binding portion is selected from the nucleotide sequences of SEQ ID NOs: 16, 21, 30, 39, 46, 52, 59, 68, 75, 82, 86, 91, 143, 149, 153, 157161, 165, 169 or any variant thereof.

In some preferred embodiments, the nucleic acid encoding the first heavy chain variable region of the first antigen-binding portion is the nucleotide sequence of SEQ ID NO: 11; and the nucleic acid encoding the first light chain variable region is the nucleotide sequence of SEQ ID NO: 16.

In some preferred embodiments, the nucleic acid encoding the first heavy chain variable region of the first antigen-binding portion is the nucleotide sequence of SEQ ID NO: 11; and the nucleic acid encoding the first light chain variable region is the nucleotide sequence of SEQ ID NO: 21.

In some preferred embodiments, the nucleic acid encoding the first heavy chain variable region of the first antigen-binding portion is the nucleotide sequence of SEQ ID NO: 25; and the nucleic acid encoding the first light chain variable region is the nucleotide sequence of SEQ ID NO: 30.

In some preferred embodiments, the nucleic acid encoding the first heavy chain variable region of the first antigen-binding portion is the nucleotide sequence of SEQ ID NO: 35; and the nucleic acid encoding the first light chain variable region is the nucleotide sequence of SEQ ID NO: 39.

In some preferred embodiments, the nucleic acid encoding the first heavy chain variable region of the first antigen-binding portion is the nucleotide sequence of SEQ ID NO: 42; and the nucleic acid encoding the first light chain variable region is the nucleotide sequence of SEQ ID NO: 46.

In some preferred embodiments, the nucleic acid encoding the first heavy chain variable region of the first antigen-binding portion is the nucleotide sequence of SEQ ID NO: 49; and the nucleic acid encoding the first light chain variable region is the nucleotide sequence of SEQ ID NO: 52.

In some preferred embodiments, the nucleic acid encoding the first heavy chain variable region of the first antigen-binding portion is the nucleotide sequence of SEQ ID NO: 54; and the nucleic acid encoding the first light chain variable region is the nucleotide sequence of SEQ ID NO: 59.

In some preferred embodiments, the nucleic acid encoding the first heavy chain variable region of the first antigen-binding portion is the nucleotide sequence of SEQ ID NO: 11; and the nucleic acid encoding the first light chain variable region is the nucleotide sequence of SEQ ID NO: 39.

In some preferred embodiments, the nucleic acid encoding the first heavy chain variable region of the first antigen-binding portion is the nucleotide sequence of SEQ ID NO: 64; and the nucleic acid encoding the first light chain variable region is the nucleotide sequence of SEQ ID NO: 68.

In some preferred embodiments, the nucleic acid encoding the first heavy chain variable region of the first antigen-binding portion is the nucleotide sequence of SEQ ID NO: 70; and the nucleic acid encoding the first light chain variable region is the nucleotide sequence of SEQ ID NO: 75.

In some preferred embodiments, the nucleic acid encoding the first heavy chain variable region of the first antigen-binding portion is the nucleotide sequence of SEQ ID NO: 80; and the nucleic acid encoding the first light chain variable region is the nucleotide sequence of SEQ ID NO: 82.

In some preferred embodiments, the nucleic acid encoding the first heavy chain variable region of the first antigen-binding portion is the nucleotide sequence of SEQ ID NO: 11; and the nucleic acid encoding the first light chain variable region is the nucleotide sequence of SEQ ID NO: 86.

In some preferred embodiments, the nucleic acid encoding the first heavy chain variable region of the first antigen-binding portion is the nucleotide sequence of SEQ ID NO: 11; and the nucleic acid encoding the first light chain variable region is the nucleotide sequence of SEQ ID NO: 91.

In some preferred embodiments, the nucleic acid encoding the first heavy chain variable region of the first antigen-binding portion is the nucleotide sequence of SEQ ID NO: 146; and the nucleic acid encoding the first light chain variable region is the nucleotide sequence of SEQ ID NO: 143.

In some preferred embodiments, the nucleic acid encoding the first heavy chain variable region of the first antigen-binding portion is the nucleotide sequence of SEQ ID NO: 151; and the nucleic acid encoding the first light chain variable region is the nucleotide sequence of SEQ ID NO: 149.

In some preferred embodiments, the nucleic acid encoding the first heavy chain variable region of the first antigen-binding portion is the nucleotide sequence of SEQ ID NO: 155; and the nucleic acid encoding the first light chain variable region is the nucleotide sequence of SEQ ID NO: 153.

In some preferred embodiments, the nucleic acid encoding the first heavy chain variable region of the first antigen-binding portion is the nucleotide sequence of SEQ ID NO: 159; and the nucleic acid encoding the first light chain variable region is the nucleotide sequence of SEQ ID NO: 157.

In some preferred embodiments, the nucleic acid encoding the first heavy chain variable region of the first antigen-binding portion is the nucleotide sequence of SEQ ID NO: 163; and the nucleic acid encoding the first light chain variable region is the nucleotide sequence of SEQ ID NO: 161.

In some preferred embodiments, the nucleic acid encoding the first heavy chain variable region of the first antigen-binding portion is the nucleotide sequence of SEQ ID NO: 167; and the nucleic acid encoding the first light chain variable region is the nucleotide sequence of SEQ ID NO: 165.

In some preferred embodiments, the nucleic acid encoding the first heavy chain variable region of the first antigen-binding portion is the nucleotide sequence of SEQ ID NO: 171; and the nucleic acid encoding the first light chain variable region is the nucleotide sequence of SEQ ID NO: 169.

In some embodiments, the nucleic acid encoding the first heavy chain of the first antigen-binding portion is selected from the nucleotide sequences of SEQ ID NOs: 175, 179 or any variant thereof; and/or the nucleic acid encoding the first light chain of the first antigen-binding portion is selected from the nucleotide sequences of SEQ ID NOs: 173, 177 or any variant thereof.

In some preferred embodiments, the nucleic acid encoding the first heavy chain of the first antigen-binding portion is the nucleotide sequence of SEQ ID NO: 175, and the nucleic acid encoding the first light chain of the first antigen-binding portion is the nucleotide sequence of SEQ ID NO: 173.

In some preferred embodiments, the nucleic acid encoding the first heavy chain of the first antigen-binding portion is the nucleotide sequence of SEQ ID NO: 175, and the nucleic acid encoding the first light chain of the first antigen-binding portion is the nucleotide sequence of SEQ ID NO: 177.

In some preferred embodiments, the nucleic acid encoding the first heavy chain of the first antigen-binding portion is the nucleotide sequence of SEQ ID NO: 179 and the nucleic acid encoding the first light chain of the first antigen-binding portion is the nucleotide sequence of SEQ ID NO: 173.

In some preferred embodiments, the nucleic acid encoding the first heavy chain of the first antigen-binding portion is the nucleotide sequence of SEQ ID NO: 179 and the nucleic acid encoding the first light chain of the first antigen-binding portion is the nucleotide sequence of SEQ ID NO: 177.

In some embodiments, the nucleic acid encoding the second heavy chain variable region of the second antigen-binding portion is selected from the nucleotide sequences of SEQ ID NOs: 113, 118, 121, 124, 127, 130, 133, 137, 139, 141 or any variant thereof; and/or the nucleic acid encoding the second light chain variable region of the second antigen-binding portion is selected from the nucleotide sequences of SEQ ID NOs: 94, 99, 101, 105, 107, 110 or any variant thereof.

In some preferred embodiments, the nucleic acid encoding the second heavy chain variable region of the second antigen-binding portion is the nucleotide sequence of SEQ ID NO: 137; and the nucleic acid encoding the second light chain variable region is the nucleotide sequence of SEQ ID NO: 94.

In some preferred embodiments, the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 94.

In some preferred embodiments, the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 99.

In some preferred embodiments, the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 101.

In some preferred embodiments, the nucleic acid encoding the second heavy chain variable region of the second antigen-binding portion is the nucleotide sequence of SEQ ID NO: 113; and the nucleic acid encoding the second light chain variable region is the nucleotide sequence of SEQ ID NO: 107.

In some preferred embodiments, the nucleic acid encoding the second heavy chain variable region of the second antigen-binding portion is the nucleotide sequence of SEQ ID NO: 137; and the nucleic acid encoding the second light chain variable region is the nucleotide sequence of SEQ ID NO: 107.

In some preferred embodiments, the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107.

In some embodiments, the nucleic acid encoding the second heavy chain of the second antigen-binding portion is the nucleotide sequence of SEQ ID NO: 183 or any variant thereof; and/or the nucleic acid encoding the second light chain of the second antigen-binding portion is the nucleotide sequence of SEQ ID NO: 181 or any variant thereof.

In some preferred embodiments, the nucleic acid encoding the first heavy chain variable region of the first antigen-binding portion of the bispecific antibody is the nucleotide sequence of SEQ ID NO: 146; and the nucleic acid encoding the first light chain variable region is the nucleotide sequence of SEQ ID NO: 143; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107.

In some preferred embodiments, the nucleic acid encoding the first heavy chain variable region of the first antigen-binding portion of the bispecific antibody is the nucleotide sequence of SEQ ID NO: 151; and the nucleic acid encoding the first light chain variable region is the nucleotide sequence of SEQ ID NO: 149; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107.

In some preferred embodiments, the nucleic acid encoding the first heavy chain variable region of the first antigen-binding portion of the bispecific antibody is the nucleotide sequence of SEQ ID NO: 155; and the nucleic acid encoding the first light chain variable region is the nucleotide sequence of SEQ ID NO: 153; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107.

In some preferred embodiments, the nucleic acid encoding the first heavy chain variable region of the first antigen-binding portion of the bispecific antibody is the nucleotide sequence of SEQ ID NO: 159; and the nucleic acid encoding the first light chain variable region is the nucleotide sequence of SEQ ID NO: 157; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107.

In some preferred embodiments, the nucleic acid encoding the first heavy chain variable region of the first antigen-binding portion of the bispecific antibody is the nucleotide sequence of SEQ ID NO: 163; and the nucleic acid encoding the first light chain variable region is the nucleotide sequence of SEQ ID NO: 161; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107.

In some preferred embodiments, the nucleic acid encoding the first heavy chain variable region of the first antigen-binding portion of the bispecific antibody is the nucleotide sequence of SEQ ID NO: 167; and the nucleic acid encoding the first light chain variable region is the nucleotide sequence of SEQ ID NO: 165; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107.

In some preferred embodiments, the nucleic acid encoding the first heavy chain variable region of the first antigen-binding portion of the bispecific antibody is the nucleotide sequence of SEQ ID NO: 171; and the nucleic acid encoding the first light chain variable region is the nucleotide sequence of SEQ ID NO: 169; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107.

In some preferred embodiments, the nucleic acid encoding the second heavy chain of the second antigen-binding portion is the nucleotide sequence of SEQ ID NO: 183, and the nucleic acid encoding the second light chain of the second antigen-binding portion is the nucleotide sequence of SEQ ID NO: 181.

In some preferred embodiments, the nucleic acid encoding the first heavy chain of the first antigen-binding portion of the bispecific antibody is the nucleotide sequence of SEQ ID NO: 175, and the nucleic acid encoding the first light chain of the first antigen-binding portion is the nucleotide sequence of SEQ ID NO: 173, the nucleic acid encoding the second heavy chain of the second antigen-binding portion is the nucleotide sequence of SEQ ID NO: 183, and the nucleic acid encoding the second light chain of the second antigen-binding portion is the nucleotide sequence of SEQ ID NO: 181.

In some preferred embodiments, the nucleic acid encoding the first heavy chain of the first antigen-binding portion of the bispecific antibody is the nucleotide sequence of SEQ ID NO: 175, and the nucleic acid encoding the first light chain of the first antigen-binding portion is the nucleotide sequence of SEQ ID NO: 177, the nucleic acid encoding the second heavy chain of the second antigen-binding portion is the nucleotide sequence of SEQ ID NO: 183, and the nucleic acid encoding the second light chain of the second antigen-binding portion is the nucleotide sequence of SEQ ID NO: 181.

In some preferred embodiments, the nucleic acid encoding the first heavy chain of the first antigen-binding portion of the bispecific antibody is the nucleotide sequence of SEQ ID NO: 179, and the nucleic acid encoding the first light chain of the first antigen-binding portion is the nucleotide sequence of SEQ ID NO: 173, the nucleic acid encoding the second heavy chain of the second antigen-binding portion is the nucleotide sequence of SEQ ID NO: 183, and the nucleic acid encoding the second light chain of the second antigen-binding portion is the nucleotide sequence of SEQ ID NO: 181.

In some preferred embodiments, the nucleic acid encoding the first heavy chain of the first antigen-binding portion of the bispecific antibody is the nucleotide sequence of SEQ ID NO: 179, and the nucleic acid encoding the first light chain of the first antigen-binding portion is the nucleotide sequence of SEQ ID NO: 177, the nucleic acid encoding the second heavy chain of the second antigen-binding portion is the nucleotide sequence of SEQ ID NO: 183, and the nucleic acid encoding the second light chain of the second antigen-binding portion is the nucleotide sequence of SEQ ID NO: 181.

In one aspect, the present disclosure provides a BCMA-binding antibody or antigen-binding portion thereof, the antibody comprises a first antigen-binding portion as previously described.

In one aspect, the present disclosure provides a vector comprising the foregoing nucleic acids.

In one aspect, the present disclosure provides a cell comprising the foregoing nucleic acids or vector.

In one aspect, the present disclosure provides compositions comprising the foregoing bispecific antibodies or antigen-binding portions thereof, nucleic acids, vectors, and/or cells.

In one aspect, the present disclosure provides an antibody-drug conjugate comprising the foregoing bispecific antibody or antigen-binding portion thereof covalently attached to a therapeutic moiety.

Preferably, the therapeutic moiety is selected from a cytotoxic moiety, a chemotherapeutic agent, a cytokine, an immunosuppressant, an immunostimulant, a lytic peptide, or a radioisotope.

The antibodies of the present disclosure are useful as therapeutic or diagnostic tools in a variety of diseases in which BCMA is adversely expressed or found.

In one embodiment of a disease associated with BCMA, the expression of BCMA in cells of the diseased tissue or organ is increased, as compared to the state in a healthy tissue or organ. An increase means an increase of at least 10%, in particular at least 20%, at least 50%, at least 100%, at least 200%, at least 500%, at least 1000%, at least 10000%, or even more. In one embodiment, expression is found only in diseased tissues, whereas expression in corresponding healthy tissues is suppressed. According to the present disclosure, diseases associated with BCMA include B-cell diseases, e.g. B-cell disorders such as plasma cell disorders and/or autoimmune diseases.

In some embodiments, the disease condition is cancer. In some embodiments, the cancer is a B-cell related cancer selected from the group consisting of multiple myeloma, malignant plasmacytoma, Hodgkin lymphoma, nodular lymphocyte predominant Hodgkin lymphoma, kahler's disease and myeloid leukemia, plasma cell leukemia, plasmacytoma, B-cell prolymphocytic leukemia, hairy cell leukemia, B-cell non-Hodgkin lymphoma (NHL), acute myelogenous leukemia (AML), chronic lymphocytic leukemia (CLL), acute lymphocytic leukemia (ALL), chronic myelogenous leukemia (CML), follicular lymphoma, burkitt lymphoma, marginal zone lymphoma, mantle cell lymphoma, large cell lymphoma, precursor B-lymphocyte lymphoma, myeloid leukemia, waldenstrom's macroglobulinaemia, diffuse large B cell lymphoma, follicular lymphoma, marginal zone lymphoma, mucosa-associated lymphoid tissue lymphoma, small cell lymphocytic lymphoma, mantle cell lymphoma, burkitt lymphoma, primary mediastinal (thymic) large B cell lymphoma, lymphoplasmacytic lymphoma, waldenstrom' s macroglobulinaemia, nodal marginal zone B cell lymphoma, splenic marginal zone lymphoma, intravascular large B-cell lymphoma, primary effusion lymphoma, lymphomatoid granulomatosis, large B-cell lymphoma rich in T cells/histiocytes, primary central nervous system lymphoma, primary cutaneous diffuse large B-cell lymphoma (leg type), EBV-positive diffuse large B-cell lymphoma of the elderly, diffuse large B-cell lymphoma associated with inflammation, intravascular large B-cell lymphoma, ALK-positive large B-cell lymphoma, plasmablastic lymphoma, large B-cell lymphoma arising in HHV8-associated multicenter Castleman's disease, unclassified B-cell lymphoma with intermediate features between diffuse large B-cell lymphoma and Burkitt' s lymphoma, unclassified B-cell lymphoma with intermediate features between diffuse large B-cell lymphoma and classic Hodgkin lymphoma, and other B-cell-related lymphomas.

In plasma cell disorders, one plasma cell clone reproduces uncontrollably. As a result, this clone produces a large number of single (monoclonal) antibodies called M-proteins. In some cases, for example, a subject with monoclonal gammopathy, the antibody produced is incomplete, consisting only of light chains or heavy chains. These abnormal plasma cells and the produced antibodies thereof are generally limited to one type. Preferably, the plasma cell disorder is selected from multiple myeloma, plasmacytoma, plasma cell leukemia, macroglobulinaemia, amyloidosis, waldenstrom's macroglobulinaemia, solitary plasmacytoma of the bone, extramedullary plasmacytoma, osteosclerotic myeloma, heavy chain disease, monoclonal gammopathy of indetermined significance, and multiple myeloma of stasis type.

In some embodiments, the disease condition is an autoimmune disorder, such as systemic lupus erythematosus or rheumatoid arthritis. In some embodiments, the therapeutic agent comprises an antibody that specifically binds to an activated T-cell antigen.

In one embodiment, the therapeutic agent comprises an antibody that specifically binds to CD3, particularly CD3ε.

Methods of treating diseases and conditions using bispecific antibodies of the present disclosure include the steps of administering to a mammal a therapeutically effective amount of an antibody or antigen-binding fragment thereof or a nucleic acid molecule or vector or cell or pharmaceutical composition of any of the preceding aspects.

In some embodiments, the present disclosure provides a method of treating or preventing a cancer disease comprising administering to a patient an antibody capable of binding to BCMA, wherein the antibody is administered to provide a serum level of at least 40 μg/ml. In various embodiments, the antibody is administered to provide a serum level of at least 50 μg/ml, at least 150 μg/ml, at least 300 μg/ml, at least 400 μg/ml, or at least 500 μg/ml. In various embodiments, the antibody is administered to provide a serum level of no more than 800 μg/ml, 700 μg/ml, 600 μg/ml, 550 μg/ml, or 500 μg/ml. In one embodiment, the serum level provided is from 40 μg/ml to 700 μg/ml, preferably from 40 μg/ml to 600 μg/ml, preferably from 50 μg/ml to 500 μg/ml, such as from 150 μg/ml to 500 μg/ml or from 300 μg/ml to 500 μg/ml. The term "serum level" as used in the present description means the concentration of the discussed substance in serum. In one embodiment, serum levels are provided for at least 7 days or at least 14 days. In one embodiment, the method comprises administering a dose of the antibody of at least 300 mg/m$^2$, such as at least 600 mg/m$^2$, and preferably at most 1500 mg/m$^2$, at most 1200 mg/m$^2$, or at most 1000 mg/m$^2$. In some embodiments, the disclosure provides a method of treating or preventing a cancer disease, comprising administering to a patient an antibody capable of binding to BCMA, wherein the antibody is administered at a dose of at least 300 mg/m$^2$, such as at least 600 mg/m$^2$, and preferably at most 1500 mg/m$^2$, at most 1200 mg/m$^2$, or at most 1000 mg/m$^2$. In some embodiments, the disclosure provides a method of treating or preventing a cancer disease comprising administering to a patient an antibody capable of binding to BCMA, wherein at least 50%, preferably 60%, 70%, 80%, or 90% of the cancer cells of the patient are positive for BCMA and/or at least 40%, preferably 50% or 60% of the cancer cells of the patient are positive for surface expression of BCMA. In this aspect, the present disclosure also provides a method of treating or preventing a cancer disease, the method comprises: a. identifying patients showing at least 50%, preferably 60%, 70%, 80%, or 90% of BCMA-positive cancer cells and/or at least 40%, preferably 50% or 60% of cancer cells that are positive for surface expression of BCMA; and b. administering to the patient an antibody capable of binding to BCMA. In one embodiment, at least 95% or at least 98% of the cancer cells of the patient are BCMA-positive. In one embodiment, at least 70%, at least 80%, or at least 90% of the cancer cells of the patient are positive for surface expression of BCMA.

In one embodiment of the methods of any of the aspects described herein, the therapeutic outcome of the cancer disease is the achievement of stable disease conditions. In one embodiment, stable disease conditions are achieved for at least 2 months, at least 3 months, or at least 6 months.

In some embodiments, the present disclosure provides methods of achieving stable disease in a cancer patient comprising administering to a patient an antibody capable of binding to BCMA. In one embodiment, stable disease is achieved for at least 2 months, at least 3 months, or at least 6 months.

In one embodiment of the method of any of the aspects described herein, the antibody is administered in a single dose or multiple doses.

In some embodiments, the present disclosure provides a method of treating or preventing a cancer disease comprising administering to a patient an antibody capable of binding to BCMA, wherein the antibody is administered in multiple doses.

If the antibody is administered in multiple doses according to the present disclosure, the antibody is preferably administered in at least 3 doses, at least 4 doses, at least 5 doses, at least 6 doses, at least 7 doses, at least 8 doses, at least 9 doses, or at least 10 doses, and preferably at most 30 doses, 25 doses, 20 doses, 15 doses, or 10 doses. Preferably, the dose of antibody is administered at intervals of at least 7 days, at least 10 days, at least 14 days, or at least 20 days. The dose of antibody is preferably administered at intervals of from 7 to 30 days, from 10 to 20 days, and preferably about 14 days.

In one embodiment, the antibody is administered so as to provide a serum level of at least 40 μg/ml. In various embodiments, the antibody is administered so as to provide a serum level of at least 50 μg/ml, at least 150 μg/ml, at least 300 μg/ml, at least 400 μg/ml, or at least 500 μg/ml. In various embodiments, the antibody is administered so as to provide a serum level of no more than 800 μg/ml, 700 μg/ml, 600 μg/ml, 550 μg/ml, or 500 μg/ml.

In one embodiment, the serum level provided is from 40 μg/ml to 700 μg/ml, preferably from 40 μg/ml to 600 μg/ml, preferably from 50 μg/ml to 500 μg/ml, such as from 150 μg/ml to 500 μg/ml or from 300 μg/ml to 500 μg/ml. In one embodiment, serum levels are provided for at least 7 days or at least 14 days. In one embodiment, the method comprises administering a dose of at least 300 mg/m$^2$, such as at least 600 mg/m$^2$ and preferably at most 1500 mg/m$^2$, at most 1200 mg/m$^2$, or at most 1000 mg/m$^2$ of the antibody.

Use of an antibody or antigen-binding fragment thereof or a nucleic acid molecule or a vector or a cell or a pharmaceutical composition of any of the preceding aspects in the manufacture of a drug for treating a BCMA-related disorder in a mammal.

According to any of the preceding aspects, optionally, the antibody is conjugated to other drugs, such as a labeled or cytotoxic conjugate.

In one aspect, the disclosure also includes kits, e.g. kits comprising an antibody, fragment thereof, homologue, derivative, etc. of the disclosure, e.g. a labeled or cytotoxic conjugate, as well as instructions for use of the antibody, a conjugate that kills a particular type of cell, etc. The instructions can include directions for using the antibody, conjugate, etc. in vitro, in vivo, or ex vivo. The antibodies may be in liquid form or solid form, usually lyophilized. The kit may contain other suitable reagents, such as buffers, reconstitution solutions, and other necessary ingredients for the intended use. Combinations of reagents packaged in predetermined amounts with instructions for their use, e.g. for therapeutic use or for conducting diagnostic assays, are contemplated. When the antibody is labeled, e.g. with an enzyme, then the kit can include a substrate and cofactors required for the enzyme (e.g. a substrate precursor that provides a detectable chromophore or fluorophore). In addition, other additives such as stabilizers, buffers (e.g. blocking buffers or lysis buffers), and the like may also be included. The relative amounts of the various reagents can be varied to provide a concentrate of reagent solution, thereby providing user flexibility, space savings, reagent savings, etc. These reagents may also be provided in dry powder form, usually in lyophilized form, including excipients which provide a reagent solution having the appropriate concentration when dissolved.

Use of an antibody or functional fragment thereof or a nucleic acid molecule or a vector or a cell or a pharmaceutical composition or kit according to any of the preceding aspects for the preparation of a reagent for inhibiting BCMA binding.

In addition, the antibodies of the present disclosure can be used in immunoassays, purification methods, and other methods using immunoglobulins or fragments thereof. Such uses are well-known in the art.

Accordingly, the present disclosure also provides compositions comprising the anti-BCMA antibodies of the present disclosure or fragments thereof, the antibody is conveniently combined with a pharmaceutically acceptable carrier, diluent, or excipient, which belongs to conventional practices in the art.

As used in the present disclosure, the term "pharmaceutical composition" refers to formulations of various prepared materials. Formulations containing a therapeutically effective amount of multivalent antibodies are in a sterile liquid solution, liquid suspension, or lyophilized form, optionally containing stabilizers or excipients.

The antibodies of the present disclosure can be used as a composition administered alone or can be used in combination with other active agents.

In some embodiments, the humanized antibodies of the disclosure are conjugated to a therapeutic moiety (i.e. a drug). The therapeutic moiety can be, for example, a cytotoxin, a chemotherapeutic agent, a cytokine, an immunosuppressant, an immunostimulant, a lytic peptide, or a radioisotope. Such conjugates are referred to herein as "antibody-drug conjugates" or "ADC".

In some embodiments, the antibody is conjugated to a cytotoxic moiety. The cytotoxic moiety may for example be selected from the following: paclitaxel; cytochalasin B; gramicidin D; ethidium bromide; emetine; mitomycin; etoposide; teniposide; vincristine; vinblastine; colchicine; doxorubicin; daunorubicin; dihydroxy anthracenedione; tubulin inhibitors such as maytansine or analogs or derivatives thereof; antimitotic agents such as monomethyl auristatin E or F or analogues or derivatives thereof; dolastatin 10 or 15 or an analogue thereof; irinotecan or an analog thereof; mitoxantrone; mithramycin; actinomycin D; 1-dehydrotestosterone; glucocorticoids; procaine; tetracaine; lidocaine; propranolol; puromycin; calicheamicin or an analogue or derivative thereof; antimetabolites such as methotrexate, 6 mercaptopurine, 6 thioguanine, cytarabine, fludarabine, 5 fluorouracil, decadiazine, hydroxyurea, asparaginase, gemcitabine or cladribine; alkylating agents such as mechlorethamine, thiopurine, chlorambucil, melphalan, carmustine (BSNU), lomustine (CCNU), cyclophosphamide, busulfan, dibromomannitol, streptozotocin, dacarbazine (DTIC), procarbazine, mitomycin C; platinum derivatives such as cisplatin or carboplatin; duocarmycin A, duocarmycin SA, rachelmycin (CC-1065) or analogs or derivatives thereof; antibiotics such as actinomycin, bleomycin, daunorubicin, doxorubicin, idarubicin, mithramycin, mitomycin, mitoxantrone, plicomycin, anthramycin (AMC); pyrrolo [2,1-c] [1,4]-benzodiazepine (PDB); diphtheria toxin and related molecules such as diphtheria A chain and active fragments thereof and hybrid molecules, ricin toxin such as ricin A or deglycosylated ricin A chain toxin, cholera toxin, Shiga-like toxin such as SLT I, SLT II, SLT IIV, LT toxin, C3 toxin, Shiga toxin, pertussis toxin, tetanus toxin, soybean Bowman-Birk protease inhibitor, pseudomonas exotoxin, azlocillin, saporin, modeccin, gelsolin, abrin A chain, modeccin A chain, $\alpha$-sarcin, aleurites fordii proteins, dianthin proteins, Phytolacca americana proteins such as PAPI, PAPII, and PAP-S, momordica charantia inhibitor, curcin, crotin, sapaonaria officinalis inhibitor, gelonin, mitomycin, restrictocin, phenomycin, and enomycin toxins; ribonuclease (RNase); DNase I, staphylococcal endotoxin A; pokeweed antiviral protein; diphtheria toxin and pseudomonas endotoxin.

In some embodiments, the antibody is conjugated to auristatin or a peptide analog, derivative, or prodrug thereof. It has been shown that auristatin interferes with microtubule dynamics, GTP hydrolysis, and nuclear and cell division and has anti-cancer and anti-fungal activity. For example, auristatin E can be reacted with p-acetylbenzoic acid or benzoylpentanoic acid to produce AEB and AEVB, respectively. Other typical auristatin derivatives include AFP, MMAF (monomethyl auristatin F), and MMAE (monomethyl auristatin E). Suitable auristatins and analogs, derivatives, and prodrugs of auristatins, as well as suitable linkers for conjugating auristatins to Ab, are described, for example, in U.S. Patent Nos. 5,635,483, 5,780,588, and 6,214,345, and International Patent Application Publication Nos. WO02088172, WO2004010957, WO2005081711, WO2005084390, WO2006132670, WO03026577, WO200700860, WO207011968 and WO205082023.

In some embodiments, the antibody is conjugated to a pyrrolo [2,1-c] [1,4]-benzodiazepine (PDB) or a peptide analog, derivative, or prodrug thereof. Suitable PDB and PDB derivatives and related art are described, for example, in Hartley J.A. et al., Cancer Res 2010; 70(17):6849-6858; Antonow D. et al., Cancer J 2008; 14(3):154-169; Howard P.W. et al., Bioorg Med Chem Lett 2009; 19:6463-6466 and Sagnou et al., Bioorg Med Chem Lett 2000; 10(18):2083-2086.

In some embodiments, the antibody is conjugated to a cytotoxic moiety selected from anthracyclines, maytansines, calicheamicins, duocarmycins, rachelmycin (CC-1065), dolastatin 10, dolastatin 15, irinotecan, monomethyl auristatin E, monomethyl auristatin F, PDB, or any analog, derivative or prodrug thereof.

In some embodiments, the antibody is conjugated to anthracycline or an analog, derivative, or prodrug thereof. In some embodiments, the antibody is conjugated to maytansine or an analog, derivative, or prodrug thereof. In some embodiments, the antibody is conjugated to a calicheamicin or an analog, derivative, or prodrug thereof. In some embodiments, the antibody is conjugated to a duocarmycin or an analog, derivative, or prodrug thereof. In some embodiments, the antibody is conjugated to rachelmycin (CC-1065) or an analog, derivative, or prodrug thereof. In some embodiments, the antibody is conjugated to dolastatin 10 or an analog, derivative, or prodrug thereof. In some embodiments, the antibody is conjugated to dolastatin 15 or an analog, derivative, or prodrug thereof. In some embodiments, the antibody is conjugated to monomethyl auristatin E or an analog, derivative, or prodrug thereof. In some embodiments, the antibody is conjugated to monomethyl auristatin F or an analog, derivative, or prodrug thereof. In some embodiments, the antibody is conjugated to a pyrrolo [2,1-c] [1,4]-benzodiazepine or an analog, derivative, or prodrug thereof. In some embodiments, the antibody is conjugated to irinotecan or an analog, derivative, or prodrug thereof.

In some embodiments, the antibody is conjugated to a cytokine (e.g. IL-2, IL-4, IL-6, IL-7, IL-10, IL-12, IL-13, IL-15, IL-18, IL-23, IL-24, IL-27, IL-28a, IL-28b, IL-29, KGF, IFN$\alpha$, IFN$\beta$, IFN$\gamma$, GM-CSF, CD40L, Flt3 ligand, stem cell factor, ancestim, and TNF$\alpha$).

In some embodiments, the antibody is conjugated to a radioisotope or a chelate containing a radioisotope. For example, the antibody can be conjugated to a chelator linker (e.g. DOTA, DTPA, or thirace) that allows the antibody to complex with the radioisotope. The antibody may also or alternatively comprise or be conjugated to one or more radiolabeled amino acids or other radiolabeled molecules. Non-limiting examples of radioisotopes include $^3$H, $^{14}$C, $^{15}$N, $^{35}$S, $^{90}$Y, $^{99}$Tc, $^{125}$I, $^{131}$I, $^{186}$Re, $^{213}$Bi, $^{225}$Ac and $^{227}$Th. For therapeutic purposes, radioisotopes emitting $\beta$ or $\alpha$ particle radiation may be used, such as $^{131}$I, $^{90}$Y, $^{211}$At, $^{212}$Bi, $^{67}$Cu, $^{186}$Re, $^{188}$Re, and $^{212}$Pb.

Techniques for coupling molecules to antibodies are well-known in the art. Typically, the nucleic acid molecule is covalently linked to a lysine or cysteine on the antibody via an N-hydroxysuccinimide ester or maleimide functional group, respectively. Conjugation methods using engineered cysteines or incorporating unnatural amino acids have been reported to improve the homogeneity of conjugates. In particular, those skilled in the art may also predict that reactive endogenous glutamine-engineered Fc-containing polypeptides are generated by use of acyl donor glutamine-containing tags (e.g. Gln peptide-containing tags or Q-tags) or by polypeptide engineering (e.g. by amino acid deletions, insertions, substitutions, or mutations in the polypeptide). Transglutaminase can then be covalently crosslinked with amine donors (e.g., small molecules containing or linked to reactive amines) to form stable and homogeneous groups of engineered polypeptides containing Fc, wherein amine donors are specifically coupled to polypeptides containing Fc through glutamine tags containing acyl donor or accessible / exposure / reactive endogenous glutamine sites (WO2012059882).

It should be understood that the therapeutic agents according to the above embodiments will be administered together with suitable pharmaceutically acceptable carriers, excipients, and other reagents incorporated into the formulation to provide improved transfer, delivery, tolerability, etc. A large number of suitable formulations can be found in all pharmacopoeias known to pharmaceutical chemists: Remington's Pharmaceutical Sciences (15th edition, Mack Publishing Company, Easton, Pa. (1975)), in particular Chapter 87 of Blaug, Seymour therein. These formulations include, for example, powders, pastes, ointments, gels, waxes, oils, lipids, lipid-containing (cationic or anionic) carriers (e.g. Lipofectin™), DNA conjugates, anhydrous syrups, oil-in-water and water-in-oil emulsions, emulsion polyethylene glycols (polyethylene glycols of various molecular weights), semi-solid gels, and semi-solid mixtures containing polyethylene glycol. Any of the foregoing mixtures may be suitable for treatments or therapies of the present disclosure, provided that the active ingredients in the formulation are not inactivated by the formulation and that the formulation is physiologically compatible and tolerates the routes of administration.

In one embodiment, the antibody can be used as a therapeutic agent. Such reagents will generally be used to treat, alleviate and/or prevent a disease or pathology associated with aberrant BCMA expression, activity, and/or signaling in a subject. Treatment regimens can be performed using standard methods by identifying a subject, e.g. a human patient, having (or at risk of or developing) a disease or disorder associated with aberrant BCMA expression, activity, and/or signaling, e.g. a BCMA-related disorder. An antibody preparation, preferably one preparation with high specificity and affinity for its target antigen, is administered to a subject and will generally have an effect due to its binding to the target. The administered antibody can eliminate or inhibit or interfere with the expression, activity, and/or signaling function of the target (e.g. BCMA). The administered antibody can eliminate or inhibit or interfere with the binding of the target (e.g. BCMA) to the endogenous ligand to which it naturally binds. For example, an antibody binds to a target, and modulates, blocks, inhibits, reduces, antagonizes, neutralizes, and/or otherwise interferes with BCMA expression, activity, and/or signaling. In some embodiments, in order to treat a disease or disorder associated with aberrant BCMA expression, an antibody having a heavy chain and light chain CDR can be administered to a subject.

In another embodiment, antibodies directed against BCMA can be used in methods known in the art and related to localization and/or quantitation of BCMA (e.g. for determining BCMA and/or levels of BCMA in an appropriate physiological sample, for diagnostic methods, for protein imaging, etc.). In a given embodiment, an antibody comprising an antibody-derived antigen-binding domain specific for BCMA, or a derivative, fragment, analog, or homolog thereof, is used as a pharmaceutically active compound (hereinafter "therapeutic agent ").

In another embodiment, BCMA polypeptides can be isolated using antibodies specific for BCMA by standard techniques such as immunoaffinity, chromatography, or immunoprecipitation. Antibodies (or fragments thereof) directed against BCMA proteins can be used to detect proteins in biological samples. In some embodiments, BCMA can be detected in a biological sample as part of a clinical testing procedure, for example, to determine the efficacy of a given treatment regimen. Detection may be facilitated by coupling (i.e. physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, $\beta$-galactosidase or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazine aminofluorescein, dansyl chloride, or phycoerythrin; one example of a luminescent material includes luminol; examples of bioluminescent materials include luciferase, luciferin, and aequorin, and examples of suitable radioactive materials include $^{125}$I, $^{131}$I, $^{35}$S or $^{3}$H.

In another embodiment, antibodies according to the present disclosure can be used as reagents for detecting the presence of BCMA or protein fragments thereof in a sample. In some embodiments, the antibody comprises a detectable label. The antibody is a polyclonal antibody, or more preferably a monoclonal antibody. Whole antibodies or fragments thereof (e.g. Fab, scFv, or F(ab')$_2$) are used. The term "labeling" with respect to an antibody is intended to encompass direct labeling of the antibody by coupling (i.e. physically linking) a detectable substance to the antibody, as well as indirect labeling of the antibody by reaction with another reagent that is directly labeled. Examples of indirect labeling include the detection of the first antibody using a fluorescently labeled second antibody, and end-labeling of the antibody with biotin so as to enable detection with fluorescently labeled streptavidin. The term "biological sample" is intended to include tissues, cells, and biological fluids isolated from a subject, as well as tissues, cells, and fluids present in a subject. Thus, the term "biological sample" as used includes blood and fractions or components of blood, including serum, plasma, or lymph. In other words, the detection method of the above embodiments can be used to detect the analyte mRNA, protein, or genomic DNA in a biological sample in vitro as well as in vivo. For example, analyte mRNA in vitro detection techniques include Norhtern hybridization and in situ hybridization. In vitro techniques for the detection of analyte proteins include enzymelinked immunosorbent assays (ELISA), western blots, immunoprecipitations, and immunofluorescence. Analyte genomic DNA in vitro detection techniques including Southern hybridization. Procedures for performing immunoassays are described, for example, in "ELISA: Theory and Practice: Methods in Molecular Biology", vol. 42, J. R. Crowther (ed.) Human Press, Totowa, N. J., 1995; "Immunoassay", E. Diamandis and T. Christopoulus, Academic Press, Inc., San Diego, Calif., 1996; and "Practice and Theory of Enzyme Immunoassays", P. Tijssen, Elsevier Science Publishers, Amsterdam, 1985. In addition, in vivo techniques for the detection of an analyte protein include introducing into a subject a labeled anti-analyte protein antibody. For example, an antibody can be labeled with a radiolabel, and the presence and location of the radiolabel in the subject can then be detected by standard imaging techniques.

The antibodies and derivatives, fragments, analogs, and homologs thereof described herein can be incorporated into pharmaceutical compositions suitable for administration. The principles and considerations involved in preparing such compositions, as well as guidance in selecting components, are well known in the art, for example, see Remington's Pharmaceutical Sciences: The Science And Practice Of Pharmacy 19th edition (Alfonso R. Gennaro et al. eds.) Mack Pub. Co., Easton, Pa.: 1995; Drug Absorption Enhancement: Concepts, Possibilities, Limitations, And Trends, Harwood Academic Publishers, Langhorne, Pa., 1994; and Peptide And Protein Drug Delivery (Advances In Parenteral Sciences, vol. 4), 1991, M. Dekker, New York.

Such compositions typically comprise an antibody and a pharmaceutically acceptable carrier. When antibody fragments are used, minimal inhibitory fragments that specifically bind to the target protein binding domain may be preferred. For example, based on the variable region sequence of an antibody, peptide molecules can be designed so as to retain the ability to bind to a target protein sequence. Such peptides can be chemically synthesized and/or produced by recombinant DNA techniques (see, e.g. Marasco et al. Proc. Natl. Acad. Sci. USA, 90: 7889-7893 (1993)).

As used herein, the term "pharmaceutically acceptable carrier" is intended to include any and all solvents, dispersion media, coatings, antibacterial and antifungal agents, isotonic and absorption delaying agents, and the like, compatible with pharmaceutical administration. Suitable pharmaceutically acceptable carriers are described in the latest edition of Remington's Pharmaceutical Sciences, a standard reference text in the art, which is incorporated herein by reference. Preferred examples of such carriers or diluents include, but are not limited to, water, saline, ringer's solution, dextrose solution, and 5% human serum albumin. Liposomes and non-aqueous vehicles such as fixed oils can also be used. It is well-known in the art that such media and reagents are used as pharmaceutically active substances. Except insofar as any conventional media or reagent is incompatible with the antibody, its use in the compositions is contemplated.

The pharmaceutical compositions of the above embodiments are formulated to be compatible with their intended routes of administration. Examples of routes of administration include parenteral, e.g. intravenous, intradermal, subcutaneous, oral (e.g. inhalation), transdermal (i.e. topical), transmucosal, and rectal administration. Solutions or suspensions for parenteral, intradermal, or subcutaneous administration may include the following components: sterile diluents for injection such as water, saline solutions, fixed oils, polyethylene glycols, glycerol, propylene glycol or other synthetic solvents; antibacterial agents such as benzyl alcohol or methyl 4-hydroxybenzoate; antioxidants such as ascorbic acid or sodium bisulfite; chelators such as ethylene diamine tetraacetic acid (EDTA); buffers, such as acetates, citrates or phosphates, and reagents to adjust the osmotic pressure, such as sodium chloride or dextrose. The pH can be adjusted with acids or bases, such as hydrochloric acid or sodium hydroxide. The parenteral preparation can be enclosed in ampoules, disposable syringes, or multiple dose vials made of glass or plastic.

Pharmaceutical compositions suitable for injection use include sterile aqueous solutions (where water is soluble) or dispersions and sterile powders for the extemporaneous preparation of sterile injectable solutions or dispersion. For intravenous administration, suitable pharmaceutically acceptable carriers include physiological saline, bacteriostatic water, Cremophor EL™ (BASF, Parsippany, N.J.), or phosphate-buffered saline (PBS). In all cases, the composition must be sterile and should have a fluidity to the extent that is easy for injection . It must be stable under the conditions of manufacture and storage, and must be preserved against the contaminating action of microorganisms such as bacteria and fungi. The carrier can be a solvent or dispersion medium containing, for example, water, ethanol, polyol (for example, glycerol, propylene glycol, liquid polyethylene glycol, and the like), and suitable mixtures thereof. The proper fluidity can be maintained, for example, by use of a coating such as lecithin, by the maintenance of the required particle size in the case of dispersion, and by use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyols (such as mannitol, and sorbitol), and sodium chloride in the composition. Prolonged absorption of injectable compositions can be brought about by including in the composition a reagent that delays absorption, for example, aluminum monostearate and gelatin.

If needed, sterile injectable solutions can be prepared by incorporating the antibody in a required amount in an appropriate solvent with one or a combination of ingredients enumerated above (as required) followed by filtered sterilization. Generally, dispersions are prepared by incorporating the antibody into a sterile vehicle that contains a basic dispersion medium and the required other ingredients from those enumerated above. In the case of sterile powders for the preparation of sterile injectable solutions, preparation methods are vacuum drying and freeze-drying that obtain a powder, the powder includes the active ingredient and any additional desired ingredient from a previously sterilefiltered solution of such ingredients.

For inhaled administration, the compound is delivered in the form of an aerosol spray from a pressurized container or distributor or sprayer containing suitable propellants such as carbon dioxide.

Systemic administration may also be performed by transmucosal or transdermal ways. For transmucosal or transdermal administration, a penetrant suitable for a permeation barrier should be used in the preparation. Such penetrants are generally known in the art, and include, for example, transmucosal administration, detergents, bile salts, and fusidic acid derivatives. Transmucosal administration can be accomplished by use of nasal sprays or suppositories. For transdermal administration, one or more of the antibodies can be formulated into ointments, salves, gels, or creams as generally known in the art.

The compounds may also be prepared in a form of suppositories (e.g. with conventional suppository bases such as cocoa butter or other glycerides) or retention enemas for rectal delivery.

In one embodiment, the antibodies can be prepared with carriers that prevent their rapid elimination from the body, such as sustained/controlled release formulations, including implants and microencapsulated delivery systems. Biodegradable, biocompatible polymers can be used, such as ethylene-vinyl acetate, polyanhydrides, polyglycolic acid, collagen, polyorthoesters, and polylactic acid. Methods for preparing such formulations will be apparent to those skilled in the art.

It is especially advantageous to formulate parenteral compositions in dosage unit form for ease of administration and uniformity of dosage. The dosage unit form, as used herein, refers to physically discrete units suited as unitary dosages for the subject to be treated; each unit contains a predetermined quantity of one or more antibodies calculated to produce the desired therapeutic effect in association with the required pharmaceutical carrier. The specification for the dosage unit forms of the above embodiments is dictated by and directly dependent on the unique characteristics of antibodies and the specific therapeutic effects to be achieved, and the limitations inherent in the art of formulating such antibodies for treating individuals.

The pharmaceutical compositions can be presented in a container, pack, or dispenser together with instructions for administration.

The formulations described herein may also contain more than one antibody depending on the particular situation to be treated, preferably those which have complementary activities but do not adversely affect each other. Alternatively, or additionally, the composition may, for example, comprise a reagent that enhances its function, such as a cytotoxic reagent, a cytokine, a chemotherapeutic agent, or a growth inhibitor. Such molecules are suitably present in combination in amounts effective for the intended purpose. For example, they may also be present in combination in a kit or in combination for use.

In one embodiment, one or more antibodies can be administered in a combination therapy, i.e. in combination with other reagents such as therapeutic agents that can be used to treat pathological conditions or disorders, such as various forms of cancer, autoimmune disorders, and inflammatory diseases. The term "in combination" means herein that the reagents are administered substantially synchronously, simultaneously, or sequentially. If administered sequentially, the first of the two compounds is still preferably detected at an effective concentration at the treatment site when the second compound is initially administered. In one instance, "in combination" can also include both an antibody of the present disclosure and another therapeutic agent in a kit.

For example, a combination therapy can comprise coformulation and/or coadministration of one or more antibodies described herein with one or more additional therapeutic agents (e.g. one or more cytokine and growth factor inhibitors, immunosuppressants, antiinflammatory agents, metabolic inhibitors, enzyme inhibitors, and/or cytotoxic or cytostatic agents, as described in more detail below). Such combination therapy may advantageously utilize lower dosages of the administered therapeutic agents, thus avoiding possible toxicities or complications associated with each monotherapy.

In one embodiment, the administration of the anti-BCMA antibody and the administration of the therapeutic agent can reduce the number of B cells in the subject.

In one embodiment, the treatment regimen is effective to reduce cytokine release associated with administration of the T cell activating therapeutic agent in the subject, as compared to a corresponding treatment regimen without administration of an anti-BCMA antibody.

For purposes of clarity and conciseness, features are described herein as part of the same or separate embodiments, however, it will be understood that the scope of the disclosure may include some embodiments having a combination of all or some of the features described.

**Examples**

**Example 1. Antigen expression and construction of stably transfected cells**

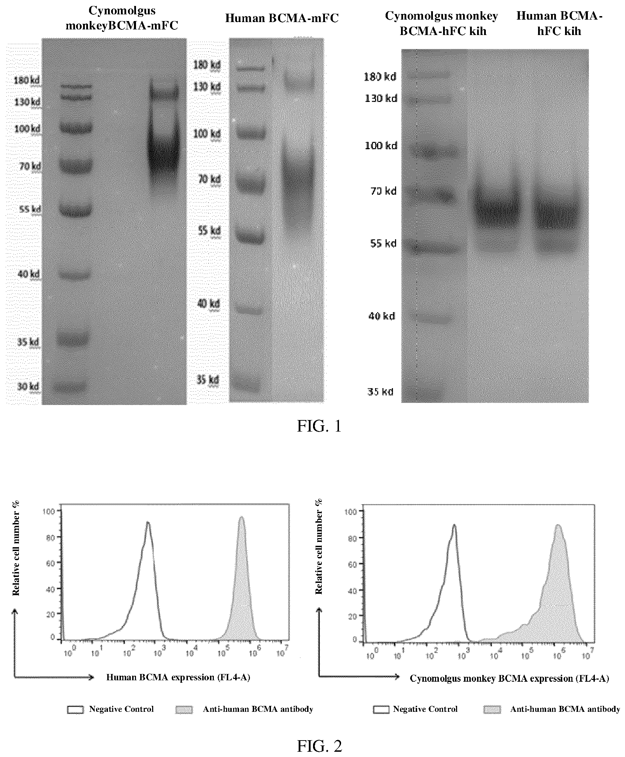

Using human BCMA (Sino biological, HG10620-M) and rhesus monkey BCMA (Sino biological, CG90103-G, identical with the extracellular region sequence of cynomolgus monkey XP_005591343.1) as templates, respectively, human BCMA (Met1-Asn53) or rhesus monkey BCMA (Met1-Asn52) were amplified, fused with mouse IgG2a Fc at C-terminal, and transfected into HEK293E cells to express the bivalent recombinant protein of human or cynomolgus monkey BCMA-mFc (the amino acid sequence shown in SEQ ID NOs. 1-2); or, fused with human IgG Fc (knob), and co-transfected into HEK293E cells with human IgG Fc (hole) by using knob-into-hole technology, the human or cynomolgus monkey BCMA-hFc kih monovalent recombinant protein (amino acid sequence shown in SEQ ID NOs. 3-5) was obtained. FIG. 1 shows SDS-PAGE results for human, cynomolgus monkey BCMA-mFc bivalent recombinant protein and BCMA-hFc kih monovalent recombinant protein.

The Lenti vector and packaging plasmid (Genecoepia) containing the full-length sequences of human and cynomolgus monkey BCMA were co-transfected into HEK293T cells. 48 h after transfection, the culture supernatant containing pseudoviruses was collected. 10 $\mu$L was used to infect $1 \times 10^6$ CHO cells, and different concentrations of puromycin were added to screen for resistance. Finally, stably transfected cells CHO-hBCMA (clone 2C2) and CHO-cynoBCMA (clone 1A2) highly expressing human or cynomolgus monkey BCMA were isolated. FIG. 2 shows that both human and monkey BCMA stably transfected cells detected by flow cytometry, both human BCMA stably transfected cells (CHO-hBCMA-2C2) and cynomolgus monkey BCMA stably transfected cells (CHO-cynoBCMA-1A2) express high levels of BCMA.

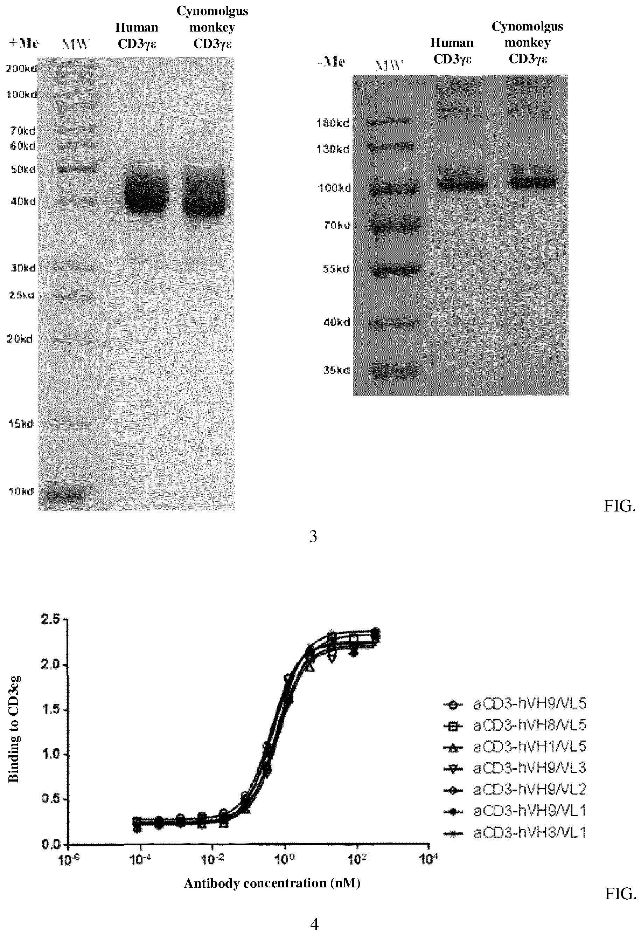

Construction of human or cynomolgus monkey CD3$\epsilon\gamma$ heterodimers: the nucleotide sequences of the extracellular regions of human CD3$\gamma$ (UniProt P09693, Gln23-Asn116) and CD3$\epsilon$ (UniProt P07766, Gln23-Asp126) are synthesized, fused with human IgG Fc hole or Fc knob respectively at the C-terminus, and to expressed to form a human CD3$\epsilon\gamma$-Fc heterodimer (the amino acid sequence of human CD3$\gamma$ IgG Fc (hole) is shown in SEQ ID NO. 6, and the amino acid sequence of human CD3$\epsilon$ IgG Fc (knob) is shown in SEQ ID NO. 7); cynomolgus monkey CD3$\gamma$ (UniProt Q95LI7, Gln23-Asn110) and CD3$\epsilon$ (UniProt Q95LI5, Gln22-Asp117) were also synthesized, C-terminus fused to cynomolgus monkey IgG Fc hole or Fc knob and expressed to form a cynomolgus monkey CD3γ-Fc heterodimer (the amino acid sequence of cynomolgus monkey CD3γ IgG Fc (hole) is shown in SEQ ID NO. 8 and the amino acid sequence of cynomolgus monkey CD3γ IgG Fc (knob) is shown in SEQ ID NO. 9). Recombinant plasmids expressing CD3γ-Fc and CD3ε-Fc were mixed with 3 mg/mL PEI (Polysciences, #24765-2) and co-transfected into HEK293E cells (culture medium OPM-293 CD03 DPM). After 7 days at 37°C under 120 rpm 5% $CO_2$, the culture supernatant was collected and purified by Protein A affinity chromatography to obtain human or cynomolgus monkey CD3εγ-Fc recombinant protein with an SDS-PAGE purity higher than 95% (FIG. 3).

**Example 2. Animal immunization and preparation of BCMA antibodies**

Six-week-old female Balb/c mice (Charles River Beijing) were immunized with BCMA-mFc recombinant protein or CHO-BCMA stably transfected cells as immunogens, mixed with an equal volume of immune adjuvant (Titermax, Sigma-Aldrich), respectively, by footpad or subcutaneous immunization. After the primary immunization, the same dose of booster immunization is given every two weeks, and antigen immunization of human and monkey is alternated. After four immunizations, BCMA antibody titers in mouse serum greater than 1:10000 were detected. Three days after tail vein rush immunization, the spleen and part of the peripheral lymph nodes of mice were harvested, and total RNA was extracted. Antibody cDNA was obtained by reverse transcription with IgG-specific primers. The light and heavy chain variable region fragments were amplified respectively, and the phage Fab library was constructed. The number of transformed colonies (pool capacity) was about $1.2 \times 10^{10}$.

**1. Screening of phage antibody libraries**

Using classical panning, after mixing 1 mL BCMA immune library phage with 3.5 mL of 2% BSA/PBS solution, Maxisorp Immunotube (Nunc) coated with human or cynomolgus monkey BCMA recombinant protein was added. After binding for 1 h, multiple washing cycles were performed with PBS-Tween (0.5% v/v) and PBS to remove non-specific or low affinity bound phage. High affinity bound phage was eluted with 100 mM triethylamine and neutralized to infect TG1 cells. After two rounds of screening, a single clone was picked and inoculated into a 96-well U-shaped plate, 1 mM IPTG was added to induce expression, the induced supernatant containing Fab was used for ELISA detection screening, and 265 positive clones were obtained through primary screening.

After re-induction of expression, 10 μg/ml of biotinylated, labeled human BCMA recombinant protein was immobilized on an SA probe for 200 s of binding time and 300 s of dissociation time and tested for affinity to Fab (Octet, Fortebio). Fifty-nine clones with higher affinity were selected to detect the binding to cell membrane surface receptors using human and cynomolgus monkey BCMA-CHO stably transfected cells, wherein, fifty-two clones could cross-recognize human and cynomolgus monkey BCMA-CHO stably transfected cells.

**2. Antibody affinity assay with Biacore**

Partial high-affinity antibody clones were selected, plasmids were extracted and transformed into TG1 cells, expression was induced overnight at 0.1 mM IPTG 30°C, recombinantly expressed Fab was extracted from a bacterial periplasmic cavity, and Fab antibody was purified by Ni-NTA affinity chromatography. The Biacore affinity assay utilized a Protein A chip (GE healthcare), which was set to capture human BCMA-hFc recombinant protein (0.5 μg/mL) at 200 RU and after the baseline was stable, a gradient dilution of antibody Fab (from 10 μg/mL, 2-fold dilutions for 7 gradients) was run through the chip at a flow rate of 30 μL/min for a binding time of 350 s and a dissociation time of 600 s. Kinetic constants were fitted using Biacore T200 evaluation software with a Langmuir 1:1 kinetics model. The results of the BCMA antibody affinity assay are shown in Table 1.

Table 1. The affinity of BCMA Antibodies

| Fab | ka (1/Ms) | kd (1/s) | KD (nM) |
| --- | --- | --- | --- |
| 3G11 | 3.33E+04 | 4.32E-04 | 13.0 |
| 6G5 | 2.94E+04 | 6.13E-05 | 2.1 |
| 15G9 | 1.03E+06 | 1.26E-02 | 12.2 |
| 15H10 | 3.78E+04 | 6.90E-04 | 18.3 |
| 16B9 | 1.17E+05 | 4.76E-04 | 4.1 |
| 23F8 | 5.77E+04 | 3.22E-04 | 5.6 |
| 23G9 | 4.07E+03 | 3.13E-04 | 76.9 |
| 29A11 | 7.64E+04 | 2.43E-04 | 3.2 |

(continued)

| Fab | ka (1/Ms) | kd (1/s) | KD (nM) |
|---|---|---|---|
| 34G2 | 1.42E+05 | 7.05E-04 | 5.0 |
| 38E2 | 9.88E+04 | 7.98E-04 | 8.1 |
| 38D9 | 5.91E+04 | 3.29E-04 | 5.6 |
| 38F9 | 5.81E+03 | 2.00E-03 | 344 |
| 40C6 | 4.21E+04 | 2.71E-04 | 6.4 |

The BCMA antibody variable region sequences are as follows:

**Table 2. Variable region sequences of BCMA murine antibody**

| BCMA murine antibody | HCVR | | HCDR1 | HCDR2 | HCDR3 | LCVR | | LCDR1 | LCDR2 | LCDR3 |
|---|---|---|---|---|---|---|---|---|---|---|
| | Amino acid sequence | Nucleotide sequence | Amino acid Sequence | Amino acid Sequence | Amino acid Sequence | Amino acid sequence | Nucleotide sequence | Amino acid Sequence | Amino acid Sequence | Amino acid Sequence |
| 3G11 | 10 | 11 | 12 | 13 | 14 | 15 | 16 | 17 | 18 | 19 |
| 6G5 | 10 | 11 | 12 | 13 | 14 | 20 | 21 | 22 | 23 | 19 |
| 15G9 | 24 | 25 | 26 | 27 | 28 | 29 | 30 | 31 | 32 | 33 |
| 15H10 | 34 | 35 | 12 | 36 | 37 | 38 | 39 | 17 | 40 | 19 |
| 16B9 | 41 | 42 | 12 | 43 | 44 | 45 | 46 | 47 | 18 | 19 |
| 23F8 | 48 | 49 | 12 | 50 | 14 | 51 | 52 | 17 | 40 | 19 |
| 23G9 | 53 | 54 | 55 | 56 | 57 | 58 | 59 | 60 | 61 | 62 |
| 29A11 | 10 | 11 | 12 | 13 | 14 | 38 | 39 | 17 | 40 | 19 |
| 34G2 | 63 | 64 | 65 | 66 | 14 | 67 | 68 | 17 | 40 | 19 |
| 38E2 | 69 | 70 | 71 | 72 | 73 | 74 | 75 | 76 | 77 | 78 |
| 38D9 | 79 | 80 | 12 | 13 | 14 | 81 | 82 | 83 | 84 | 19 |
| 38F9 | 10 | 11 | 12 | 13 | 14 | 85 | 86 | 87 | 88 | 89 |
| 40C6 | 10 | 11 | 12 | 13 | 14 | 90 | 91 | 92 | 23 | 19 |

3G11

The amino acid sequence of the 3G11 heavy chain VH is shown in SEQ ID NO. 10, of which the encoding nucleic acid is shown in SEQ ID NO. 11, and the HCDR1, HCDR2, and HCDR3 are shown in SEQ ID NO. 12, 13 and 14, respectively.

<------------FR1------------> CDR1 <-----FR2-----> CDR2 <---------

EIQLVQSGPELKKPGETVKISCKASGYIF**TNFGMN**WVRQAPGKALKWMG**WINTY**

**TGEQIYADDFKG**RFAFSLETSA

-------FR3-----------> CDR3 <----FR4--->

STAYLQINNLKNEDTATYFCAR**GEIYYGYDVGFVY**WGQGTLVTVSA

Nucleic acid sequence

GAGATCCAGTTGGTGCAGTCTGGACCTGAGCTGAAGAAGCCTGGAGAGACAG
TCAAGATCTCCTGCAAGGCTTCTGGGTATATCTTCACAAACTTTGGAATGAACT
GGGTGAGGCAGGCTCCAGGAAAGGCTTTAAAGTGGATGGGCTGGATAAACAC
CTACACTGGAGAACAAATATATGCTGATGACTTCAAGGGACGGTTTGCCTTCTC
TTTGGAACCTCTGCCAGCACTGCCTATTTGCAGATCAACAACCTCAAAAATGA
GGACACGGCTACATATTTCTGTGCAAGAGGGGAGATCTACTATGGTTACGACGT
GGGCTTTGTTTACTGGGGCCAAGGGACTCTGGTCACTGTCTCTGCA

The amino acid sequence of the 3G11 light chain VK is shown in SEQ ID NO. 15, of which the encoding nucleic acid is shown in SEQ ID NO. 16, and the HCDR1, HCDR2, and HCDR3 are shown in SEQ ID NO. 17, 18 and 19, respectively.

<---------FR1--------->     CDR1     <-----FR2-----> CDR2 <---------------

DIVLTQSPASLTVSLGQRATISC**RASKSVSTSGYSYMH**WYQQKPGQPPKLLIY**LAS**

**DLES**GVSARFSGSGSGTDFT

--FR3----------> CDR3     <---FR4-->

LNIHPVEEEDAATYYC**QHSRELPWT**FGGGTKLEIK

Nucleic acid sequence

GATATTGTGCTAACTCAGTCTCCTGCTTCCTTAACTGTATCTCTGGGGCAGAGGG
CCACCATCTCATGCAGGGCCAGCAAAAGTGTCAGTACATCTGGCTATAGTTATAT
GCACTGGTACCAACAGAAACCAGGACAGCCACCCAAACTCCTCATCTATCTTG
CATCCGACCTAGAATCTGGGGTCTCTGCCAGGTTCAGTGGCAGTGGGTCTGGG
ACAGACTTCACCCTCAACATCCATCCTGTGGAGGAGGAGGATGCTGCAACCTAT
TACTGTCAACACAGTAGGGAACTTCCGTGGACGTTCGGTGGAGGCACCAAGCT
GGAAATAAAA

6G5

The amino acid sequence of the 6G5 heavy chain VH is shown in SEQ ID NO. 10, of which the encoding nucleic acid is shown in SEQ ID NO. 11, and the HCDR1, HCDR2, and HCDR3 are shown in SEQ ID NO. 12, 13 and 14,

respectively.

```
<------------FR1------------> CDR1 <-----FR2-----> CDR2      <---------
EIQLVQSGPELKKPGETVKISCKASGYIFTNFGMNWVRQAPGKALKWMGWINTY
TGEQIYADDFKGRFAFSLETSA
```

```
-------FR3-----------> CDR3 <----FR4--->
STAYLQINNLKNEDTATYFCARGEIYYGYDVGFVYWGQGTLVTVSA
```

Nucleic acid sequence

GAGATCCAGTTGGTGCAGTCTGGACCTGAGCTGAAGAAGCCTGGAGAGACAG

TCAAGATCTCCTGCAAGGCTTCTGGGTATATCTTCACAAACTTTGGAATGAACT

GGGTGAGGCAGGCTCCAGGAAAGGCTTTAAAGTGGATGGGCTGGATAAACAC

CTACACTGGAGAACAAATATATGCTGATGACTTCAAGGGACGGTTTGCCTTCTC

TTTGGAAACCTCTGCCAGCACTGCCTATTTGCAGATCAACAACCTCAAAAATGA

GGACACGGCTACATATTTCTGTGCAAGAGGGGAGATCTACTATGGTTACGACGT

GGGCTTTGTTTACTGGGGCCAAGGGACTCTGGTCACTGTCTCTGCA

The amino acid sequence of the 6G5 light chain VK is shown in SEQ ID NO. 20, of which the encoding nucleic acid is shown in SEQ ID NO. 21, and the HCDR1, HCDR2, and HCDR3 are shown in SEQ ID NO. 22, 23 and 19, respectively.

```
<---------FR1--------->    CDR1    <-----FR2-----> CDR2 <---------------
DIVLTQSPASLAISLGQRATISCRASKSVTTSGYSYIHWYQQKPGQPPKLLIYLASD
LEAGVPARFSGSGSGTDFT
```

```
--FR3----------> CDR3   <---FR4-->
LNIHPVEEEDAATYYCQHSRELPWTFGGGTKLELK
```

Nucleic acid sequence

GACATTGTGCTAACACAGTCTCCTGCTTCCTTAGCTATATCTCTGGGGCAGAGG

GCCACCATCTCATGCAGGGCCAGCAAAGTGTCACTACATCTGGCTATAGTTAT

ATACACTGGTACCAACAGAAACCAGGACAGCCACCCAAACTCCTCATCTATCTT

GCATCCGACCTAGAAGCTGGGGTCCCTGCCAGGTTCAGTGGCAGTGGGTCTGG

GACAGACTTCACCCTCAACATCCATCCTGTGGAGGAAGAGGATGCTGCAACCT

ATTACTGTCAGCACAGTAGGGAGCTTCCGTGGACGTTCGGTGGAGGCACCAAG

CTGGAGCTGAAA

15G9

The amino acid sequence of the 15G9 heavy chain VH is shown in SEQ ID NO. 24, of which the encoding nucleic acid is shown in SEQ ID NO. 25, and the HCDR1, HCDR2, and HCDR3 are shown in SEQ ID NO. 26, 27 and 28, respectively.

<------------FR1------------> CDR1 <-----FR2-----> CDR2 <---------

QVQLQQSGPELVKPGASVNLSCKASGYTF**TNYVIH**WVKQKPGQGLEWIG**YTIPY**

**TDVTKYNEKFKA**KATLTSDKSS

-------FR3-----------> CDR3 <---FR4--->

STAFMELSSLTSEDSAVYYCAR**YDFDGYFDY**WGQGTTLTVSS

Nucleic acid sequence

CAGGTCCAGCTGCAACAGTCTGGACCTGAGCTGGTGAAGCCTGGGGCTTCAGT

GAATTTGTCCTGCAAGGCTTCTGGATACACATTCACTAATTATGTTATACACTGG

GTAAAGCAGAAGCCTGGGCAGGGCCTTGAGTGGATTGGATATACTATCCCTTAC

ACTGATGTTACTAAGTACAATGAGAAATTCAAAGCCAAGGCCACACTGACTTC

AGACAAATCCTCCAGCACAGCCTTCATGGAGCTCAGCAGCCTGACCTCTGAGG

ACTCTGCGGTCTATTACTGTGCAAGATATGATTTCGACGGCTACTTTGACTACTG

GGGCCAAGGCACCACTCTCACAGTCTCCTCA

The amino acid sequence of the 15G9 light chain VK is shown in SEQ ID NO. 29, of which the encoding nucleic acid is shown in SEQ ID NO. 30, and the HCDR1, HCDR2, and HCDR3 are shown in SEQ ID NO. 31, 32 and 33, respectively.

```
<---------FR1---------->      CDR1       <-----FR2-----> CDR2 <--------------
```

DIVMTQAAFSNPVTLGTSASISC**RSSKSLLHSNGITYLY**WYLQKPGQSPQLLIY**Q**

**MSNLAS**GVPDRFSSSGSGTDF

```
--FR3-----------> CDR3   <---FR4-->
```

TLRISRVEAEDVGVYYC**AQNLELPWT**FGGGTKLELK

Nucleic acid sequence

GATATTGTGATGACTCAGGCTGCATTCTCCAATCCAGTCACTCTTGGAACATCA

GCTTCCATCTCCTGCAGGTCTAGTAAGAGTCTCCTACATAGTAATGGCATCACTT

ATTTGTATTGGTATCTGCAGAAGCCAGGCCAGTCTCCTCAGCTCCTGATTTATCA

GATGTCCAACCTTGCCTCAGGAGTCCCAGACAGGTTCAGTAGCAGTGGGTCAG

GAACTGATTTCACACTGAGAATCAGCAGAGTGGAGGCTGAGGATGTGGGTGTT

TATTACTGTGCTCAAAATCTAGAACTTCCGTGGACGTTCGGTGGAGGCACCAAG

CTGGAGCTGAAA

15H10

The amino acid sequence of the 15H10 heavy chain VH is shown in SEQ ID NO. 34, of which the encoding nucleic acid is shown in SEQ ID NO. 35, and the HCDR1, HCDR2, and HCDR3 are shown in SEQ ID NO. 12, 36 and 37, respectively.

```
<------------FR1------------> CDR1 <-----FR2-----> CDR2        <---------
```

EVQLQQSGPELKKPGETVKISCKASGYTF**TNFGMN**WVRQTPGKALKWMG**WINT**

**YTGDLIYGDDFKG**RFAFSLETSA

```
-------FR3-----------> CDR3  <---FR4--->
```

STAYLQINNLKNEDTATYFCAR**GEIYYGYDVGFAY**WGQGTLVTVSA

Nucleic acid sequence

GAGGTCCAGCTGCAGCAGTCTGGACCTGAGCTGAAGAAGCCTGGAGAGACAG

TCAAGATCTCCTGCAAGGCTTCTGGGTATACCTTCACAAACTTTGGAATGAACT

GGGTGAGGCAGACTCCAGGAAAGGCTTTAAAGTGGATGGGCTGGATAAACAC

CTACACTGGAGACTTAATATATGGTGATGACTTTAAGGGACGGTTTGCCTTCTCT

TTGGAAACCTCTGCCAGCACTGCCTATTTACAGATCAACAACCTCAAAAATGAG

GACACGGCTACATATTTCTGTGCAAGAGGGGAGATCTACTATGGTTACGACGTG

GGGTTTGCTTACTGGGGCCAAGGGACTCTGGTCACTGTCTCTGCA

The amino acid sequence of the 15H10 light chain VK is shown in SEQ ID NO. 38, of which the encoding nucleic acid is shown in SEQ ID NO. 39, and the HCDR1, HCDR2, and HCDR3 are shown in SEQ ID NO. 17, 40 and 19, respectively.

<---------FR1--------->     CDR1     <-----FR2-----> CDR2 <---------------

DIVLTQSPASLAVSLGQRATISC**RASKSVSTSGYSYMH**WYQQKPGQPPKLLIY**LAS**

**NLES**GVPARFSGSGSGTDFT

--FR3----------> CDR3   <---FR4-->

LNIHPVEEEDAATYYC**QHSRELPWT**FGGGTKLEIK

Nucleic acid sequence

GACATTGTGCTGACACAGTCTCCTGCTTCCTTAGCTGTATCTCTGGGGCAGAGG

GCCACCATCTCATGCAGGGCCAGCAAAAGTGTCAGTACATCTGGCTATAGTTAT

ATGCACTGGTACCAACAGAAACCAGGACAGCCACCCAAACTCCTCATCTATCTT

GCATCCAACCTAGAATCTGGGGTCCCTGCCAGGTTCAGTGGTAGTGGGTCTGG

GACAGACTTCACCCTCAACATCCATCCTGTGGAGGAGGAGGATGCTGCAACCT

ATTACTGTCAGCACAGTAGGGAGCTTCCGTGGACGTTCGGTGGAGGCACCAAG

CTGGAAATCAAA

16B9

The amino acid sequence of the 16B9 heavy chain VH is shown in SEQ ID NO. 41, of which the encoding nucleic acid is shown in SEQ ID NO. 42, and the HCDR1, HCDR2, and HCDR3 are shown in SEQ ID NO. 12, 43 and 44, respectively.

```
<------------FR1------------> CDR1 <-----FR2-----> CDR2 <---------
```

EIQLVQSGPELKKPGETVKISCKASGYIF**TNFGMN**WVRQTPGKALKWMG**WINTY TGETIYADDFKG**LFAFSLEVPA

```
-------FR3-----------> CDR3 <----FR4--->
```

STAYLQINNLKNEDTATYFCAR**GEIYYGFDVGFAY**WGQGTLVTVSA

Nucleic acid sequence

GAGATCCAGTTGGTGCAGTCTGGACCTGAGCTGAAGAAGCCTGGAGAGACAG

TCAAGATCTCCTGCAAGGCTTCTGGGTATATTTTCACAAACTTTGGAATGAACT

GGGTGAGGCAGACTCCAGGAAAGGCTTTAAAGTGGATGGGCTGGATAAACAC

CTACACTGGAGAAACAATATATGCTGATGACTTCAAGGGACTATTTGCCTTCTCT

TTGGAAGTGCCTGCCAGCACTGCCTATTTGCAGATCAACAACCTCAAAAATGA

GGACACGGCTACATATTTCTGTGCAAGAGGGGAGATCTACTATGGTTTCGACGT

GGGATTTGCTTACTGGGGCCAAGGGACTCTGGTCACTGTCTCTGCA

The amino acid sequence of the 16B9 light chain VK is shown in SEQ ID NO. 45, of which the encoding nucleic acid is shown in SEQ ID NO. 46, and the HCDR1, HCDR2, and HCDR3 are shown in SEQ ID NO. 47, 18 and 19, respectively.

```
<---------FR1---------> CDR1 <-----FR2----> CDR2 <---------------
```

DIVLTQSPASLTVSLGQRATISC**RASKSVSTSGYSYIH**WYQQKPGQPPKLLIY**LASD LES**GVPARFSGSGSGTDFT

LNIHPVEEEDAATYYC**QHSRELPWT**FGGGTKLELK

Nucleic acid sequence

GACATTGTGCTAACACAGTCTCCTGCTTCCTTAACTGTATCTCTGGGGCAGAGG

GCCACCATCTCATGCAGGGCCAGCAAAGTGTCAGTACATCTGGCTATAGTTAT

ATACACTGGTACCAACAGAAACCAGGACAGCCACCCAAACTCCTCATCTATCTT

GCATCCGACCTAGAATCTGGGGTCCCTGCCAGGTTCAGTGGCAGTGGGTCTGG

GACAGACTTCACCCTCAACATCCATCCTGTGGAGGAGGAGGATGCTGCAACCT

ATTACTGTCAACACAGTAGGGAACTTCCGTGGACGTTCGGTGGAGGCACCAAG

CTGGAGCTGAAA

23F8

The amino acid sequence of the 23F8 heavy chain VH is shown in SEQ ID NO. 48, of which the encoding nucleic acid is shown in SEQ ID NO. 49, and the HCDR1, HCDR2, and HCDR3 are shown in SEQ ID NO. 12, 50 and 14, respectively.

<------------FR1------------> CDR1 <-----FR2----->    CDR2    <---------

EIQLVQSGPELKKPGETVKISCTASGYTF**TNFGMN**WVRQAPGKALRWMG**WINTY**

**TGDQIYADDFKG**RFAFSLETSA

-------FR3----------->    CDR3   <----FR4--->

STAYLQINNLKNEDTATYFCAR**GEIYYGYDVGFVY**WGQGTLVTVSA

Nucleic acid sequence

GAGATCCAGTTGGTGCAGTCTGGACCTGAGCTGAAGAAGCCTGGAGAGACAG

TCAAGATCTCCTGCACGGCTTCTGGGTATACCTTCACAAACTTTGGAATGAACT

GGGTGAGGCAGGCTCCAGGAAAGGCTTTAAGGTGGATGGGCTGGATAAACAC

CTACACTGGAGACCAAATATATGCTGATGACTTCAAGGGACGGTTTGCCTTCTC

TTTGGAAACCTCTGCCAGCACTGCCTATTTGCAGATCAACAACCTCAAAAATGA

GGACACGGCTACATATTTCTGTGCAAGAGGGGAGATCTACTATGGTTACGACGT

GGGCTTTGTTTATTGGGGCCAAGGGACTCTGGTCACTGTCTCTGCA

The amino acid sequence of the 23F8 light chain VK is shown in SEQ ID NO. 51, of which the encoding nucleic acid is shown in SEQ ID NO. 52, and the HCDR1, HCDR2, and HCDR3 are shown in SEQ ID NO. 17, 40 and 19, respectively.

```
<---------FR1--------->      CDR1     <-----FR2-----> CDR2  <---------------
```

DIVLTQSPASLAVSLGQRATISC**RASKSVSTSGYSYMH**WYQQKPGQPPKLLIY**LAS**

**NLES**GVPARFSGSGSGTDFT

```
         --FR3----------> CDR3   <---FR4-->
```

LNIHPVEEEDAATYYC**QHSRELPWT**FGGGTKLEIK

Nucleic acid sequence

GACATTGTGCTAACACAGTCTCCTGCTTCCTTAGCTGTATCTCTGGGGCAGAGG

GCCACCATCTCATGCAGGGCCAGCAAAAGTGTCAGTACATCTGGCTATAGTTAT

ATGCACTGGTACCAACAGAAACCAGGACAGCCACCCAAACTCCTCATCTATCTT

GCATCCAACCTAGAATCTGGGGTCCCTGCCAGGTTCAGTGGCAGTGGGTCTGG

GACAGACTTCACCCTCAACATCCATCCTGTGGAGGAGGAGGATGCTGCAACCT

ATTACTGTCAGCACAGTAGGGAGCTTCCGTGGACGTTCGGTGGAGGCACCAAG

CTGGAAATAAAA

23G9

The amino acid sequence of the 23G9 heavy chain VH is shown in SEQ ID NO. 53, of which the encoding nucleic acid is shown in SEQ ID NO. 54, and the HCDR1, HCDR2, and HCDR3 are shown in SEQ ID NO. 55, 56 and 57, respectively.

```
<------------FR1------------> CDR1 <-----FR2----->ffff   CDR2        <---------
```

EVQLQQSGTVLTRPGASVKLSCKASGYSF**SSYWMH**WLKQRPGQGLEWIG**AIYPG**

**NSRTTYNQKFKG**EAKLTADTSA

```
         -----FR3-----------> CDR3 <---FR4--->
```

STAYMDLSSLTNADSAVYFC**TREETY**WGQGTLVTVSA

Nucleic acid sequence

GAGGTTCAGCTCCAGCAGTCTGGGACTGTGCTGACAAGGCCTGGGGCTTCCGT

GAAGTTGTCCTGCAAGGCTTCTGGCTACAGCTTTTCCAGCTACTGGATGCACTG

GCTAAAACAGAGGCCTGGACAGGGTCTAGAATGGATTGGTGCTATTTATCCTGG

AAATAGTCGTACTACCTACAACCAGAAGTTCAAGGGCGAGGCCAAACTGACTG

CGGACACTTCCGCCAGCACTGCCTACATGGACCTCAGCAGCCTGACAAATGCC

GACTCTGCGGTCTATTTCTGTACAAGAGAGGAGACTTACTGGGGCCAAGGGAC

TCTGGTCACTGTCTCTGCA

The amino acid sequence of the 23G9 light chain VK is shown in SEQ ID NO. 58, of which the encoding nucleic acid is shown in SEQ ID NO. 59, and the HCDR1, HCDR2, and HCDR3 are shown in SEQ ID NOs. 60, 61 and 62, respectively.

<---------FR1--------->     CDR1     <-----FR2-----> CDR2 <--------------

DVVMTQTPLSLSVTIGQPASISC**KSSQGLLDSYGVTYLN**WLFQRPGQPPKRLIY**LV**

**SELDS**GVPDRFTGSGSGTDF

---FR3----------> CDR3   <---FR4-->

TLRISRVEAEDLGIYYC**WQGSHFPLT**FGAGTKLELK

Nucleic acid sequence

GATGTTGTGATGACCCAGACTCCACTCTCTTTGTCGGTTACCATTGGACAACCA

GCCTCCATCTCTTGCAAGTCAAGTCAGGGCCTCTTAGATAGTTATGGAGTGACA

TATTTGAATTGGTTGTTTCAGAGGCCAGGCCAGCCTCCAAAGCGCCTAATCTAT

CTAGTGTCTGAACTGGACTCTGGAGTCCCTGACAGGTTCACTGGCAGTGGATC

AGGGACAGATTTCACACTGAGAATCAGCAGAGTGGAGGCTGAGGATTTGGGAA

TTTATTATTGCTGGCAAGGTTCACATTTTCCTCTCACGTTCGGTGCTGGGACCAA

GCTGGAGCTGAAA

29A11

The amino acid sequence of the 29A11 heavy chain VH is shown in SEQ ID NO. 10, of which the encoding nucleic acid is shown in SEQ ID NO. 11, and the HCDR1, HCDR2, and HCDR3 are shown in SEQ ID NOs. 12, 13 and 14, respectively.

```
<------------FR1------------> CDR1 <-----FR2------>    CDR2      <---------
```

EIQLVQSGPELKKPGETVKISCKASGYIF**TNFGMN**WVRQAPGKALKWMG**WINTY**

**TGEQIYADDFKG**RFAFSLETSA

```
-------FR3----------->    CDR3   <----FR4--->
```

STAYLQINNLKNEDTATYFCAR**GEIYYGYDVGFVY**WGQGTLVTVSA

Nucleic acid sequence

GAGATCCAGCTGGTGCAGTCTGGACCTGAGCTGAAGAAGCCTGGAGAGACAG

TCAAGATCTCCTGCAAGGCTTCTGGGTATATCTTCACAAACTTTGGAATGAACT

GGGTGAGGCAGGCTCCAGGAAAGGCTTTAAAGTGGATGGGCTGGATAAACAC

CTACACTGGAGAACAAATATATGCTGATGACTTCAAGGGACGGTTTGCCTTCTC

TTTGGAACCTCTGCCAGCACTGCCTATTTGCAGATCAACAACCTCAAAAATGA

GGACACGGCTACATATTTCTGTGCAAGAGGGGAGATCTACTATGGTTACGACGT

GGGCTTTGTTTACTGGGGCCAAGGGACTCTGGTCACTGTCTCTGCA

The amino acid sequence of the 29A11 light chain VK is shown in SEQ ID NO. 38, of which the encoding nucleic acid is shown in SEQ ID NO. 39, and the HCDR1, HCDR2, and HCDR3 are shown in SEQ ID NOs. 17, 40 and 19, respectively.

```
<---------FR1--------->    CDR1    <-----FR2-----> CDR2 <---------------
```

DIVLTQSPASLAVSLGQRATISC**RASKSVSTSGYSYMH**WYQQKPGQPPKLLIY**LAS**

**NLES**GVPARFSGSGSGTDFT

```
--FR3----------> CDR3    <---FR4-->
```

LNIHPVEEEDAATYYC**QHSRELPWT**FGGGTKLEIK

Nucleic acid sequence

GATATTGTGCTAACTCAGTCTCCTGCTTCCTTAGCCGTATCTCTGGGGCAGAGG

GCCACCATCTCATGCAGGGCCAGCAAAGTGTCAGTACATCTGGCTATAGTTAT

ATGCACTGGTACCAACAGAAACCAGGACAGCCACCCAAACTCCTCATCTATCTT

GCATCCAACCTAGAATCTGGGGTCCCTGCCAGGTTCAGTGGCAGTGGGTCTGG

GACAGACTTCACCCTCAACATCCATCCTGTGGAGGAGGAGGATGCTGCAACCT

ATTACTGTCAGCACAGTAGGGAGCTTCCGTGGACGTTCGGTGGAGGCACCAAG

CTGGAAATCAAA

34G2

The amino acid sequence of the 34G2 heavy chain VH is shown in SEQ ID NO. 63, of which the encoding nucleic acid is shown in SEQ ID NO. 64, and the HCDR1, HCDR2, and HCDR3 are shown in SEQ ID NOs. 65, 66 and 14, respectively.

```
        <------------FR1------------> CDR1 <-----FR2------>      CDR2       <---------

        EVQLQQSGPELRKPGETVKISCKASGYTFTNYGMNWVKQTPGKGLKWMGWINT

        YTGERIYADDFKGRFAFSLETSA


        -------FR3----------->      CDR3    <----FR4--->

        STAYLQINNLKNEDTATYFCARGEIYYGYDVGFVYWGQGTLVTVSA
```

Nucleic acid sequence

GAGGTCCAGTTGCAGCAGTCTGGACCTGAGCTGAGGAAGCCTGGAGAGACAG

TCAAGATCTCCTGCAAGGCTTCTGGGTATACCTTCACAAACTATGGAATGAACT

GGGTGAAGCAGACTCCAGGAAAGGGTTTAAAGTGGATGGGCTGGATAAACAC

CTACACTGGAGAACGAATATATGCTGATGACTTCAAGGGACGGTTTGCCTTCTC

TTTGGAAACCTCTGCCAGCACTGCCTATTTGCAGATCAACAACCTCAAAAATGA

GGACACGGCTACATATTTCTGTGCAAGAGGGGAGATCTACTATGGTTACGACGT

GGGCTTTGTTTACTGGGGCCAAGGGACTCTGGTCACTGTCTCTGCA

The amino acid sequence of the 34G2 light chain VK is shown in SEQ ID NO. 67, of which the encoding nucleic acid is shown in SEQ ID NO. 68, and the HCDR1, HCDR2, and HCDR3 are shown in SEQ ID NOs. 17, 40 and 19, respectively.

<---------FR1--------->     CDR1     <-----FR2-----> CDR2 <---------------

DIVLTQSPASLVVSLGQRATISC**RASKSVSTSGYSYMH**WYQQKPGQPPKLLIY**LAS**

**NLES**GVPARFSGSGSGTDFT


--FR3----------> CDR3   <---FR4-->

LNIHPVEEEDAATYYC**QHSRELPWT**FGGGTRLEIK


Nucleic acid sequence

GACATTGTGCTGACACAGTCTCCTGCTTCCTTAGTTGTATCTCTGGGGCAGAGG

GCCACCATCTCATGCAGGGCCAGCAAAAGTGTCAGTACATCTGGCTATAGTTAT

ATGCACTGGTACCAACAGAAACCAGGACAGCCACCCAAACTCCTCATCTATCTT

GCATCCAACCTAGAATCTGGGGTCCCTGCCAGGTTCAGTGGCAGTGGGTCTGG

GACAGACTTCACCCTCAACATCCATCCTGTGGAGGAGGAGGATGCTGCAACCT

ATTACTGTCAGCACAGTAGGGAGCTTCCGTGGACGTTCGGTGGAGGCACCAGA

CTGGAAATAAAA


38E2

The amino acid sequence of the 38E2 heavy chain VH is shown in SEQ ID NO. 69, of which the encoding nucleic acid is shown in SEQ ID NO. 70, and the HCDR1, HCDR2, and HCDR3 are shown in SEQ ID NOs. 71, 72 and 73, respectively. <-----------FR1-----------> CDR1 <-----FR2-----> CDR2 < ---------


<------------FR1------------> CDR1 <-----FR2----->     CDR2     <---------

QVQLQQSGAELVRPGTSVKMSCKAAGYTF**TNYWIG**WVKQRPGHGLEWIG**DIYP**

**GSGDTNYNGKFKG**KATLAADISS


-------FR3----------->    CDR3    <----FR4--->

NAAYMQLSSLTSEDSAIYYCAR**SYYRNDAASFAY**WGQGTLVTVSA


Nucleic acid sequence

CAGGTCCAGCTGCAGCAGTCTGGAGCTGAGCTGGTAAGGCCTGGGACTTCAGT

GAAGATGTCCTGCAAGGCTGCTGGATACACCTTCACTAACTACTGGATAGGTTG

GGTAAAGCAGAGGCCTGGACATGGCCTTGAGTGGATTGGAGATATTTACCCTGG

AAGTGGTGATACTAATTACAATGGGAAGTTCAAGGGCAAGGCCACACTGGCTG

CAGACATTTCCTCCAACGCAGCCTACATGCAGCTCAGCAGCCTGACATCTGAG

GACTCTGCCATCTATTACTGTGCAAGAAGCTACTATAGGAACGACGCGGCCTCA

TTTGCTTACTGGGGCCAAGGGACTCTGGTCACTGTCTCTGCA

The amino acid sequence of the 38E2 light chain VK is shown in SEQ ID NO. 74, of which the encoding nucleic acid is shown in SEQ ID NO. 75, and the HCDR1, HCDR2, and HCDR3 are shown in SEQ ID NOs. 76, 77 and 78, respectively.

<---------FR1---------> CDR1 <-----FR2-----> CDR2 <--------------------

DIVMTQSHKFMSTSVGDRVNITC**KASQDVDTAVA**WYQQKPSQSPKLLIY**WTSTR**

**HT**GVPDRFTGSGSGTDFTLTIS

FR3--------> CDR3 <---FR4-->

NVQSEDLADYFC**QQFSSYPLT**FGAGTKLELK

Nucleic acid sequence

GACATTGTGATGACCCAGTCTCACAAATTCATGTCCACATCAGTAGGAGACAGG

GTCAACATCACCTGCAAGGCCAGTCAGGATGTGGATACTGCTGTAGCCTGGTAT

CAACAGAAACCAAGTCAATCTCCTAAACTACTGATTTACTGGACATCCACCCGG

CACACTGGAGTCCCTGATCGCTTCACAGGCAGTGGATCTGGGACAGATTTCAC

TCTCACCATTAGCAATGTGCAGTCTGAAGACTTGGCAGATTATTTCTGTCAACA

ATTTAGTAGCTATCCTCTCACGTTCGGTGCTGGGACCAAGCTGGAGCTGAAA

38D9

The amino acid sequence of the 38D9 heavy chain VH is shown in SEQ ID NO. 79, of which the encoding nucleic acid is shown in SEQ ID NO. 80, and the HCDR1, HCDR2, and HCDR3 are shown in SEQ ID NOs. 12, 13 and 14, respectively.

```
<-----------FR1------------> CDR1 <-----FR2----> CDR2 <---------
```

EVQLQQSGPELKKPGETVKISCKASGYIF**TNFGMN**WVRQAPGKALKWMG**WINT**

**YTGEQIYADDFKG**RFAFSLETSA

```
-------FR3-----------> CDR3 <----FR4--->
```

STAYLQINNLKNEDTATYFCAR**GEIYYGYDVGFVY**WGQGTLVTVSA

Nucleic acid sequence

GAGGTCCAGTTGCAGCAGTCTGGACCTGAGCTGAAGAAGCCTGGAGAGACAG

TCAAGATCTCCTGCAAGGCTTCTGGGTATATCTTCACGAACTTTGGAATGAACT

GGGTGAGGCAGGCTCCAGGAAAGGCTTTAAAGTGGATGGGCTGGATAAACAC

CTACACTGGAGAACAAATATATGCTGATGACTTCAAGGGACGGTTTGCCTTCTC

TTTGGAAACCTCTGCCAGCACTGCCTATTTGCAGATCAACAACCTCAAAAATGA

GGACACGGCTACATATTTCTGTGCAAGAGGGGAGATCTACTATGGTTACGACGT

GGGCTTTGTTTACTGGGGCCAAGGGACTCTGGTCACTGTCTCTGCA

The amino acid sequence of the 38D9 light chain VK is shown in SEQ ID NO. 81, of which the encoding nucleic acid is shown in SEQ ID NO. 82, and the HCDR1, HCDR2, and HCDR3 are shown in SEQ ID NOs. 83, 84 and 19, respectively.

```
<---------FR1---------> CDR1 <-----FR2-----> CDR2 <---------------
```

DIVLTQSPASLTVSLGQRATISC**GASKSVSTSGYSYMH**WYQQKPGQPPKLLIY**LGS**

**DLES**GVPARFSGSGSGTDFT

```
--FR3----------> CDR3 <---FR4-->
```

LNIHPVEEEDAATYYC**QHSRELPWT**FGGGTKLEIK

Nucleic acid sequence

GACATTGTGCTGACACAGTCTCCTGCTTCCTTAACTGTATCTCTGGGGCAGAGG

GCCACCATCTCATGCGGGGCCAGCAAAAGTGTCAGTACATCTGGCTATAGTTAT

ATGCACTGGTACCAACAGAAACCAGGACAGCCACCCAAACTCCTCATCTATCTT

GGATCCGACCTAGAATCTGGGGTCCCTGCCAGGTTCAGTGGCAGTGGGTCTGG

GACAGACTTCACCCTCAACATCCATCCTGTGGAGGAGGAGGATGCTGCAACCT

ATTACTGTCAACACAGTAGGGAACTTCCGTGGACGTTCGGTGGAGGCACCAAG

CTGGAAATCAAA

38F9

The amino acid sequence of the 38F9 heavy chain VH is shown in SEQ ID NO. 10, of which the encoding nucleic acid is shown in SEQ ID NO. 11, and the HCDR1, HCDR2, and HCDR3 are shown in SEQ ID NOs. 12, 13 and 14, respectively.

<------------FR1------------> CDR1 <-----FR2----> CDR2 <---------

EIQLVQSGPELKKPGETVKISCKASGYIF**TNFGMN**WVRQAPGKALKWMG**WINTY**

**TGEQIYADDFKG**RFAFSLETSA

-------FR3-----------> CDR3 <----FR4--->

STAYLQINNLKNEDTATYFCAR**GEIYYGYDVGFVY**WGQGTLVTVSA

Nucleic acid sequence

GAGATCCAGTTGGTGCAGTCTGGACCTGAGCTGAAGAAGCCTGGAGAGACAG

TCAAGATCTCCTGCAAGGCTTCTGGGTATATCTTCACAAACTTTGGAATGAACT

GGGTGAGGCAGGCTCCAGGAAAGGCTTTAAAGTGGATGGGCTGGATAAACAC

CTACACTGGAGAACAAATATATGCTGATGACTTCAAGGGACGGTTTGCCTTCTC

TTTGGAAACCTCTGCCAGCACTGCCTATTTGCAGATCAACAACCTCAAAAATGA

GGACACGGCTACATATTTCTGTGCAAGAGGGGAGATCTACTATGGTTACGACGT

GGGCTTTGTTTACTGGGGCCAAGGGACTCTGGTCACTGTCTCTGCA

The amino acid sequence of the 38F9 light chain VK is shown in SEQ ID NO. 85, of which the encoding nucleic acid is shown in SEQ ID NO. 86, and the HCDR1, HCDR2, and HCDR3 are shown in SEQ ID NOs. 87, 88 and 89, respectively.

<-------FR1--------->     CDR1    <-----FR2-----> CDR2 <--------------

NIVLTQSPASLAVSLGQRATISC**RASESVDSSGNSFMH**WYQQKPGQPPKLLIY**LES**

**NLES**GVPARFSGSGSRTDFT

FR3------------> CDR3   <---FR4-->

LTIEPVEADDAATYYC**QQSNEDPWT**FGGGTKLEIK

Nucleic acid sequence

AACATTGTGCTGACCCAATCTCCAGCTTCTTTGGCTGTGTCTCTAGGGCAGAGG

GCCACCATATCCTGCAGAGCCAGTGAAAGTGTTGATAGTTCTGGCAATAGTTTT

ATGCACTGGTACCAGCAGAAACCAGGACAGCCACCCAAACTCCTCATCTATCTT

GAATCCAACCTAGAATCTGGGGTCCCTGCCAGGTTCAGTGGCAGTGGGTCTAG

GACAGACTTCACCCTCACCATTGAACCTGTGGAGGCTGATGATGCTGCAACCTA

TTACTGTCAGCAAAGTAATGAGGATCCGTGGACGTTCGGTGGAGGCACCAAGC

TGGAAATCAAA

40C6

The amino acid sequence of the 40C6 heavy chain VH is shown in SEQ ID NO. 10, of which the encoding nucleic acid is shown in SEQ ID NO. 11, and the HCDR1, HCDR2, and HCDR3 are shown in SEQ ID NOs. 12, 13 and 14, respectively.

<-----------FR1------------> CDR1 <-----FR2----->    CDR2    <---------

EIQLVQSGPELKKPGETVKISCKASGYIF**TNFGMN**WVRQAPGKALKWMG**WINTY**

**TGEQIYADDFKG**RFAFSLETSA

-------FR3----------->   CDR3   <----FR4--->

STAYLQINNLKNEDTATYFCAR**GEIYYGYDVGFVY**WGQGTLVTVSA

Nucleic acid sequence

GAGATCCAGTTGGTGCAGTCTGGACCTGAGCTGAAGAAGCCTGGAGAGACAG

TCAAGATCTCCTGCAAGGCTTCTGGGTATATCTTCACAAACTTTGGAATGAACT

GGGTGAGGCAGGCTCCAGGAAAGGCTTTAAAGTGGATGGGCTGGATAAACAC

CTACACTGGAGAACAAATATATGCTGATGACTTCAAGGGACGGTTTGCCTTCTC

TTTGGAAACCTCTGCCAGCACTGCCTATTTGCAGATCAACAACCTCAAAAATGA

GGACACGGCTACATATTTCTGTGCAAGAGGGGAGATCTACTATGGTTACGACGT

GGGCTTTGTTTACTGGGGCCAAGGGACTCTGGTCACTGTCTCTGCA

The amino acid sequence of 40C6 light chain VK is shown in SEQ ID NO. 90, of which the encoding nucleic acid is shown in SEQ ID NO. 91, and the LCDR1, LCDR2, and LCDR3 are shown in SEQ ID NOs. 92, 23 and 19, respectively.

<---------FR1--------->    CDR1    <-----FR2-----> CDR2 <---------------

DIVLTQSPASLAISLGQRATISC**RASRSVTTSGYSYIH**WYQQKPGQPPKLLIY**LASD**

**LEA**GVPARFSGSGSGTDFT

--FR3----------> CDR3    <---FR4-->

LNIHPVEEEDAATYYC**QHSRELPWT**FGGGTKLEIK

Nucleic acid sequence

GACATTGTGCTGACACAGTCTCCTGCTTCCTTAGCTATATCTCTGGGGCAGAGG

GCCACCATCTCATGCAGGGCCAGCAGAAGTGTCACTACATCTGGCTATAGTTAT

ATACACTGGTACCAACAGAAACCAGGACAGCCACCCAAACTCCTCATCTATCTT

GCATCCGACCTAGAAGCTGGGGTCCCTGCCAGGTTCAGTGGCAGTGGGTCTGG

GACAGACTTCACCCTCAACATCCATCCTGTGGAGGAAGAGGATGCTGCAACCT

ATTACTGTCAGCACAGTAGGGAGCTTCCGTGGACGTTCGGTGGAGGCACCAAG

CTGGAAATAAAA

**Example 3. Construction of BCMA×CD3 bispecific antibody**

**1. CD3 humanized antibody**

Murine hybridoma CD3 antibody (EMBO J.1985.4(2): 337-344; J. Immunol.1986,137(4): 1097-100; J.Exp.Med.1991,174: 319-326; J. Immunol.1991,147(9): 3047-52) recognizes the human and cynomolgus monkey CD3 receptor, the sequence of which is as follows: the anti-CD3 murine monoclonal antibody light chain amino acid sequence is shown in SEQ ID NO. 184:

QAVVTQESALTTSPGETVTLTCR**SSTGAVTTSNYAN**WVQQKPDHLFTGLIG**GTNK RAP**GVPARFSGSLIGDKAALTITGAQTEDEAIYFCA**LWYSNLWV**FGGGTKLTVL

the amino acid sequence of the anti-CD3 murine monoclonal antibody heavy chain is shown in SEQ ID NO. 185:

EVQLVESGGGLVQPKGSLKLSCAASGFTFN**TYAMN**WVRQAPGKGLEWVAR**IRSK YNNYATYYADS**VKDRFTISRDDSQSILYLQMNNLKTEDTAMYYCVR**HGNFGNSY VSWFAY**WGQGTLVTVSS

the anti-CD3 murine monoclonal antibody was humanized, the human germline gene IMGT_hVL7-43 with the highest homology was selected for light chain CDR transplantation and human IGLJ3 * 02 was selected for FM4; human IMGT hVH3-73 was selected for heavy chain CDR transplantation and human IGHJ4 * 01 was selected for FM4. The different heavy chain and light chain variants were designed.

The sequence of the light chain variable region of the humanized CD3 antibody is as follows:

Table 3. Light chain variable region sequences of CD3 humanized antibodies

| CD3 humanized antibody | SEQ ID NOs: 93-111 | | | | |
| --- | --- | --- | --- | --- | --- |
| | LCVR | | LCDR1 | LCDR2 | LCDR3 |
| | Amino acid sequence | Nucleotide sequence | Amino acid sequence | Amino acid sequence | Amino acid sequence |
| hVL1 | 93 | 94 | 95 | 96 | 97 |
| hVL2 | 98 | 99 | 95 | 96 | 97 |
| hVL3 | 100 | 101 | 102 | 103 | 97 |
| hVL4 | 104 | 105 | 102 | 103 | 97 |
| hVL5 | 106 | 107 | 95 | 96 | 108 |
| hVL6 | 109 | 110 | 95 | 111 | 108 |

The amino acid sequence of hVL1 is shown in SEQ ID NO. 93, of which the encoding nucleic acid is shown in SEQ ID NO. 94, and the LCDR1, LCDR2, and LCDR3 are shown in SEQ ID NOs. 95, 96 and 97, respectively.

QAVVTQEPSLTVSPGGTVTLTCR**SSTGAVTTSNYAN**WVQQKPGQAPRGLI**GGTNK RAP**WTPARFSGSLLGGKAALTLSGAQPEDEAEYYC**ALWYSNLWV**FGGGTKLTVL

The amino acid sequence of hVL2 is shown in SEQ ID NO. 98, of which the encoding nucleic acid is shown in SEQ ID NO. 99, and the LCDR1, LCDR2, and LCDR3 are shown in SEQ ID NOs. 95, 96 and 97, respectively.

QAVVTQEPSLTVSPGGTVTLTCR**SSTGAVTTSNYAN**WVQEKPGQAPRGLI**GGTNK RAP**WTPARFSGSLLGGKAALTLSGAQPEDEAEYYC**ALWYSNLWV**FGGGTKLTVL

The amino acid sequence of hVL3 is shown in SEQ ID NO. 100, of which the encoding nucleic acid is shown in SEQ ID NO. 101, and the LCDR1, LCDR2, and LCDR3 are shown in SEQ ID NOs. 102, 103 and 97, respectively.

EAVVTQEPSLTVSPGGTVTLTCE**SSDGAVTTSNYAN**WVQEKPGQAPRGLI**GGTNK**

**EAP**WTPARFSGSLLGGKAALTLSGAQPEDEAEYYC**ALWYSNLWV**FGGGTKLTVL

The amino acid sequence of hVL4 is shown in SEQ ID NO. 104, of which the encoding nucleic acid is shown in SEQ ID NO. 105, and the LCDR1, LCDR2, and LCDR3 are shown in SEQ ID NOs. 102, 103 and 97, respectively.

QAVVTQEPSLTVSPGGTVTLTCE**SSDGAVTTSNYAN**WVQEKPGQAPRGLI**GGTNK**

**EAP**WTPARFSGSLLGGKAALTLSGAQPEDEAEYYC**ALWYSNLWV**FGGGTKLTVL

The amino acid sequence of hVL5 is shown in SEQ ID NO. 106, of which the encoding nucleic acid is shown in SEQ ID NO. 107, and the LCDR1, LCDR2, and LCDR3 are shown in SEQ ID NOs. 95, 96 and 108, respectively.

QAVVTQEPSLTVSPGGTVTLTCR**SSTGAVTTSNYAN**WVQEKPGQAPRGLI**GGTNK**

**RAP**WTPARFSGSLLGGKAALTITGAQAEDEAEYYC**VLWYSNLWV**FGGGTKLTVL

The amino acid sequence of hVL6 is shown in SEQ ID NO. 109, of which the encoding nucleic acid is shown in SEQ ID NO. 110, and the LCDR1, LCDR2, and LCDR3 are shown in SEQ ID NOs. 95, 111 and 108, respectively.

QAVVTQEPSLTVSPGGTVTLTCR**SSTGAVTTSNYAN**WFQEKPGQAPRGLI**YGTNK**

**RAP**WTPARFSGSLLGGKAALTLSGAQAEDEAEYYC**VLWYSNLWV**FGGGTKLTVL

The sequences of the heavy chain variable region of the humanized CD3 antibody is as follows:

Table 4. Heavy chain variable region sequences of CD3 humanized antibodies

| CD3 humanized antibody | SEQ ID NOs: 112-141 | | | | |
| | HCVR | | HCDR1 | HCDR2 | HCDR3 |
| | Amino acid sequence | Nucleotide sequence | Amino acid Sequence | Amino acid Sequence | Amino acid Sequence |
| hVH1 | 112 | 113 | 114 | 115 | 116 |
| hVH2 | 117 | 118 | 119 | 115 | 116 |
| hVH3 | 120 | 121 | 119 | 115 | 122 |
| hVH4 | 123 | 124 | 119 | 115 | 125 |
| hVH5 | 126 | 127 | 119 | 115 | 128 |
| hVH6 | 129 | 130 | 119 | 115 | 131 |
| hVH7 | 132 | 133 | 134 | 135 | 116 |
| hVH8 | 136 | 137 | 114 | 115 | 116 |
| hVH9 | 138 | 139 | 114 | 115 | 116 |
| hVH10 | 140 | 141 | 114 | 115 | 116 |

The amino acid sequence of hVH1 is shown in SEQ ID NO. 112, of which the encoding nucleic acid is shown in SEQ ID NO. 113, and the HCDR1, HCDR2, and HCDR3 are shown in SEQ ID NOs. 114, 115 and 116, respectively.

EVQLVESGGGLVQPGGSLRLSCAASGFTF**NTYAMN**WVRQAPGKGLEWVSR**IRSK YNNYATYYADSVKD**RFTISRDDSKNTLYLQMNSLKTEDTAVYYCVR**HGNFGNSY VSWFAY**WGQGTLVTVSS

The amino acid sequence of hVH2 is shown in SEQ ID NO. 117, of which the encoding nucleic acid is shown in SEQ ID NO. 118, and the HCDR1, HCDR2, and HCDR3 are shown in SEQ ID NOs. 119, 115 and 116, respectively.

EVQLVESGGGLVQPGGSLRLSCAASGFTF**STYAMN**WVRKAPGKGLEWVSR**IRSK YNNYATYYADSVKD**RFTISRDDSKNTLYLQMNSLRAEDTAVYYCVR**HGNFGNSY VSWFAY**WGQGTLVTVSS

The amino acid sequence of hVH3 is shown in SEQ ID NO. 120, of which the encoding nucleic acid is shown in SEQ ID NO. 121, and the HCDR1, HCDR2, and HCDR3 are shown in SEQ ID NOs. 119, 115 and 122, respectively.

EVQLVESGGGLVQPGGSLRLSCAASGFTF**STYAMN**WVRKAPGKGLEWVSR**IRSK YNNYATYYADSVKD**RFTISRDDSKNTLYLQMNSLRAEDTAVYYCVR**HGNFGESY VSWFAY**WGQGTLVTVSS

The amino acid sequence of hVH4 is shown in SEQ ID NO. 123, of which the encoding nucleic acid is shown in SEQ ID NO. 124, and the HCDR1, HCDR2, and HCDR3 are shown in SEQ ID NOs. 119, 115 and 125, respectively.

EVQLVESGGGLVQPGGSLRLSCAASGFTF**STYAMN**WVRKAPGKGLEWVSR**IRSK YNNYATYYADSVKD**RFTISRDDSKNTLYLQMNSLRAEDTAVYYCVR**HGNFGQSY VSWFAY**WGQGTLVTVSS

The amino acid sequence of hVH5 is shown in SEQ ID NO. 126, of which the encoding nucleic acid is shown in SEQ ID NO. 127, and the HCDR1, HCDR2, and HCDR3 are shown in SEQ ID NOs. 119, 115 and 128, respectively.

EVQLVESGGGLVQPGGSLRLSCAASGFTF**STYAMN**WVRKAPGKGLEWVSR**IRSK YNNYATYYADSVKD**RFTISRDDSKNTLYLQMNSLRAEDTAVYYCVR**HGNFGDSY VSWFAY**WGQGTLVTVSS

The amino acid sequence of hVH6 is shown in SEQ ID NO. 129, of which the encoding nucleic acid is shown in SEQ ID NO. 130, and the HCDR1, HCDR2, and HCDR3 are shown in SEQ ID NOs. 119, 115 and 131, respectively.

EVQLVESGGGLVQPGGSLRLSCAASGFTF**STYAMN**WVRKAPGKGLEWVSR**IRSK YNNYATYYADSVKD**RFTISRDDSKNTLYLQMNSLRAEDTAVYYCVR**HGNFGTSY VSWFAY**WGQGTLVTVSS

The amino acid sequence of hVH7 is shown in SEQ ID NO. 132, of which the encoding nucleic acid is shown in SEQ ID NO. 133, and the HCDR1, HCDR2, and HCDR3 are shown in SEQ ID NOs. 134, 135 and 116, respectively.

EVQLVESGGGLVQPGGSLRLSCAASGFTF**SDYAMN**WVRKAPGKGLEWVSR**IRSK YNNYATYYADSVED**RFTISRDDSKNTLYLQMNSLRAEDTAVYYCVR**HGNFGNSY VSWFAY**WGQGTLVTVSS

The amino acid sequence of hVH8 is shown in SEQ ID NO. 136, of which the encoding nucleic acid is shown in SEQ ID NO. 137, and the HCDR1, HCDR2, and HCDR3 are shown in SEQ ID NOs. 114, 115 and 116, respectively.

EVQLVESGGGLVQPGGSLRLSCAASGFTF**NTYAMN**WVRKAPGKGLEWVGR**IRSK YNNYATYYADSVKD**RFTISRDDSKNSLYLQMNSLKTEDTAVYYCAR**HGNFGNSY VSWFAY**WGQGTLVTVSS

The amino acid sequence of hVH9 is shown in SEQ ID NO. 138, of which the encoding nucleic acid is shown in SEQ ID NO. 139, and the HCDR1, HCDR2, and HCDR3 are shown in SEQ ID NOs. 114, 115 and 116, respectively.

EVQLVESGGGLVQPGGSLRLSCAASGFTF**NTYAMN**WVRKAPGKGLEWVGR**IRSK YNNYATYYADSVKD**RFTISRDDSKNSLYLQMNSLKTEDTAVYYCVR**HGNFGNSY VSWFAY**WGQGTLVTVSS

The amino acid sequence of hVH10 is shown in SEQ ID NO. 140, of which the encoding nucleic acid is shown in SEQ ID NO. 141, and the HCDR1, HCDR2, and HCDR3 are shown in SEQ ID NOs. 114, 115 and 116, respectively.

EVQLVESGGGLVQPGGSLRLSCAASGFTF**NTYAMN**WVRKAPGKGLEWVAR**IRSK YNNYATYYADSVKD**RFTISRDDSKNSLYLQMNSLKTEDTAVYYCVR**HGNFGNSY VSWFAY**WGQGTLVTVSS

After the light and heavy chain humanized variants have been respectively subjected to full sequence synthesis, are cloned into the eukaryotic expression vector containing the constant region of the light chain of antibody λ or the constant region CH1-CH3 of the heavy chain of human IgG4, co-transfected into HEK293E cells, and cultured at 37°C under 120 rpm 5% $CO_2$ for 5 to 6 days, and then the culture supernatant is collected and purified by Protein A chromatography column.

**2. ELISA affinity assay**

Human CD3εγ protein was coated at 4°C overnight. After blocking with 2% skim milk, different dilutions of CD3 antibody were added to each well and incubated for 1 h; after the secondary antibody was added to the HPR-labeled goat anti-human IgG Fc and color development of TMB solution, the reaction was stopped with concentrated sulfuric acid and the absorbance was read at 450 nm (the results were shown in FIG. 4).

FIG. 4 shows a combination of anti-CD3 humanized antibodies (including aCD3-hVH8/VL1, aCD3-hVH9/VL1, aCD3-hVH9/VL2, aCD3-hVH9/VL3, aCD3-hVH1/VL5, aCD3-hVH8/VL5, aCD3-hVH9/VL5) binding to human CD3εγ protein, the anti-CD3 humanized antibodies bind to CD3εγ recombinant protein with high affinity.

**3. Jurkat T cell affinity assay**

Jurkat cells in the logarithmic growth phase were blocked with 3% BSA for 30 min, added to 96-well U-plate at

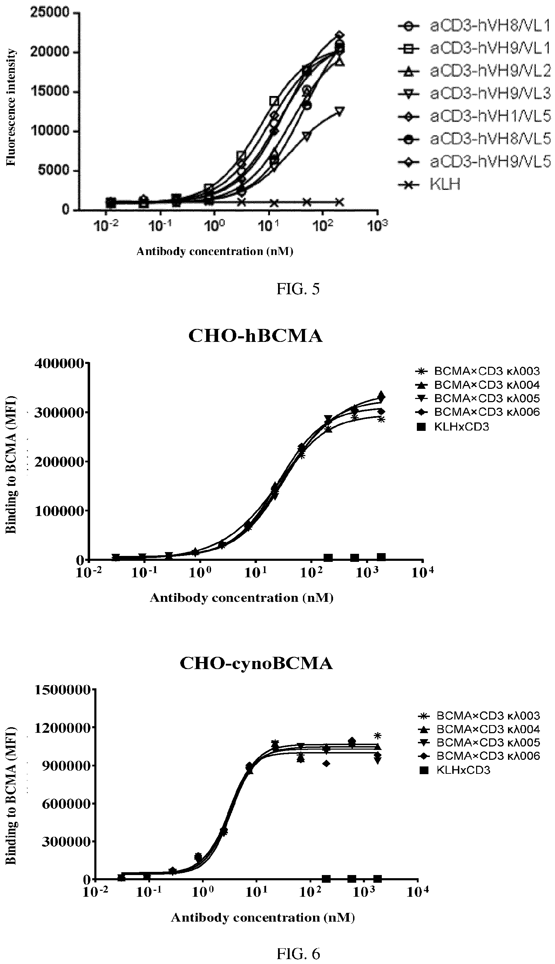

$5 \times 10^4$ cells per well, centrifuged to discard the supernatant, and added with 50 μL of gradient diluted antibody (antibody concentration: from 30 μg/mL, 3-fold dilution for 5 gradients) per well, and incubated at 4°C for 1 h. After washing off the primary antibody, a 1:300 dilution of the secondary antibody, Alexa Fluro647-labeled goat anti-human IgG Fc (Jackson ImmunoResearch, 109-606-170) was added and incubated for 45 min at 4°C. After washing, each well was resuspended in 50 μL PBS for FACS (iQue, Intellicyt) assay (results were shown in FIG. 5).

FIG. 5 shows a combination of anti-CD3 humanized antibodies bound to Jurkat cells, wherein the CD3 humanized antibody hVH9/VL5 (aCD3-hVH9/VL5) binds to Jurkat cells with moderate affinity.

### 4. Screening a combination of BCMA antibody and CD3 antibody

BCMA humanized antibodies h38E2, h23F8, h40C6, h38D9, h29A11, h15H10, and h34G2 (Table 5) were obtained by selecting some BCMA clones with higher affinity and performing CDR transplantation with the human germline genes with the highest homology. Referring to Schaefer W et al. (PNAS, 2011), the combination of humanized BCMA antibody and CD3 antibody aCD3-hVH9/VL5 (the amino acid sequence of the light chain variable region is shown in SEQ ID NO. 106, and the amino acid sequence of the heavy chain variable region is shown in SEQ ID NO. 138) was screened: the light chain variable region of the humanized BCMA antibody was cloned into a eukaryotic expression vector containing the κ light chain constant region, and the heavy chain variable region was cloned into a eukaryotic expression vector containing the mutated human IgG4 constant region; the light chain variable region of humanized CD3 antibody was cloned into a eukaryotic expression vector containing λ light chain constant region as well as heavy chain hinge region and constant regions CH2 and CH3, and the heavy chain variable region of CD3 antibody was cloned into a eukaryotic expression vector containing human CH1 constant region. The four plasmids encoding the light and heavy chain genes were mixed in the ratio of 1:1:1:1 and transfected into HEK293E cells. After expression for 5-6 days, the culture supernatant was collected. After one-step purification by Protein A, SEC-HPLC showed monomer content> 55% for relative activity evaluation. BCMA-50 (synthesized according to US201213678247) was constructed as a BCMA50-CD3 bispecific antibody with the CD3 humanized antibody as the method described above, or expressing BCMA50 × CD3 (BiTE) with reference to the literature.

Table 5. Humanized BCMA antibody variable region sequences

| BCMA human ized antibo dy | LCVR | | LCD R1 | LCD R2 | LCDR 3 | HCVR | | HCD R1 | HCDR2 | HCD R3 |
|---|---|---|---|---|---|---|---|---|---|---|
| | Ami no acid sequ ence | Nucl eotid e sequ ence | Amin o acid Seque nce | Amin o acid Seque ence | Amino acid Seque nce | Amin o acid Seque nce | Nucl eotid e Seque ence | Amin o acid Seque ence | Amino acid Sequen ce | Amin o acid Seque ence |
| h38E2 | 142 | 143 | 144 | 77 | 78 | 145 | 146 | 71 | 147 | 73 |
| h23F8 | 148 | 149 | 17 | 40 | 19 | 150 | 151 | 12 | 50 | 14 |
| h40C6 | 152 | 153 | 92 | 23 | 19 | 154 | 155 | 12 | 13 | 14 |
| h38D9 | 156 | 157 | 83 | 84 | 19 | 158 | 159 | 12 | 13 | 14 |
| h29A1 1 | 160 | 161 | 17 | 40 | 19 | 162 | 163 | 12 | 13 | 14 |
| h15H1 0 | 164 | 165 | 17 | 40 | 19 | 166 | 167 | 12 | 36 | 37 |
| h34G2 | 168 | 169 | 17 | 40 | 19 | 170 | 171 | 65 | 66 | 14 |

h38E2

The amino acid sequence of the h38E2 light chain VK is shown in SEQ ID NO. 142, of which the encoding nucleic acid is shown in SEQ ID NO. 143, and the LCDR1, LCDR2, and LCDR3 are shown in SEQ ID NOs. 144, 77 and 78, respectively.

DIQMTQSPSSLSASVGDRVTITC**QASQDVDTAVA**WYQKKPGKAPKLLIY**WTSTRH**

**T**GVPSRFSGSGSGTDFTFTISSLQPEDIATYFC**QQFSSYPLT**FGQGTKVEIK

Nucleic acid sequence

GACATCCAGATGACACAGAGCCCTAGCAGCCTGTCTGCCAGCGTGGGAGACAG
AGTGACCATCACCTGTCAGGCCAGCCAGGATGTGGATACAGCCGTGGCCTGGT
ATCAGAAGAAGCCTGGCAAGGCCCCTAAGCTGCTGATCTACTGGACCAGCACC
AGACACACAGGCGTGCCCTCTAGATTCAGCGGCTCTGGCTCTGGCACCGACTT
CACCTTCACAATCAGCAGCCTGCAGCCTGAGGATATCGCTACCTACTTCTGCCA
GCAGTTCAGCAGCTACCCTCTGACATTCGGCCAGGGCACCAAGGTGGAAATCA
AG

The amino acid sequence of the h38E2 heavy chain VH is shown in SEQ ID NO. 145, of which the encoding nucleic acid is shown in SEQ ID NO. 146, and the HCDR1, HCDR2, and HCDR3 are shown in SEQ ID NOs. 71, 147 and 73, respectively.

QVQLVQSGAEVKKPGSSVKVSCKASGYTF**TNYWIG**WVREAPGQGLEWMG**DIYP GSGDTNYNGKFQG**RVTLTADISSNTAYMELSSLRSEDTAVYYCAR**SYYRNDAASF AY**WGQGTLVTVSS

Nucleic acid sequence

CAGGTTCAGCTGGTTCAGTCTGGCGCCGAAGTGAAGAAACCTGGCAGCAGCG
TGAAGGTGTCCTGCAAGGCTAGCGGCTACACATTCACCAACTACTGGATCGGCT
GGGTCCGAGAGGCTCCTGGACAAGGACTTGAGTGGATGGGCGACATCTACCCT
GGCAGCGGCGACACAAACTACAACGGCAAGTTCCAGGGCAGAGTGACCCTGA
CAGCCGACATCAGCAGCAACACCGCCTACATGGAACTGAGCAGCCTGAGAAG
CGAGGACACCGCCGTGTACTACTGCGCCAGAAGCTACTACAGAAACGACGCCG
CCAGCTTCGCTTACTGGGGACAGGGAACACTGGTCACCGTGTCTAGT

h23F8

The amino acid sequence of the h23F8 light chain VK is shown in SEQ ID NO. 148, of which the encoding nucleic acid is shown in SEQ ID NO. 149, and the LCDR1, LCDR2, and LCDR3 are shown in SEQ ID NOs. 17, 40 and 19, respectively.

DIVLTQSPASLAVSPGQRATITC**RASKSVSTSGYSYMH**WYQKKPGQPPKLLIY**LAS**

**NLES**GVPARFSGSGSGTDFTLTINPVEANDTANYYC**QHSRELPWT**FGQGTKVEIK

Nucleic acid sequence

GACATCGTGCTGACACAGAGCCCTGCTTCTCTGGCTGTGTCTCCTGGCCAGAG

AGCCACCATCACCTGTAGAGCCAGCAAGAGCGTGTCCACCAGCGGCTACTCTT

ACATGCACTGGTATCAGAAGAAGCCCGGCCAGCCTCCTAAGCTGCTGATCTACC

TGGCTAGCAACCTCGAAAGCGGAGTGCCTGCTAGATTTTCTGGCAGCGGCTCT

GGCACCGACTTCACCCTGACAATCAACCCCGTGGAAGCCAACGACACCGCCA

ACTACTACTGCCAGCACAGCAGAGAGCTGCCCTGGACATTTGGCCAGGGCACC

AAGGTGGAAATCAAG

The amino acid sequence of the h23F8 heavy chain VH is shown in SEQ ID NO. 150, of which the encoding nucleic acid is shown in SEQ ID NO. 151, and the HCDR1, HCDR2, and HCDR3 are shown in SEQ ID NOs. 12, 50 and 14, respectively.

QVQLVQSGSELKKPGASVKVSCKASGYTF**TNFGMN**WVREAPGQGLEWMG**WIN**

**TYTGDQIYADDFKG**RFAFSLDTSASTAYLQISSLKAEDTAVYFCAR**GEIYYGYDV**

**GFVY**WGQGTLVTVSS

Nucleic acid sequence

CAGGTTCAGCTGGTGCAGTCTGGCAGCGAGCTGAAGAAACCTGGCGCCTCTGT

GAAGGTGTCCTGCAAGGCTAGCGGCTACACATTCACCAACTTCGGCATGAACT

GGGTCCGAGAGGCTCCTGGACAGGGACTCGAATGGATGGGCTGGATCAACACC

TACACCGGCGACCAGATCTACGCCGACGACTTCAAGGGCAGATTCGCCTTCAG

CCTGGACACCTCTGCCAGCACAGCTTACCTGCAGATCAGCAGCCTGAAGGCCG

AGGATACCGCCGTGTACTTCTGTGCCAGAGGCGAGATCTACTACGGCTACGATG

TGGGCTTCGTCTACTGGGGACAGGGAACACTGGTCACCGTTAGCTCT

h40C6

The amino acid sequence of the h40C6 light chain VK is shown in SEQ ID NO. 152, of which the encoding nucleic acid is shown in SEQ ID NO. 153, and the LCDR1, LCDR2, and LCDR3 are shown in SEQ ID NOs. 92, 23 and

19, respectively.

DIVLTQSPASLAVSPGQRATITC**RASRSVTTSGYSYIH**WYQKKPGQPPKLLIY**LASD LEA**GVPARFSGSGSGTDFTLTINPVEANDTANYYC**QHSRELPWT**FGQGTKVEIK

Nucleic acid sequence

GACATCGTGCTGACACAGAGCCCTGCTTCTCTGGCTGTGTCTCCTGGCCAGAG AGCCACCATCACCTGTAGAGCCAGCAGAAGCGTGACCACCAGCGGCTACTCTT ACATCCACTGGTATCAGAAGAAGCCCGGCCAGCCTCCTAAGCTGCTGATCTACC TGGCCAGCGATCTGGAAGCTGGCGTGCCAGCTAGATTTTCTGGCAGCGGCTCT GGCACCGACTTCACCCTGACAATCAACCCCGTGGAAGCCAACGACACCGCCA ACTACTACTGCCAGCACAGCAGAGAGCTGCCCTGGACATTTGGCCAGGGCACC AAGGTGGAAATCAAG

The amino acid sequence of the h40C6 heavy chain VH is shown in SEQ ID NO. 154, of which the encoding nucleic acid is shown in SEQ ID NO. 155, and the HCDR1, HCDR2, and HCDR3 are shown in SEQ ID NOs. 12, 13 and 14, respectively.

QVQLVQSGSELKKPGASVKVSCKASGYIF**TNFGMN**WVREAPGQGLEWMG**WINT YTGEQIYADDFKG**RFAFSLDTSASTAYLQISSLKAEDTAVYFCAR**GEIYYGYDVG FVY**WGQGTLVTVSS

Nucleic acid sequence

CAGGTTCAGCTGGTGCAGTCTGGCAGCGAGCTGAAGAAACCTGGCGCCTCTGT GAAGGTGTCCTGCAAGGCTAGCGGCTACATCTTCACCAACTTCGGCATGAACT GGGTCCGAGAGGCTCCTGGACAGGGACTCGAATGGATGGGCTGGATCAACACC TACACCGGCGAGCAGATCTACGCCGATGACTTCAAAGGCAGATTCGCGTTCAG CCTGGACACCAGCGCCAGCACAGCTTACCTGCAGATCAGCTCTCTGAAGGCCG AGGATACCGCCGTGTACTTCTGTGCCAGAGGCGAGATCTACTACGGCTACGACG TGGGCTTTGTGTACTGGGGCCAGGGAACACTGGTCACCGTTAGCTCT

h38D9

The amino acid sequence of the h38D9 light chain VK is shown in SEQ ID NO. 156, of which the encoding nucleic acid is shown in SEQ ID NO. 157, and the LCDR1, LCDR2, and LCDR3 are shown in SEQ ID NOs. 83, 84 and 19, respectively.

DIVLTQSPASLAVSPGQRATITC**RASKSVSTSGYSYMH**WYQKKPGQPPKLLIY**LGS**

**DLES**GVPARFSGSGSGTDFTLTINPVEANDTANYYC**QHSRELPWT**FGQGTKVEIK

Nucleic acid sequence

GACATCGTGCTGACACAGAGCCCTGCTTCTCTGGCTGTGTCTCCTGGCCAGAG

AGCCACCATCACCTGTAGAGCCAGCAAGAGCGTGTCCACCAGCGGCTACTCTT

ACATGCACTGGTATCAGAAGAAGCCCGGCCAGCCTCCTAAGCTGCTGATCTACC

TGGGTAGCGACCTCGAAAGCGGAGTGCCTGCTAGATTTTCTGGCAGCGGCTCT

GGCACCGACTTCACCCTGACAATCAACCCCGTGGAAGCCAACGACACCGCCA

ACTACTACTGCCAGCACAGCAGAGAGCTGCCCTGGACATTTGGCCAGGGCACC

AAGGTGGAAATCAAG

The amino acid sequence of the h38D9 heavy chain VH is shown in SEQ ID NO. 158, of which the encoding nucleic acid is shown in SEQ ID NO. 159, and the HCDR1, HCDR2, and HCDR3 are shown in SEQ ID NOs. 12, 13 and 14, respectively.

QVQLVQSGSELKKPGASVKVSCKASGYIF**TNFGMN**WVREAPGQGLEWMG**WINT**

**YTGEQIYADDFKG**RFAFSLDTSASTAYLQISSLKAEDTAVYFCAR**GEIYYGYDVG**

**FVY**WGQGTLVTVSS

Nucleic acid sequence

CAGGTTCAGCTGGTGCAGTCTGGCAGCGAGCTGAAGAAACCTGGCGCCTCTGT
GAAGGTGTCCTGCAAGGCTAGCGGCTACATCTTCACCAACTTCGGCATGAACT
GGGTCCGAGAGGCTCCTGGACAGGGACTCGAATGGATGGGCTGGATCAACACC
TACACCGGCGAGCAGATCTACGCCGATGACTTCAAAGGCAGATTCGCGTTCAG
CCTGGACACCAGCGCCAGCACAGCTTACCTGCAGATCAGCTCTCTGAAGGCCG
AGGATACCGCCGTGTACTTCTGTGCCAGAGGCGAGATCTACTACGGCTACGACG
TGGGCTTTGTGTACTGGGGCCAGGGAACACTGGTCACCGTTAGCTCT

h29A 11

The amino acid sequence of the h29A11 light chain VK is shown in SEQ ID NO. 160, of which the encoding nucleic acid is shown in SEQ ID NO. 161, and the LCDR1, LCDR2, and LCDR3 are shown in SEQ ID NOs. 17, 40 and 19, respectively.

DIVLTQSPASLAVSPGQRATITC**RASKSVSTSGYSYMH**WYQKKPGQPPKLLIY**LAS
NLES**GVPARFSGSGSGTDFTLTINPVEANDTANYYC**QHSRELPWT**FGQGTKVEIK

Nucleic acid sequence

GACATCGTGCTGACACAGAGCCCTGCTTCTCTGGCTGTGTCTCCTGGCCAGAG
AGCCACCATCACCTGTAGAGCCAGCAAGAGCGTGTCCACCAGCGGCTACTCTT
ACATGCACTGGTATCAGAAGAAGCCCGGCCAGCCTCCTAAGCTGCTGATCTACC

TGGCTAGCAACCTCGAAAGCGGAGTGCCTGCTAGATTTTCTGGCAGCGGCTCT
GGCACCGACTTCACCCTGACAATCAACCCCGTGGAAGCCAACGACACCGCCA
ACTACTACTGCCAGCACAGCAGAGAGCTGCCCTGGACATTTGGCCAGGGCACC
AAGGTGGAAATCAAG

The amino acid sequence of h29A11 heavy chain VH is shown in SEQ ID NO. 162, of which the encoding nucleic acid is shown in SEQ ID NO. 163, and the HCDR1, HCDR2, and HCDR3 are shown in SEQ ID NOs. 12, 13 and 14, respectively.

QVQLVQSGSELKKPGASVKVSCKASGYIF**TNFGMN**WVREAPGQGLEWMG**WINT
YTGEQIYADDFKG**RFAFSLDTSASTAYLQISSLKAEDTAVYFCAR**GEIYYGYDVG
FVY**WGQGTLVTVSS

Nucleic acid sequence

CAGGTTCAGCTGGTGCAGTCTGGCAGCGAGCTGAAGAAACCTGGCGCCTCTGT

GAAGGTGTCCTGCAAGGCTAGCGGCTACATCTTCACCAACTTCGGCATGAACT

GGGTCCGAGAGGCTCCTGGACAGGGACTCGAATGGATGGGCTGGATCAACACC

TACACCGGCGAGCAGATCTACGCCGATGACTTCAAAGGCAGATTCGCGTTCAG

CCTGGACACCAGCGCCAGCACAGCTTACCTGCAGATCAGCTCTCTGAAGGCCG

AGGATACCGCCGTGTACTTCTGTGCCAGAGGCGAGATCTACTACGGCTACGACG

TGGGCTTTGTGTACTGGGGCCAGGGAACACTGGTCACCGTTAGCTCT

h15H10

The amino acid sequence of the h15H10 light chain VK is shown in SEQ ID NO. 164, of which the encoding nucleic acid is shown in SEQ ID NO. 165, and the LCDR1, LCDR2, and LCDR3 are shown in SEQ ID NOs. 17, 40 and 19, respectively.

DIVLTQSPASLAVSPGQRATITC**RASKSVSTSGYSYMH**WYQKKPGQPPKLLIY**LAS**

**NLES**GVPARFSGSGSGTDFTLTINPVEANDTANYYC**QHSRELPWT**FGQGTKVEIK

Nucleic acid sequence

GACATCGTGCTGACACAGAGCCCTGCTTCTCTGGCTGTGTCTCCTGGCCAGAG

AGCCACCATCACCTGTAGAGCCAGCAAGAGCGTGTCCACCAGCGGCTACTCTT

ACATGCACTGGTATCAGAAGAAGCCCGGCCAGCCTCCTAAGCTGCTGATCTACC

TGGCTAGCAACCTCGAAAGCGGAGTGCCTGCTAGATTTTCTGGCAGCGGCTCT

GGCACCGACTTCACCCTGACAATCAACCCCGTGGAAGCCAACGACACCGCCA

ACTACTACTGCCAGCACAGCAGAGAGCTGCCCTGGACATTTGGCCAGGGCACC

AAGGTGGAAATCAAG

The amino acid sequence of the h15H10 heavy chain VH is shown in SEQ ID NO. 166, of which the encoding nucleic acid is shown in SEQ ID NO. 167, and the HCDR1, HCDR2, and HCDR3 are shown in SEQ ID NOs. 12, 36 and 37, respectively.

QVQLVQSGSELKKPGASVKVSCKASGYTF**TNFGMN**WVREAPGQGLEWMG**WIN TYTGDLIYGDDFKG**RFAFSLDTSASTAYLQISSLKAEDTAVYFCAR**GEIYYGYDV GFAY**WGQGTLVTVSS

Nucleic acid sequence

CAGGTTCAGCTGGTGCAGTCTGGCAGCGAGCTGAAGAAACCTGGCGCCTCTGT GAAGGTGTCCTGCAAGGCTAGCGGCTACACATTCACCAACTTCGGCATGAACT GGGTCCGAGAGGCTCCTGGACAGGGACTCGAATGGATGGGCTGGATCAACACC TACACCGGCGACCTGATCTACGGCGACGACTTCAAGGGCAGATTCGCCTTCAG CCTGGACACCTCTGCCAGCACAGCTTACCTGCAGATCAGCAGCCTGAAGGCCG AGGATACCGCCGTGTACTTCTGTGCCAGAGGCGAGATCTACTACGGCTACGATG TGGGCTTCGCCTACTGGGGACAGGGAACACTGGTCACCGTTAGCTCT

h34G2

The amino acid sequence of the h34G2 light chain VK is shown in SEQ ID NO. 168, of which the encoding nucleic acid is shown in SEQ ID NO. 169, and the LCDR1, LCDR2, and LCDR3 are shown in SEQ ID NOs. 17, 40 and 19, respectively.

DIVLTQSPASLAVSPGQRATITC**RASKSVSTSGYSYMH**WYQKKPGQPPKLLIY**LAS NLES**GVPARFSGSGSGTDFTLTINPVEANDTANYYC**QHSRELPWT**FGQGTKVEIK

Nucleic acid sequence

GACATCGTGCTGACACAGAGCCCTGCTTCTCTGGCTGTGTCTCCTGGCCAGAG AGCCACCATCACCTGTAGAGCCAGCAAGAGCGTGTCCACCAGCGGCTACTCTT ACATGCACTGGTATCAGAAGAAGCCCGGCCAGCCTCCTAAGCTGCTGATCTACC TGGCTAGCAACCTCGAAAGCGGAGTGCCTGCTAGATTTTCTGGCAGCGGCTCT GGCACCGACTTCACCCTGACAATCAACCCCGTGGAAGCCAACGACACCGCCA ACTACTACTGCCAGCACAGCAGAGAGCTGCCCTGGACATTTGGCCAGGGCACC AAGGTGGAAATCAAG

The amino acid sequence of the h34G2 heavy chain VH is shown in SEQ ID NO. 170, of which the encoding nucleic acid is shown in SEQ ID NO. 171, and the HCDR1, HCDR2, and HCDR3 are shown in SEQ ID NOs. 65, 66 and

14, respectively.

QVQLVQSGSELKKPGASVKVSCKASGYTF**TNYGMN**WVREAPGQGLEWMG**WIN TYTGERIYADDFKG**RFAFSLDTSASTAYLQISSLKAEDTAVYFCAR**GEIYYGYDV GFVY**WGQGTLVTVSS

Nucleic acid sequence

CAGGTTCAGCTGGTGCAGTCTGGCAGCGAGCTGAAGAAACCTGGCGCCTCTGT GAAGGTGTCCTGCAAGGCTAGCGGCTACACATTCACCAACTACGGCATGAACT GGGTCCGAGAGGCTCCTGGACAGGGACTCGAATGGATGGGCTGGATCAACACC TACACCGGCGAAAGGATCTACGCCGACGACTTCAAGGGCAGATTCGCCTTCAG CCTGGACACCTCTGCCAGCACAGCTTACCTGCAGATCAGCAGCCTGAAGGCCG AGGATACCGCCGTGTACTTCTGTGCCAGAGGCGAGATCTACTACGGCTACGATG TGGGCTTCGTCTACTGGGGACAGGGAACACTGGTCACCGTTAGCTCT

**5.** T-cell-dependent cellular cytotoxicity assay (TDCC)

Freshly isolated PBMC was mixed with RPMI-8226 cells in the logarithmic growth phase (effector/target cell = 10:1), and 50 $\mu$L of gradient diluted antibody (initial concentration 40 $\mu$g/mL, 10-fold dilution for 7 gradients) was added to each well, and cultured at 37°C in 5% $CO_2$ for 24 h. After the end of the culture, centrifuging at 1000 rpm for 3 min, the supernatant was taken to detect the release of LDH, transferred 50 $\mu$L of the supernatant to a black ELISA plate, added with 50 $\mu$L/well of LDH detection substrate, 10 min later, stop the reaction and detect (Biotek Synergy HT), and the killing rate was calculated according to the following formula; after discarding the supernatant, the remaining cells in the wells were washed twice with 4% calf blood, blocked with 100 $\mu$g/mL human IgG for 10 min, and T cell early activation marker CD69 and late marker CD25 were added to detect antibodies (CD25-PE, CD4-APC, CD69-FITC, and CD8-APC) and incubating on ice for 20 min. After the incubation, 200 $\mu$L of 4% calf blood was added to each well for washing three times, the supernatant was discarded, 60 $\mu$L/well propidium iodide (PI) solution (1:200 dilution) was added, incubated on ice for 5 min, and detected by flow cytometer (BD FACS celesta). The formula for calculating the killing rate is as follows:

$$\text{Killing Rate(\%)} = \frac{\text{Experimental well} - \text{Target cell spontaneous}}{\text{Target cell maximum release} - \text{Target cell spontaneous}} \times 100\%$$

The killing activity of the bispecific antibody combination is shown in Table 6.

Table 6. Killing activity of bispecific antibodies

| Antibodies\ Units (nM) | SEC-HPLC Monom er % | Killin g EC$_{50}$ | Upregulation of CD69 EC$_{50}$ (%) | | Upregulation of CD25 EC$_{50}$ (%) | |
|---|---|---|---|---|---|---|
| | | | CD8$^+$T | CD4$^+$T | CD8$^+$T | CD4$^+$T |
| **h38E2-CD3** | 56.2 | 13.64 | 2.32(69) | 7.40(55) | 3.65(38) | 3.65(29) |
| **h23F8-CD3** | 58.4 | 0.81 | 0.49(59) | 0.44(41) | 0.79(34) | 0.57(25) |

(continued)

| Antibodies\ Units (nM) | SEC-HPLC Monom er % | Killin g $EC_{50}$ | Upregulation of CD69 $EC_{50}$ (%) | | Upregulation of CD25 $EC_{50}$ (%) | |
|---|---|---|---|---|---|---|
| | | | CD8+T | CD4+T | CD8+T | CD4+T |
| **h40C6-CD3** | 57.3 | 1.12 | 0.21(44) | 0.63(37) | 0.49(28) | 0.45(25) |
| **h38D9-CD3** | 55.1 | 1.04 | 0.37(52) | 0.52(57) | 1.75(35) | 0.77(29) |
| **h29A11-CD3** | 56.3 | 0.69 | 3.39(58) | 0.37(47) | 3.85(36) | 0.45(33) |
| **h15H10-CD3** | 67.6 | 1.83 | 0.94(62) | 3.39(58) | 2.64(41) | 3.85(36) |
| **h34G2-CD3** | 64.0 | 1.02 | 0.57(55) | 0.68(42) | 1.28(33) | 1.18(27) |
| **BCMA50-CD3** | 40.9 | 3.84 | 6.51(56) | 1.02(34) | 9.86(24) | 7.94(22) |
| **BCMA50×CD3 ( BiTE)** | N.D. | 0.81 | 0.16(65) | 1.90(52) | 0.30(39) | 0.64(27) |
| **KLH-CD3** | 85.8 | - | -(11) | -(15) | -(8) | -(10) |

## 6. Construction of BCMA×CD3 κλ humanized bispecific antibody

Clone 29A11 with higher killing efficiency and clone 6G5 with homologous sequence were selected for humanized sequence optimization. The heavy chain variable region was derived from murine IGHV9-3, and the light chain variable region was derived from murine IGKV3-12. The light and heavy chain CDR grafts were transplanted into human germline genes IGHV7-4-1*02 and IGVK_7_3, respectively. The humanized BCMA antibody sequences were synthesized and designed. Meanwhile, charge variants were introduced in the BCMA antigen arm and the humanized CD3 antibody arm containing the λ light chain ($V\kappa_{BCMA}$: $Gln_{42}Lys$; $VH_{BCMA}$: $Gln_{39}Glu$; $V\lambda_{CD3}$: $Gln_{40}Glu$; $VH_{CD3}$: $Gln_{39}Lys$) to construct a novel BCMA-CD3 κλ humanized bispecific antibody with the native IgG configuration (Table 7). The Fc portion of the bispecific antibody adopts the human IgG4 knob-into-hole structure to achieve heterodimer pairing (Atwell et al. 1997), and through mutations $Ser_{228}Pro$, $Leu_{235}Glu$, and $Pro_{329}Ala$, the hinge region is kept stable and interactions with Fcγ receptors and C1q are reduced.

Plasmids encoding the corresponding antibody fragments were mixed in a ratio of κ light chain: λ light chain: heavy chain 1: heavy chain 2 = 2:2:1:1 with 3 mg/mL PEI, then co-transfected into CHO-S cells and cultured in 500 mL CD CHO AGT medium (Gibco #12490-001) at 37°C, 5% $CO_2$ 150 rpm. 4% CHO Feed C+ supplement (Gibco #A25031-05) was added on the 2nd, 4th and 6th day of transient transfection, respectively. When the cell viability decreased to about 85%, the fermentation broth was harvested, filtered, and purified by Protein A affinity chromatography, and the SEC-HPLC monomer content was higher than 92%; through Capto S ImpAct ion exchange chromatography, 50-300 mM NaCl/ phosphate pH6.0 gradient elution and combined elution peak, the monomer content was further increased to more than 98-99% (Table 8).

**EP 4 257 607 A1**

Table 7. BCMA×CD3 κλ bispecific antibodies

| Bispecific antibody | BCMA antibody | CD3 antibody |
|---|---|---|
| BCMA×CD3 κλ003, introduction of charge variants in the BCMA and CD3 antigen arms: $V\kappa_{BCMA}$: $Gln_{42}Lys$; $VH_{BCMA}$: $Gln_{39}Glu$; $V\lambda_{CD3}$: $Gln_{40}Glu$; $VH_{CD3}$: $Gln_{39}Lys$ | Light chain 1: the amino acid sequence thereof is shown in SEQ ID NO. 172, and the encoding nucleic acid thereof is shown in SEQ ID NO. 173 DIVLTQSPASLAVSPGQRATI TCRASKSVSTSGYSYMHWY QKKPGQPPKLLIYLASNLES GVPARFSGSGSGTDFTLTINP VEAEDTANYYCQHSRELPW TFGQGTKVEIKRTVAAPSVFI FPPSDEQLKSGTASVVCLLN NFYPREAKVQWKVDNALQ SGNSQESVTEQDSKDSTYSL SSTLTLSKADYEKHKVYACE | Light chain 2: the amino acid sequence thereof is shown in SEQ ID NO. 180, and the encoding nucleic acid thereof is shown in SEQ ID NO. 181 QAVVTQEPSLTVSPGGTVTLT CRSSTGAVTTSNYANWVQEK PGQAPRGLIGGTNKRAPWTP ARFSGSLLGGKAALTITGAQ AEDEAEYYCVLWYSNLWVF GGGTKLTVLGQPKAAPSVTL FPPSSEELQANKATLVCLISDF YPGAVTVAWKADSSPVKAG VETTTPSKQSNNKYAASSYL SLTPEQWKSHRSYSCQVTHE |

(continued)

| Bispecific antibody | BCMA antibody | CD3 antibody |
|---|---|---|
| | VTHQGLSSPVTKSFNRGEC<br><br>Heavy chain 1: the amino acid sequence thereof is shown in SEQ ID NO. 174, and the encoding nucleic acid thereof is shown in SEQ ID NO. 175<br><br>QVQLVQSGSELKKPGASVK<br><br>VSCKASGYIFTNFGMNWVR<br><br>EAPGQGLEWMGWINTYTG<br><br>EQIYADGFTGRFVFSLDTSA<br><br>STAYLQISSLKAEDTAVYFC<br><br>ARGEIYYGYDVGFVYWGQ<br><br>GTLVTVSSASTKGPSVFPLA<br><br>PCSRSTSESTAALGCLVKDY<br><br>FPEPVTVSWNSGALTSGVHT<br><br>FPAVLQSSGLYSLSSVVTVPS<br><br>SSLGTKTYTCNVDHKPSNT<br><br>KVDKRVESKYGPPCPPCPAP<br><br>EFEGGPSVFLFPPKPKDTLMI<br><br>SRTPEVTCVVVDVSQEDPEV<br><br>QFNWYVDGVEVHNAKTKP<br><br>REEQFNSTYRVVSVLTVLHQ<br><br>DWLNGKEYKCKVSNKGLA<br><br>SSIEKTISKAKGQPREPQVCT<br><br>LPPSQEEMTKNQVSLSCAVK<br><br>GFYPSDIAVEWESNGQPENN<br><br>YKTTPPVLDSDGSFFLVSKL<br><br>TVDKSRWQEGNVFSCSVMH<br><br>EALHNHYTQKSLSLSLGK | GSTVEKTVAPTECS<br><br>Heavy chain 2: the amino acid sequence thereof is shown in SEQ ID NO. 182, and the encoding nucleic acid thereof is shown in SEQ ID NO. 183<br><br>EVQLVESGGGLVQPGGSLRL<br><br>SCAASGFTFNTYAMNWVRK<br><br>APGKGLEWVGRIRSKYNNYA<br><br>TYYADSVKDRFTISRDDSKN<br><br>SLYLQMNSLKTEDTAVYYCV<br><br>RHGNFGNSYVSWFAYWGQG<br><br>TLVTVSSASTKGPSVFPLAPC<br><br>SRSTSESTAALGCLVKDYFPE<br><br>PVTVSWNSGALTSGVHTFPA<br><br>VLQSSGLYSLSSVVTVPSSSL<br><br>GTKTYTCNVDHKPSNTKVD<br><br>KRVESKYGPPCPPCPAPEFEG<br><br>GPSVFLFPPKPKDTLMISRTP<br><br>EVTCVVVDVSQEDPEVQFN<br><br>WYVDGVEVHNAKTKPREEQ<br><br>FNSTYRVVSVLTVLHQDWLN<br><br>GKEYKCKVSNKGLASSIEKTI<br><br>SKAKGQPREPQVYTLPPCQE<br><br>EMTKNQVSLWCLVKGFYPS<br><br>DIAVEWESNGQPENNYKTTP<br><br>PVLDSDGSFFLYSKLTVDKSR<br><br>WQEGNVFSCSVMHEALHNH<br><br>YTQKSLSLSLGK |
| BCMA×CD3<br>κλ004 | Light chain 1: the amino acid sequence thereof is shown in SEQ ID NO. 176, and the | Light chain 2: the amino acid sequence thereof is shown in SEQ ID NO. 180, and the |

(continued)

| Bispecific antibody | BCMA antibody | CD3 antibody |
|---|---|---|
| introduction of charge variants in the BCMA and CD3 antigen arms: $V\kappa_{BCMA}$: $Gln_{42}Lys$; $VH_{BCMA}$: $Gln_{39}Glu$; $V\lambda_{CD3}$: $Gln_{40}Glu$; $VH_{CD3}$: $Gln_{39}Lys$ | encoding nucleic acid thereof is shown in SEQ ID NO. 177<br><br>DIVLTQSPASLAVSPGQRATI<br>TCRASKSVTTSGYSYIHWY<br>QKKPGQPPKLLIYLASDLEA<br>GVPARFSGSGSGTDFTLTINP<br>VEAEDTANYYCQHSRELPW<br>TFGQGTKVEIKRTVAAPSVFI<br>FPPSDEQLKSGTASVVCLLN<br>NFYPREAKVQWKVDNALQ<br>SGNSQESVTEQDSKDSTYSL<br>SSTLTLSKADYEKHKVYACE<br>VTHQGLSSPVTKSFNRGEC<br>Heavy chain 1: the amino acid sequence thereof is shown in SEQ ID NO. 174, and the encoding nucleic acid thereof is shown in SEQ ID NO. 175<br><br>QVQLVQSGSELKKPGASVK<br>VSCKASGYIFTNFGMNWVR<br>EAPGQGLEWMGWINTYTG<br>EQIYADGFTGRFVFSLDTSA<br>STAYLQISSLKAEDTAVYFC<br>ARGEIYYGYDVGFVYWGQ<br>GTLVTVSSASTKGPSVFPLA<br>PCSRSTSESTAALGCLVKDY<br>FPEPVTVSWNSGALTSGVHT<br>FPAVLQSSGLYSLSSVVTVPS<br>SSLGTKTYTCNVDHKPSNT<br>KVDKRVESKYGPPCPPCPAP<br>EFEGGPSVFLFPPKPKDTLMI | encoding nucleic acid thereof is shown in SEQ ID NO. 181<br><br>QAVVTQEPSLTVSPGGTVTLT<br>CRSSTGAVTTSNYANWVQEK<br>PGQAPRGLIGGTNKRAPWTP<br>ARFSGSLLGGKAALTITGAQ<br>AEDEAEYYCVLWYSNLWVF<br>GGGTKLTVLGQPKAAPSVTL<br>FPPSSEELQANKATLVCLISDF<br>YPGAVTVAWKADSSPVKAG<br>VETTTPSKQSNNKYAASSYL<br>SLTPEQWKSHRSYSCQVTHE<br>GSTVEKTVAPTECS<br>Heavy chain 2: the amino acid sequence thereof is shown in SEQ ID NO. 182, and the encoding nucleic acid thereof is shown in SEQ ID NO. 183<br><br>EVQLVESGGGLVQPGGSLRL<br>SCAASGFTFNTYAMNWVRK<br>APGKGLEWVGRIRSKYNNYA<br>TYYADSVKDRFTISRDDSKN<br>SLYLQMNSLKTEDTAVYYCV<br>RHGNFGNSYVSWFAYWGQG<br>TLVTVSSASTKGPSVFPLAPC<br>SRSTSESTAALGCLVKDYFPE<br>PVTVSWNSGALTSGVHTFPA<br>VLQSSGLYSLSSVVTVPSSSL<br>GTKTYTCNVDHKPSNTKVD<br>KRVESKYGPPCPPCPAPEFEG<br>GPSVFLFPPKPKDTLMISRTP |

| Bispecific antibody | BCMA antibody | CD3 antibody |
|---|---|---|
| | SRTPEVTCVVVDVSQEDPEV QFNWYVDGVEVHNAKTKP REEQFNSTYRVVSVLTVLHQ DWLNGKEYKCKVSNKGLA SSIEKTISKAKGQPREPQVCT LPPSQEEMTKNQVSLSCAVK GFYPSDIAVEWESNGQPENN YKTTPPVLDSDGSFFLVSKL TVDKSRWQEGNVFSCSV<br><br>MHEALHNHYTQKSLSLSLG K | EVTCVVVDVSQEDPEVQFN WYVDGVEVHNAKTKPREEQ FNSTYRVVSVLTVLHQDWLN GKEYKCKVSNKGLASSIEKTI SKAKGQPREPQVYTLPPCQE EMTKNQVSLWCLVKGFYPS DIAVEWESNGQPENNYKTTP PVLDSDGSFFLYSKLTVDKSR WQEGNVFSCSVMHEALHNH YTQKSLSLSLGK |
| BCMA×CD3 κλ005 introduction of charge variants in the BCMA and CD3 antigen arms:<br>V$\kappa_{BCMA}$: Gln$_{42}$Lys;<br>VH$_{BCMA}$: Gln$_{39}$Glu; V$\lambda_{CD3}$: Gln$_{40}$Glu; VH$_{CD3}$: Gln$_{39}$Lys | Light chain 1: the amino acid sequence thereof is shown in SEQ ID NO. 172, and the encoding nucleic acid thereof is shown in SEQ ID NO. 173<br>DIVLTQSPASLAVSPGQRATI TCRASKSVSTSGYSYMHWY QKKPGQPPKLLIYLASNLES GVPARFSGSGSGTDFTLTINP VEAEDTANYYCQHSRELPW TFGQGTKVEIKRTVAAPSVFI FPPSDEQLKSGTASVVCLLN NFYPREAKVQWKVDNALQ SGNSQESVTEQDSKDSTYSL SSTLTLSKADYEKHKVYACE VTHQGLSSPVTKSFNRGEC<br>Heavy chain 1: the amino acid sequence thereof is shown in SEQ ID NO. 178, and the encoding nucleic acid thereof is | Light chain 2: the amino acid sequence thereof is shown in SEQ ID NO. 180, and the encoding nucleic acid thereof is shown in SEQ ID NO. 181<br>QAVVTQEPSLTVSPGGTVTLT CRSSTGAVTTSNYANWVQEK PGQAPRGLIGGTNKRAPWTP ARFSGSLLGGKAALTITGAQ AEDEAEYYCVLWYSNLWVF GGGTKLTVLGQPKAAPSVTL FPPSSEELQANKATLVCLISDF YPGAVTVAWKADSSPVKAG VETTTPSKQSNNKYAASSYL SLTPEQWKSHRSYSCQVTHE GSTVEKTVAPTECS<br>Heavy chain 2: the amino acid sequence thereof is shown in SEQ ID NO. 182, and the encoding nucleic acid thereof is |

(continued)

| Bispecific antibody | BCMA antibody | CD3 antibody |
|---|---|---|
| | shown in SEQ ID NO. 179<br><br>QVQLVQSGSELKKPGASVK VSCKASGYIFTNFGMNWVR EAPGQGLEWMGWINTYTG EQIYADGFTGRFVFSLDTSV STAYLQISSLKAEDTAVYFC ARGEIYYGYDVGFVYWGQ GTLVTVSSASTKGPSVFPLA PCSRSTSESTAALGCLVKDY FPEPVTVSWNSGALTSGVHT FPAVLQSSGLYSLSSVVTVPS SSLGTKTYTCNVDHKPSNT KVDKRVESKYGPPCPPCPAP EFEGGPSVFLFPPKPKDTLMI SRTPEVTCVVVDVSQEDPEV QFNWYVDGVEVHNAKTKP REEQFNSTYRVVSVLTVLHQ DWLNGKEYKCKVSNKGLA SSIEKTISKAKGQPREPQVCT LPPSQEEMTKNQVSLSCAVK GFYPSDIAVEWESNGQPENN YKTTPPVLDSDGSFFLVSKL TVDKSRWQEGNVFSCSVMH EALHNHYTQKSLSLSLGK | shown in SEQ ID NO. 183<br><br>EVQLVESGGGLVQPGGSLRL SCAASGFTFNTYAMNWVRK APGKGLEWVGRIRSKYNNYA TYYADSVKDRFTISRDDSKN SLYLQMNSLKTEDTAVYYCV RHGNFGNSYVSWFAYWGQG TLVTVSSASTKGPSVFPLAPC SRSTSESTAALGCLVKDYFPE PVTVSWNSGALTSGVHTFPA VLQSSGLYSLSSVVTVPSSSL GTKTYTCNVDHKPSNTKVD KRVESKYGPPCPPCPAPEFEG GPSVFLFPPKPKDTLMISRTP EVTCVVVDVSQEDPEVQFN WYVDGVEVHNAKTKPREEQ FNSTYRVVSVLTVLHQDWLN GKEYKCKVSNKGLASSIEKTI SKAKGQPREPQVYTLPPCQE EMTKNQVSLWCLVKGFYPS DIAVEWESNGQPENNYKTTP PVLDSDGSFFLYSKLTVDKSR WQEGNVFSCSVMHEALHNH YTQKSLSLSLGK |
| BCMA×CD3 κλ006 introduction of charge variants in the BCMA and CD3 antigen arms: | Light chain 1: the amino acid sequence thereof is shown in SEQ ID NO. 176, and the encoding nucleic acid thereof is shown in SEQ ID NO. 177 | Light chain 2: the amino acid sequence thereof is shown in SEQ ID NO. 180, and the encoding nucleic acid thereof is shown in SEQ ID NO. 181 |

(continued)

| Bispecific antibody | BCMA antibody | CD3 antibody |
|---|---|---|
| V$\kappa_{BCMA}$: Gln$_{42}$Lys; | DIVLTQSPASLAVSPGQRATI TCRASKSVTTSGYSYIHWY QKKPGQPPKLLIYLASDLEA | QAVVTQEPSLTVSPGGTVTLT CRSSTGAVTTSNYANWVQEK PGQAPRGLIGGTNKRAPWTP |

(continued)

| Bispecific antibody | BCMA antibody | CD3 antibody |
|---|---|---|
| VH_BCMA: Gln39Glu; Vλ_CD3: Gln40Glu; VH_CD3: Gln39Lys | GVPARFSGSGSGTDFTLTINP VEAEDTANYYCQHSRELPW TFGQGTKVEIKRTVAAPSVFI FPPSDEQLKSGTASVVCLLN NFYPREAKVQWKVDNALQ SGNSQESVTEQDSKDSTYSL SSTLTLSKADYEKHKVYACE VTHQGLSSPVTKSFNRGEC Heavy chain 1: the amino acid sequence thereof is shown in SEQ ID NO. 178, and the encoding nucleic acid thereof is shown in SEQ ID NO. 179 QVQLVQSGSELKKPGASVK VSCKASGYIFTNFGMNWVR EAPGQGLEWMGWINTYTG EQIYADGFTGRFVFSLDTSV STAYLQISSLKAEDTAVYFC ARGEIYYGYDVGFVYWGQ GTLVTVSSASTKGPSVFPLA PCSRSTSESTAALGCLVKDY FPEPVTVSWNSGALTSGVHT FPAVLQSSGLYSLSSVVTVPS SSLGTKTYTCNVDHKPSNT KVDKRVESKYGPPCPPCPAP EFEGGPSVFLFPPKPKDTLMI SRTPEVTCVVVDVSQEDPEV QFNWYVDGVEVHNAKTKP REEQFNSTYRVVSVLTVLHQ DWLNGKEYKCKVSNKGLA SSIEKTISKAKGQPREPQVCT LPPSQEEMTKNQVSLSCAVK | ARFSGSLLGGKAALTITGAQ AEDEAEYYCVLWYSNLWVF GGGTKLTVLGQPKAAPSVTL FPPSSEELQANKATLVCLISDF YPGAVTVAWKADSSPVKAG VETTTPSKQSNNKYAASSYL SLTPEQWKSHRSYSCQVTHE GSTVEKTVAPTECS Heavy chain 2: the amino acid sequence thereof is shown in SEQ ID NO. 182, and the encoding nucleic acid thereof is shown in SEQ ID NO. 183 EVQLVESGGGLVQPGGSLRL SCAASGFTFNTYAMNWVRK APGKGLEWVGRIRSKYNNYA TYYADSVKDRFTISRDDSKN SLYLQMNSLKTEDTAVYYCV RHGNFGNSYVSWFAYWGQG TLVTVSSASTKGPSVFPLAPC SRSTSESTAALGCLVKDYFPE PVTVSWNSGALTSGVHTFPA VLQSSGLYSLSSVVTVPSSSL GTKTYTCNVDHKPSNTKVD KRVESKYGPPCPPCPAPEFEG GPSVFLFPPKPKDTLMISRTP EVTCVVVDVSQEDPEVQFN WYVDGVEVHNAKTKPREEQ FNSTYRVVSVLTVLHQDWLN GKEYKCKVSNKGLASSIEKTI SKAKGQPREPQVYTLPPCQE EMTKNQVSLWCLVKGFYPS |

(continued)

| Bispecific antibody | BCMA antibody | CD3 antibody |
|---|---|---|
| | GFYPSDIAVEWESNGQPENN YKTTPPVLDSDGSFFLVSKL TVDKSRWQEGNVFSCSVMH EALHNHYTQKSLSLSLGK | DIAVEWESNGQPENNYKTTP PVLDSDGSFFLYSKLTVDKSR WQEGNVFSCSVMHEALHNH YTQKSLSLSLGK |

Table 8. Purity of BCMA×CD3 κλ bispecific antibody

| HPLC detection | HPLC-SEC (after Protein A) | | | HPLC-SEC (after cation exchange chromatography) | | |
|---|---|---|---|---|---|---|
| | Mer | Monom er | Fragm ent | Mer | Monome r | Fragmen t |
| BCMA×CD3 κλ003 | 2.3% | 94.4% | 3.3% | 1.8% | 98.2% | 0.0% |
| BCMA×CD3 κλ004 | 1.4% | 95.2% | 3.4% | 1.0% | 99.0% | 0.0% |
| BCMA×CD3 κλ005 | 2.0% | 91.9% | 6.2% | 1.6% | 98.4% | 0.0% |
| BCMA×CD3 κλ006 | 0.5% | 96.4% | 3.1% | 0.6% | 99.4% | 0.0% |

## Example 4. Binding activity of BCMA×CD3 κλ bispecific antibody

The affinity of the bispecific antibody BCMA antigen arm was determined by measuring binding to BCMA recombinant protein, overexpressing stably transfected cells, or BCMA+ tumor cells, respectively, and the affinity of the bispecific antibody CD3 arm was determined by measuring binding to CD3 recombinant antigen, Jurkat cells, or freshly isolated peripheral blood T cells.

## 1. Affinity assay of BCMA×CD3 κλ bispecific antibody to antigen

10 μg/mL human or cynomolgus monkey BCMA or CD3εγ recombinant antigen was bound to a CM5 chip (GE healthcare) by amino coupling, controlling the amount of antigen bound to approximately 200 RU. After the baseline plateau, gradient-diluted antibodies (from 10 μg/mL, 2-fold dilutions for 7 gradients) flowed through the chip at a flow rate of 30 μL/min for a binding time of 350 seconds and a dissociation time of 600 seconds. Kinetic constants were fitted using Biacore T200 evaluation software with a 1:1 binding model. The results of the affinity assay are shown in Table 9.

Table 9. Affinity of BCMA×CD3 κλ bispecific antibodies to BCMA and CD3 recombinant antigens

| KD (nM) | Human BCMA | Cynomolgus monkey BCMA | Human CD3εγ | Cynomolgus monkey CD3εγ |
|---|---|---|---|---|
| BCMA×CD3 κλ003 | 1.3 | 0.16 | 31 | 43 |
| BCMA×CD3 κλ004 | 1.0 | 0.12 | 32 | 43 |
| BCMA×CD3 κλ005 | 1.1 | 0.09 | 28 | 20 |
| BCMA×CD3 κλ006 | 1.2 | 0.10 | 27 | 33 |

## 2. Binding of BCMA×CD3 κλ bispecific antibody to BCMA stably transfected cells

CHO-human BCMA and CHO-cynomolgus monkey BCMA stably transfected cells in the logarithmic growth phase prepared in Example 1 were adjusted to $5 \times 10^5$ cells/ml with 4% calf serum (Hyclone, SH30626.06), 100 μl/well cell suspension was added to a 96-well U-plate, centrifuged at 300g for 5 min, the supernatant was discarded, and 100 μL of gradient diluted antibody (initial concentration 1800 nM, 3-fold dilution for 10 gradients) was added to each well and incubated at 4°C for 60 min. 50 μL/well Alexa Fluro647 labeled goat anti-human IgG Fc (1:300 dilution) was added to the secondary antibody, incubated on ice for 20 min, 50 μL/well PI solution (1:300) was added after washing once, incubated for 5 min, and flow cytometer was used to perform detection. FIG. 6 and Table 10 show that the BCMA×CD3 κλ bispecific antibody binds to human, cynomolgus monkey BCMA stably transfected cells with high affinity.

Table 10. Binding of BCMA×CD3 κλ bispecific antibodies to BCMA stably transfected cells

| EC$_{50}$ (nM) | Human BCMA-CHO | Cynomolgus monkey BCMA-CHO |
|---|---|---|
| BCMA×CD3 κλ003 | 16 | 3 |
| BCMA×CD3 κλ004 | 22 | 3 |
| BCMA×CD3 κλ005 | 24 | 3 |
| BCMA×CD3 κλ006 | 16 | 3 |
| KLH×CD3 | - | - |

### 3. Binding of BCMA×CD3 κλ bispecific antibody to BCMA$^+$ tumor cells

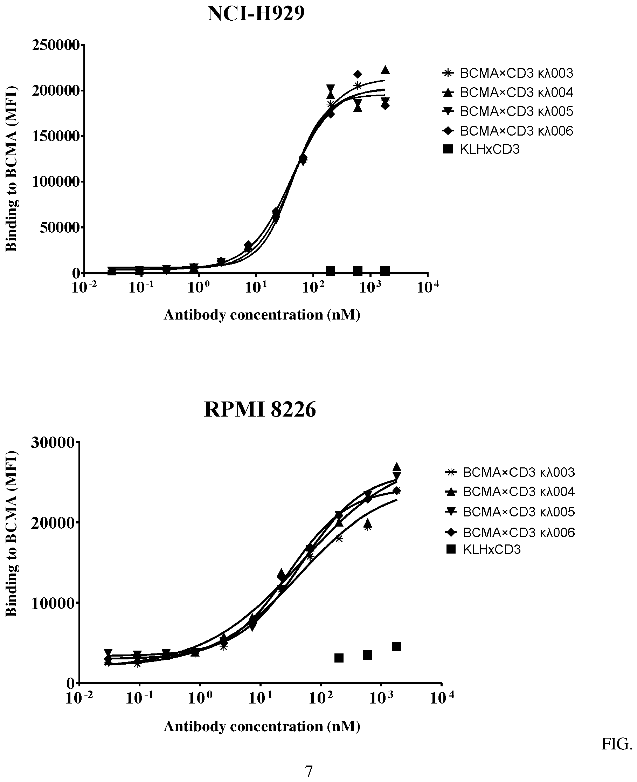

NCI-H929 and RPMI-8226 cells in the logarithmic growth phase were added with 200 μg/mL murine IgG (Jackson ImmunoResearch, 115-005-03), blocked with an ice bath for 30 min, adjusted cells to $5 \times 10^5$ cells/mL with 4% calf serum, 100 μL/well was added to 96-well U-plate, centrifuged at 300g for 5 min, the supernatant was discarded, and 100 μL of gradient diluted antibody (initial concentration 1800 nM, 3-fold dilution for 10 gradients) was added to each well and incubated at 4°C for 60 min. The primary antibody was washed off, 50 μL/well Alexa Fluro647 labeled goat anti-human IgG Fc (1:300 dilution) was added, incubated on ice for 20 min, 50 μL/well PI was added after washing once, incubated for 5 min, and flow cytometer was used to perform detection. The results are shown in FIG. 7 and Table 11. the BCMA×CD3 κλ bispecific antibody binds to BCMA+ tumor cells NCI-H929 and RPMI-8226 with high affinity.

Table 11. Binding of BCMA×CD3 κλ bispecific antibodies to BCMA+ tumor cells

| EC$_{50}$(nM) | NCI-H929 | RPMI-8226 |
|---|---|---|
| BCMA×CD3 κλ003 | 34 | 34 |
| BCMA×CD3 κλ004 | 36 | 39 |
| BCMA×CD3 κλ005 | 33 | 38 |
| BCMA×CD3 κλ006 | 32 | 19 |
| KLH×CD3 | - | - |

### 4. Binding of BCMA×CD3 κλ bispecific antibody to Jurkat cells

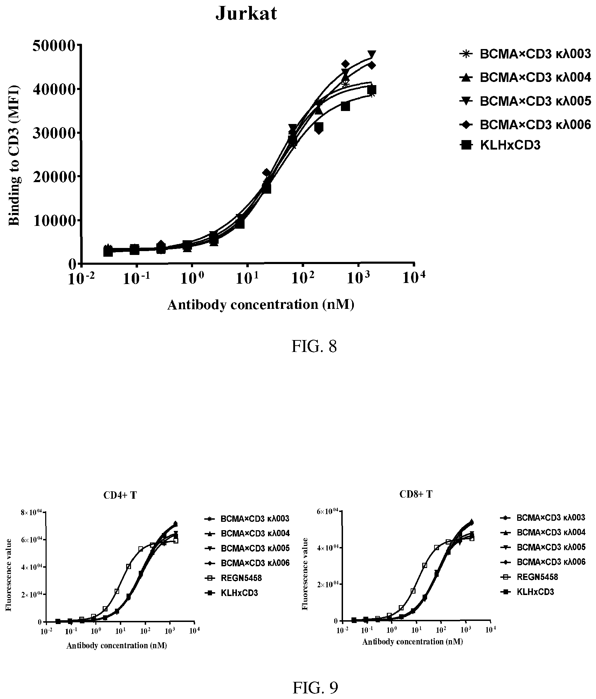

Jurkat cells in the logarithmic growth phase were added with 200 μg/mL murine IgG (Jackson ImmunoResearch, 115-005-03) and incubated on ice for 30 min. Adjusted cells to $5 \times 10^5$ cells/mL with 4% calf serum, 100 μL/well was added to 96-well U-plate, centrifuged at 300g to remove the supernatant, and 100 μL of gradient diluted antibody (initial concentration 1800 nM, 3-fold dilution for 10 gradients) was added to each well and incubated at 4°C for 60 min. 50 μL/well Alexa Fluro647 labeled goat anti-human IgG Fc (1:300 dilution) was added to the secondary antibody, incubated on ice for 20 min, 50 μL/well PI was added after washing once, incubated for 5 min, and flow cytometer (BD C6) was used to perform detection. The results are shown in FIG. 8 and Table 12. The BCMA×CD3 κλ bispecific antibody binds to human leukemia T cell line Jurkat cells with moderate affinity.

Table 12. Binding of BCMA×CD3 κλ bispecific antibodies to Jurkat cells

| EC$_{50}$(nM) | Jurkat |
|---|---|
| BCMA×CD3 κλ003 | 66 |
| BCMA×CD3 κλ004 | 61 |
| BCMA×CD3 κλ005 | 51 |
| BCMA×CD3 κλ006 | 56 |
| KLH×CD3 | 76 |

**5. Binding of BCMA×CD3 κλ bispecific antibody to peripheral blood T cells**

Fresh human or cynomolgus monkey peripheral blood was taken and PBMC was isolated by Ficoll-Paque Plu (GE, 17-1440-03). PBMC was adjusted to $5 \times 10^5$ cells/mL with 4% calf serum (Hyclone, SH30626.06), 100 μL/well was added to a 96-well U-plate, centrifuged to remove the supernatant and 100 μL of gradient diluted antibody (initial concentration 1800 nM, 3-fold dilution, 10 gradients) was added to each well and incubated at 4°C for 60 min. 50 μL/well Alexa Fluro647 labeled goat anti-human IgG Fc (1:300 dilution) was added to the secondary antibody, ice bath for 20 min, 50 μL/well PI was added after washing once, incubated for 5 min, and flow cytometer (BD C6) was used to perform detection. The control antibody REGN5458 was synthesized and prepared according to US20200024356.

As shown in FIG. 9 and Table 13, the BCMA×CD3 κλ bispecific antibody recognizes human peripheral blood CD4+ T and CD8+ T cells with an affinity of about 60-97 nM, which is weaker than the binding force of the BCMA antigen arm to the BCMA receptor, so that the bispecific antibody preferentially enriches into tumor cells.

Table 13. Binding of BCMA×CD3 κλ bispecific antibodies to peripheral blood T cells

| $EC_{50}$(nM) | CD4+ T cell | CD8+ T cell |
|---|---|---|
| BCMA×CD3 κλ003 | 87 | 97 |
| BCMA×CD3 κλ004 | 77 | 84 |
| BCMA×CD3 κλ005 | 66 | 60 |
| BCMA×CD3 κλ006 | 91 | 93 |
| KLH×CD3 | 57 | 62 |

**Example 5. BCMA×CD3 κλ bispecific antibody-mediated TDCC**

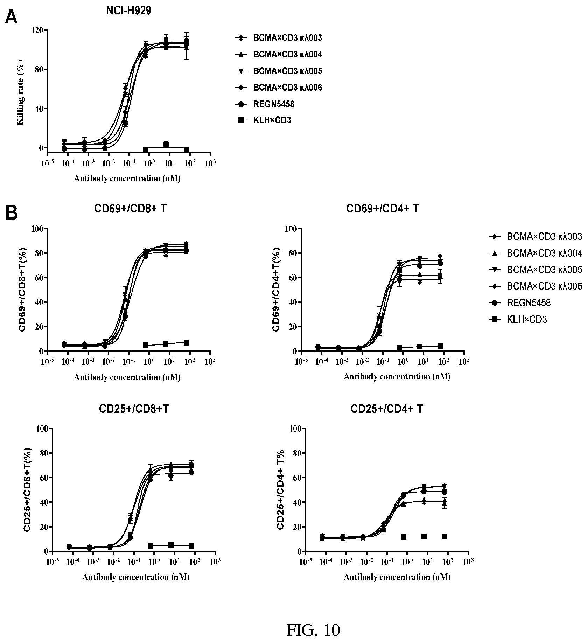

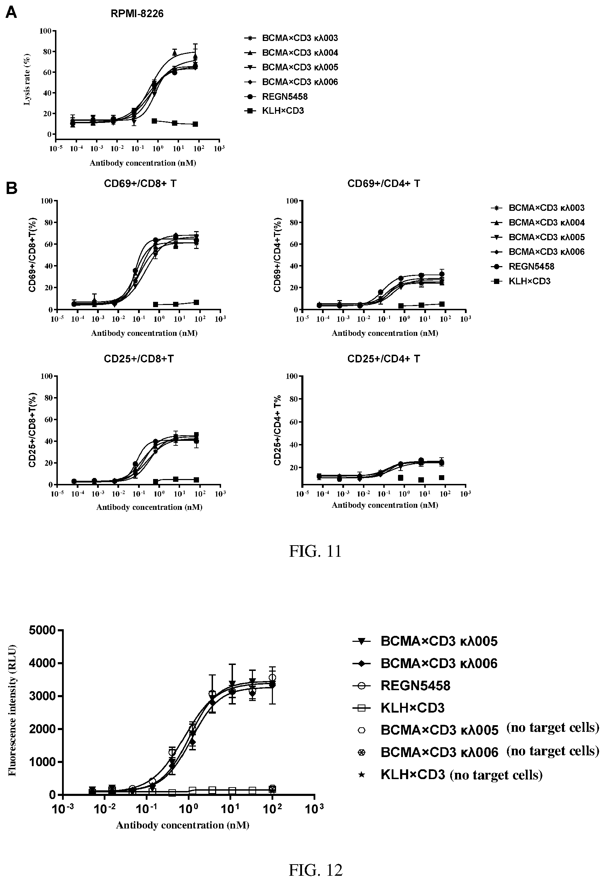

Freshly isolated PBMC was mixed with target cells, NCI-H929 and RPMI-8226 cells, in the logarithmic growth phase, respectively, with effect/target cell = 8: 1, 50 μL of gradient diluted antibody (antibody concentration from 66.7 nM, 10-fold dilution for 7 gradients) was added to each well and cultured at 37°C in 5% $CO_2$ for 24 h. After the end of the culture, 50 μL of the supernatant was transferred to a new black ELISA plate, 50 μL/well of LDH detection substrate was added, the reaction was stopped after 10 min and LDH release was detected; the remaining cells in the wells were washed twice with 4% calf blood, incubated with 100 μg/mL human IgG for 10 min, and T cell were added to activate and detect antibodies (CD25-PE, CD4-APC, CD69-FITC, and CD8-APC) and incubating on ice for 20 min. The supernatant was washed and discarded, 60 μL/well PI was added, incubated on ice for 5 min, and detected by flow cytometer. FIGs. 10A and 10B show the killing of NCI-H929 cells and activation of T cells by the BCMA×CD3 κλ bispecific antibody, respectively. FIGs. 11A and 11B show the killing of RPMI-8226 cells and activation of T cells by the BCMA×CD3 κλ bispecific antibody, respectively. For tumor cells, NCI-H929 and RPMI-8226 with different expression levels of BCMA, the BCMA×CD3 κλ bispecific antibody can mediate effective killing of T cells, and the killing activity is equivalent to that of the control antibody.

**Example 6. Activation of the T cell activation pathway by the BCMA×CD3 κλ bispecific antibody**

The luciferase reporter plasmid pGL4.30[luc2P/NFAT-RE/Hygro] (Promega, E8481) was transfected into Jurkat cells and selected by adding 200 μg/ml hygromycin B to obtain Jurkat-NFAT luc stably transfected reporter cells. Jurkat-NFAT-luc reporter cells in the logarithmic growth phase and target cells RPMI-8226 were taken, centrifuged to discard the supernatant, and resuspended to $2 \times 10^6$ cells/ml. 50 μl/well of the target cells were inoculated into a 96-well plate, the supernatant was discarded by centrifugation at 300g for 5 min, and 50 μl/well of the Jurkat-NFAT-luc reporter cells were inoculated into a 96-well plate, and 50 μl of gradient diluted BCMA×CD3 κλ bispecific antibody or control antibody KLH×CD3 (initial concentration 20 μg/ml, 10-fold dilution for 10 gradients) was added to each well, and cultured at 37°C for 6 h in 5% $CO_2$. After the end of the culture, 100 μL of detection reagent was added to each well according to ONE-Glo Luciferase Assay System instructions, allowed to stand at room temperature for 3 min, and detected on a microplate reader (Biotek Synergy HT). The detection results are shown in FIG. 12. The BCMA×CD3 κλ bispecific antibody can activate the NFAT signaling pathway of T cells when RPMI-8226 tumor cells are used as target cells; in the absence of target cells, the NFAT signaling pathway is not activated.

**Example 7. Non-specific activation of PBMC by BCMA×CD3 κλ bispecific antibody**

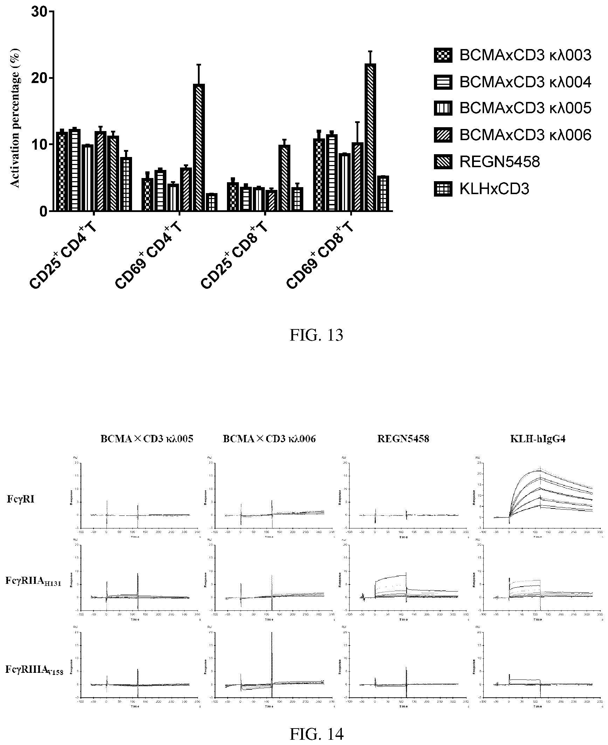

Freshly isolated PBMC was taken, and 50 μL of gradient diluted antibody (antibody concentration from 66.7 nM, 10-fold dilution for 7 gradients) was added to each well and cultured at 37°C in 5% $CO_2$ for 24 h. After the end of the culture, 50 μL of the supernatant was transferred to a new black ELISA plate, 50 μL/well of LDH detection substrate was added, the reaction was stopped after 10 min and LDH release was detected; the remaining cells in the wells were washed twice with 4% calf blood, incubated with 100 μg/mL human IgG for 10 min, and T cell were added to activate and detect antibodies (CD25-PE, CD4-APC, CD69-FITC, and CD8-APC) and incubating on ice for 20 min. The supernatant was washed and discarded, 60 μL/well PI was added, incubated on ice for 5 min, and detected by flow cytometer. The results are shown in FIG. 13, in the absence of target cells, the BCMA×CD3 κλ bispecific antibody had no activation of peripheral blood T cells, comparable to the negative control KLH×CD3.

**Example 8. Binding of BCMA×CD3 κλ bispecific antibodies to Fc receptors**

50 Mg/ml His-Tag antibody was amino-coupled to CM5 chip to capture FcγRI, FcγRIIA$_{H131}$, and FcγRIIIAv$_{158}$ recombinant proteins with His6 tag, respectively, with capture time of 40 s and flow rate of 10 μL/min, after baseline stable, gradient diluted antibody (initial concentration 37.5 μg/mL, 2-fold dilution) flowed through the chip with a flow rate of 30 μL/min, binding time of 120 s, dissociation time of 200 s, and affinity constant was obtained by fitting with Biacore evaluation software. The results are shown in FIG. 14. The BCMA×CD3 κλ bispecific antibody did not bind to activated receptors FcγRI, FcγRIIA$_{H131}$, and FcγRIIIAv$_{158}$; wild-type IgG4 and control antibodies bound to FcγRI, FcγRIIA$_{H131}$ or FcγRIIIAv$_{158}$, respectively.

**Example 9. Blocking of BCMA receptor binding to a ligand by BCMA×CD3 κλ bispecific antibody**

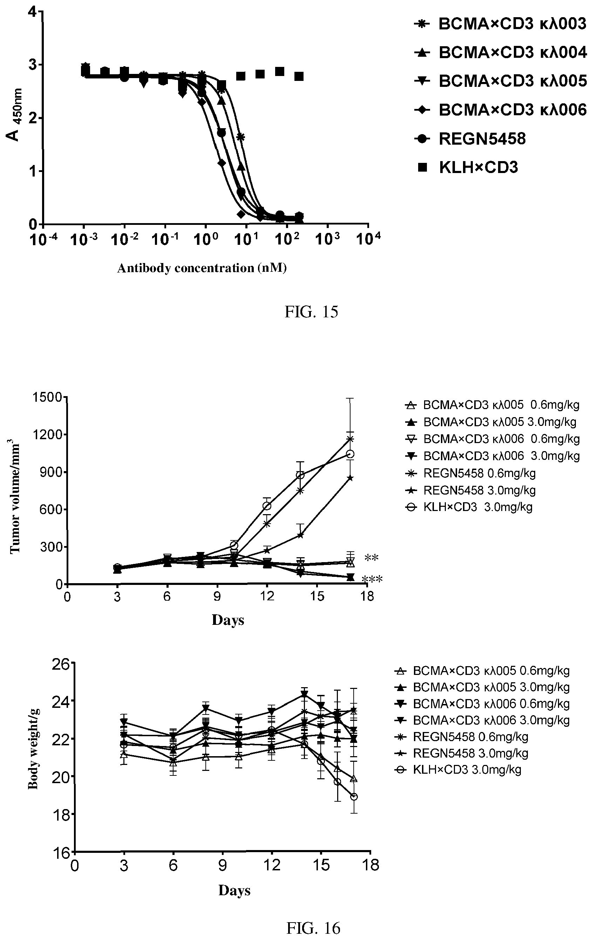

1 μg/mL human BCMA recombinant antigen was prepared with pH 9.6 carbonic acid buffer, added to a maxisorp96-well plate, and coated overnight at 4°C. After blocking with 5% skim milk/PBS for 1 h at room temperature, 50 μL antibody per well (4-fold dilution from 30 μg/mL for a total of 10 gradients) was added and incubated for 30 min at room temperature. 50 μL of biotinylated human APRIL recombinant protein (Novoprotein, CU89) at a concentration of 240 ng/mL was added and incubation was continued for 30 min. After 3 washes with 0.05% PBST (PBS+ 0.05% Tween-20) and PBS respectively, 100 μL of SA-HRP (diluted 1:8000) was added to each well and incubated at room temperature for 45 min. Washing 3 times with 0.05% PBST and PBS respectively, 100 μL TMB color-developing solution was added into each well, after 10 min, 50 μL concentrated sulfuric acid was added into each well to stop the reaction, read OD450 nm by a microplate reader, and perform three-parameter fitting curve using Graphpad software. The results are shown in FIG. 15. The BCMA×CD3 κλ bispecific antibody effectively blocked the binding of BCMA to the receptor APRIL.

**Example 10. Immune reconstitution of subcutaneous NCI-H929 transplanted tumor model in mice**

Six to eight-week-old female B-NGD mice (Bio-Tech Co. Ltd.) were subcutaneously inoculated with $2 \times 10^6$ NCI-H929 cells (mixed with Matrigel at 1:1). When the tumors grew to 60 mm³, they were randomly divided into administration group 3.0 mg/kg, administration group 0.6 mg/kg, administration group 0.12 mg/kg and negative control group KLH×CD3 3 mg/kg. Each mouse received $1 \times 10^7$ PBMC cells via tail vein injection. Three days later, the first dose was administered to the mouse once every 5 days for a total of 2 doses. The tumor volume and body weight of the mice were monitored every 2 days. At the end of the experiment, the mice were killed by cervical dislocation, and the tumors were collected and weighed. The results are shown in FIG. 16. The in vivo efficacy of BCMA×CD3 κλ bispecific antibody was dose-related. The tumor inhibition rates of low-dose, medium-dose, and high-dose were 55%, 95%, and 108% (BCMA×CD3 κλ005) and 46%, 94%, and 108% (BCMA×CD3 κλ006), respectively. The effect was significantly better than that of the control antibody. The tumor-bearing mice tolerated the above doses well, without weight loss and other adverse reactions.

**Claims**

1. A bispecific antibody or antigen-binding portion thereof comprising:

(a) a first antigen-binding portion or an antigen-binding fragment thereof bound to a BCMA antigen on the surface of a target cell, the first antigen-binding portion comprises a first heavy chain and a first light chain, the first antigen-binding portion comprises a first binding domain that binds to the first antigen, wherein the first binding domain comprises a heavy chain CDR selected from the amino acid sequences of SEQ ID NOs: 12, 13, 14, 26, 27, 28, 36, 37, 43, 44, 50, 55, 56, 57, 65, 66, 71, 72, 73 and 147, or any variant thereof, and/or a light chain CDR selected from the amino acid sequences of SEQ ID Nos: 17, 18, 19, 22, 23, 31, 32, 33, 40, 47, 60, 61, 62, 76, 77, 78, 83, 84, 87, 88, 89, 92 and 144 or any variant; and

(b) a second antigen-binding portion or antigen-binding fragment thereof bound to a CD3 antigen on the surface of a T cell, the second antigen-binding portion comprises a second heavy chain and a second light chain, the second antigen-binding portion comprises a second binding domain that binds to a second antigen.

2. The bispecific antibody or antigen-binding portion thereof of claim 1, wherein the first binding domain comprises a heavy chain CDR1 selected from the amino acid sequences of SEQ ID NOs: 12, 26, 55, 65, 71 or any variant thereof, a heavy chain CDR2 selected from the amino acid sequences of SEQ ID NOs: 13, 27, 36, 43, 50, 56, 66, 72, 147 or any variant thereof, a heavy chain CDR3 selected from the amino acid sequences of SEQ ID NOs: 14, 28, 37, 44, 57, 73 or any variant thereof; and/or the first binding domain comprises a light chain CDR1 selected from the amino acid sequences of SEQ ID NOs: 17, 22, 31, 47, 60, 76, 83, 87, 92, 144 or any variant thereof, a light chain CDR2 selected from the amino acid sequences of SEQ ID NOs: 18, 23, 32, 40, 61, 77, 84, 88 or any variant thereof, a light chain CDR3 selected from the amino acid sequences of SEQ ID NOs: 19, 33, 62, 78, 89 or any variant thereof.

3. The bispecific antibody or antigen-binding portion thereof of claim 1 or 2, wherein, the heavy chain CDR1, CDR2, and CDR3 sequences of the first binding domain are selected from: first heavy chain CDR1, CDR2, and CDR3 sequences of the amino acid sequences of SEQ ID NOs: 12, 13 and 14, first heavy chain CDR1, CDR2, and CDR3 sequences of the amino acid sequences of SEQ ID NOs: 26, 27 and 28, first heavy chain CDR1, CDR2, and CDR3 sequences of the amino acid sequences of SEQ ID NOs: 12, 36 and 37, first heavy chain CDR1, CDR2, and CDR3 sequences of the amino acid sequences of SEQ ID NOs: 12, 43 and 44, first heavy chain CDR1, CDR2, and CDR3 sequences of the amino acid sequences of SEQ ID NOs: 12, 50 and 14, first heavy chain CDR1, CDR2, and CDR3 sequences of the amino acid sequences of SEQ ID NOs: 55, 56 and 57, first heavy chain CDR1, CDR2, and CDR3 sequences of the amino acid sequences of SEQ ID NOs: 65, 66 and 14, first heavy chain CDR1, CDR2, and CDR3 sequences of the amino acid sequences of SEQ ID NOs: 71, 72 and 73; first heavy chain CDR1, CDR2, and CDR3 sequences of the amino acid sequences of SEQ ID NOs: 71, 147 and 73; and the light chain CDR1, CDR2, and CDR3 sequences of the first binding domain are selected from: first light chain CDR1, CDR2 and CDR3 sequences of the amino acid sequences of SEQ ID NOs: 17, 18 and 19, first light chain CDR1, CDR2 and CDR3 sequences of the amino acid sequences of SEQ ID NOs: 22, 23 and 19, first light chain CDR1, CDR2 and CDR3 sequences of the amino acid sequences of SEQ ID NOs: 31, 32 and 33, first light chain CDR1, CDR2 and CDR3 sequences of the amino acid sequences of SEQ ID NOs: 17, 40 and 19, first light chain CDR1, CDR2 and CDR3 sequences of the amino acid sequences of SEQ ID NOs: 47, 18 and 19, first light chain CDR1, CDR2 and CDR3 sequences of the amino acid sequences of SEQ ID NOs: 60, 61 and 62, first light chain CDR1, CDR2 and CDR3 sequences of the amino acid sequences of SEQ ID NOs: 76, 77 and 78, first light chain CDR1, CDR2 and CDR3 sequences of the amino acid sequences of SEQ ID NOs: 83, 84 and 19, first light chain CDR1, CDR2, and CDR3 sequences of the amino acid sequences of SEQ ID NOs: 87, 88 and 89, first light chain CDR1, CDR2, and CDR3 sequences of the amino acid sequences of SEQ ID NOs: 92, 23 and 19, first light chain CDR1, CDR2, and CDR3 sequences of the amino acid sequences of SEQ ID NOs: 144, 77 and 78;

preferably, the first binding domain comprises first heavy chain CDR1, CDR2, and CDR3 sequences of the amino acid sequences of SEQ ID NOs: 12, 13 and 14, and first light chain CDR1, CDR2, and CDR3 sequences of the amino acid sequences of SEQ ID NOs: 17, 18 and 19;

preferably, the first binding domain comprises first heavy chain CDR1, CDR2, and CDR3 sequences of the amino acid sequences of SEQ ID NOs: 12, 13 and 14 and first light chain CDR1, CDR2, and CDR3 sequences of the amino acid sequences of SEQ ID NOs: 22, 23 and 19;

preferably, the first binding domain comprises first heavy chain CDR1, CDR2, and CDR3 sequences of the amino acid sequences of SEQ ID NOs: 26, 27, and 28 and first light chain CDR1, CDR2, and CDR3 sequences of the amino acid sequences of SEQ ID NOs: 31, 32, and 33;

preferably, the first binding domain comprises first heavy chain CDR1, CDR2, and CDR3 sequences of the amino acid sequences of SEQ ID NOs: 12, 36 and 37 and first light chain CDR1, CDR2, and CDR3 sequences of the amino acid sequences of SEQ ID NOs: 17, 40 and 19;

preferably, the first binding domain comprises first heavy chain CDR1, CDR2, and CDR3 sequences of the amino acid sequences of SEQ ID NOs: 12, 43 and 44 and first light chain CDR1, CDR2, and CDR3 sequences of the amino acid sequences of SEQ ID NOs: 47, 18 and 19;

preferably, the first binding domain comprises first heavy chain CDR1, CDR2, and CDR3 sequences of the amino acid sequences of SEQ ID NOs: 12, 50 and 14 and first light chain CDR1, CDR2, and CDR3 sequences of the amino acid sequences of SEQ ID NOs: 17, 40 and 19;

preferably, the first binding domain comprises first heavy chain CDR1, CDR2, and CDR3 sequences of the amino acid sequences of SEQ ID NOs: 55, 56 and 57 and first light chain CDR1, CDR2, and CDR3 sequences of the amino acid sequences of SEQ ID NOs: 60, 61 and 62;

preferably, the first binding domain comprises first heavy chain CDR1, CDR2, and CDR3 sequences of the amino acid sequences of SEQ ID NOs: 12, 13 and 14 and first light chain CDR1, CDR2, and CDR3 sequences of the amino acid sequences of SEQ ID NOs: 17, 40 and 19;

preferably, the first binding domain comprises first heavy chain CDR1, CDR2, and CDR3 sequences of the amino acid sequences of SEQ ID NOs: 65, 66 and 14 and first light chain CDR1, CDR2, and CDR3 sequences of the amino acid sequences of SEQ ID NOs: 17, 40 and 19;

preferably, the first binding domain comprises first heavy chain CDR1, CDR2, and CDR3 sequences of the amino acid sequences of SEQ ID NOs: 71, 72, and 73 and first light chain CDR1, CDR2, and CDR3 sequences of the amino acid sequences of SEQ ID NOs: 76, 77, and 78;

preferably, the first binding domain comprises first heavy chain CDR1, CDR2, and CDR3 sequences of the amino acid sequences of SEQ ID NOs: 12, 13 and 14 and first light chain CDR1, CDR2, and CDR3 sequences of the amino acid sequences of SEQ ID NOs: 83, 84 and 19;

preferably, the first binding domain comprises first heavy chain CDR1, CDR2, and CDR3 sequences of the amino acid sequences of SEQ ID NOs: 12, 13 and 14 and first light chain CDR1, CDR2, and CDR3 sequences of the amino acid sequences of SEQ ID NOs: 87, 88 and 89;

preferably, the first binding domain comprises first heavy chain CDR1, CDR2, and CDR3 sequences of the amino acid sequences of SEQ ID NOs: 12, 13 and 14 and first light chain CDR1, CDR2, and CDR3 sequences of the amino acid sequences of SEQ ID NOs: 92, 23 and 19;

preferably, the first binding domain comprises first heavy chain CDR1, CDR2, and CDR3 sequences of the amino acid sequences of SEQ ID NOs: 71, 147, 73 and first light chain CDR1, CDR2, and CDR3 sequences of the amino acid sequences of SEQ ID NOs: 144, 77, 78.

4. The bispecific antibody or antigen-binding portion thereof of any of claims 1 to 3, wherein the first binding domain comprises a first heavy chain variable region selected from the amino acid sequences of SEQ ID NOs: 10, 24, 34, 41, 48, 53, 63, 69, 79, 145, 150, 154, 158, 162, 166 and 170 or any variant thereof, and/or comprises a first light chain variable region selected from the amino acid sequences of SEQ ID NOs: 15, 20, 29, 38, 45, 51, 58, 67, 74, 81, 85, 90, 142, 148, 152, 156, 160, 164 and 168 or any variant thereof;

preferably, the first binding domain comprises a first heavy chain variable region of the amino acid sequence of SEQ ID NO: 10 or any variant thereof, and a first light chain variable region of the amino acid sequence of SEQ ID NO: 15 or any variant thereof; preferably, the first binding domain comprises a first heavy chain variable region of the amino acid sequence of SEQ ID NO: 10 or any variant thereof, and a first light chain variable region of the amino acid sequence of SEQ ID NO: 20 or any variant thereof; preferably, the first binding domain comprises a first heavy chain variable region of the amino acid sequence of SEQ ID NO: 24 or any variant thereof, and a first light chain variable region of the amino acid sequence of SEQ ID NO: 29 or any variant thereof; preferably, the first binding domain comprises a first heavy chain variable region of the amino acid sequence of SEQ ID NO: 34 or any variant thereof, and a first light chain variable region of the amino acid sequence of SEQ ID NO: 38 or any variant thereof; preferably, the first binding domain comprises a first heavy chain variable region of the amino acid sequence of SEQ ID NO: 41 or any variant thereof, and a first light chain variable region of the amino acid sequence of SEQ ID NO: 45 or any variant thereof; preferably, the first binding domain comprises a first heavy chain variable region of the amino acid sequence of SEQ ID NO: 48 or any variant thereof, and a first light chain variable region of the amino acid sequence of SEQ ID NO: 51 or any variant thereof; preferably, the first binding domain comprises a first heavy chain variable region of the amino acid sequence of SEQ ID NO: 53 or any variant thereof, and a first light chain variable region of the amino acid sequence of SEQ ID NO: 58 or any variant thereof; preferably, the first binding domain comprises a first heavy chain variable region of the amino acid sequence of SEQ ID NO: 10 or any variant thereof, and a first light chain variable region of the amino acid sequence of SEQ ID NO: 38 or any variant thereof; preferably, the first binding domain comprises a first heavy chain variable region of the amino acid sequence of SEQ ID NO: 63 or any variant thereof, and a first light chain variable region of the amino acid sequence of SEQ ID NO: 67 or any variant thereof; preferably, the first binding domain comprises a first heavy chain variable region of the amino acid sequence of SEQ ID NO: 69 or any variant thereof, and a first light chain variable region of the amino acid sequence of SEQ ID NO: 74 or any variant thereof; preferably, the first binding domain comprises a first heavy chain variable region of the amino acid sequence of SEQ ID NO: 79 or any variant thereof, and a first light chain variable region of the amino acid sequence of SEQ ID NO: 81 or any variant thereof;

preferably, the first binding domain comprises a first heavy chain variable region of the amino acid sequence of SEQ ID NO: 10 or any variant thereof, and a first light chain variable region of the amino acid sequence of SEQ ID NO: 85 or any variant thereof;

preferably, the first binding domain comprises a first heavy chain variable region of the amino acid sequence of SEQ ID NO: 10 or any variant thereof, and a first light chain variable region of the amino acid sequence of SEQ ID NO: 90 or any variant thereof;

preferably, the first binding domain comprises a first heavy chain variable region of the amino acid sequence of SEQ ID NO: 145 or any variant thereof, and a first light chain variable region of the amino acid sequence of SEQ ID NO: 142 or any variant thereof;

preferably, the first binding domain comprises a first heavy chain variable region of the amino acid sequence of SEQ ID NO: 150 or any variant thereof, and a first light chain variable region of the amino acid sequence of SEQ ID NO: 148 or any variant thereof;

preferably, the first binding domain comprises a first heavy chain variable region of the amino acid sequence of SEQ ID NO: 154 or any variant thereof, and a first light chain variable region of the amino acid sequence of SEQ ID NO: 152 or any variant thereof;

preferably, the first binding domain comprises a first heavy chain variable region of the amino acid sequence of SEQ ID NO: 158 or any variant thereof, and a first light chain variable region of the amino acid sequence of SEQ ID NO: 156 or any variant thereof;

preferably, the first binding domain comprises a first heavy chain variable region of the amino acid sequence of SEQ ID NO: 162 or any variant thereof, and a first light chain variable region of the amino acid sequence of SEQ ID NO: 160 or any variant thereof;

preferably, the first binding domain comprises a first heavy chain variable region of the amino acid sequence of SEQ ID NO: 166 or any variant thereof, and a first light chain variable region of the amino acid sequence of SEQ ID NO: 164 or any variant thereof;

preferably, the first binding domain comprises a first heavy chain variable region of the amino acid sequence of SEQ ID NO: 170 or any variant thereof, and a first light chain variable region of the amino acid sequence of SEQ ID NO: 168 or any variant thereof.

5. The bispecific antibody or antigen-binding portion thereof of any of claims 1 to 4, wherein, the second binding domain comprises a heavy chain CDR selected from the amino acid sequences of SEQ ID NOs: 114, 115, 116, 119, 122, 125, 128, 131, 134, 135 or any variant thereof; and/or a light chain CDR selected from the amino acid sequences of SEQ ID NOs: 95, 96, 97, 102, 103, 108, 111 or any variant thereof;

preferably, the second binding domain comprises a second heavy chain CDR1 selected from the amino acid sequences of SEQ ID NOs: 114, 119, 134 or any variant thereof, a second heavy chain CDR2 selected from the amino acid sequences of SEQ ID NOs: 115, 135 or any variant thereof, a second heavy chain CDR3 selected from the amino acid sequences of SEQ ID NOs: 116, 122, 125, 128, 131 or any variant thereof; and/or a second light chain CDR1 selected from the amino acid sequences of SEQ ID NOs: 95, 102 or any variant thereof, a second light chain CDR2 selected from the amino acid sequences of SEQ ID NOs: 96, 103, 111 or any variant thereof, a second light chain CDR3 selected from the amino acid sequences of SEQ ID NOs: 91, 108 or any variant thereof;

preferably, the heavy chain CDR1, CDR2, and CDR3 sequences of the second binding domain are selected from: second heavy chain CDR1, CDR2, and CDR3 sequences of the amino acid sequences of SEQ ID NOs: 114, 115 and 116, second heavy chain CDR1, CDR2, and CDR3 sequences of the amino acid sequences of SEQ ID NOs: 119, 115 and 116, second heavy chain CDR1, CDR2, and CDR3 sequences of the amino acid sequences of SEQ ID NOs: 119, 115 and 122, second heavy chain CDR1, CDR2, and CDR3 sequences of the amino acid sequences of SEQ ID NOs: 119, 115 and 125, second heavy chain CDR1, CDR2, and CDR3 sequences of the amino acid sequences of SEQ ID NOs: 119, 115 and 128, second heavy chain CDR1, CDR2, and CDR3 sequences of the amino acid sequences of SEQ ID NOs: 119, 115 and 131, second heavy chain CDR1, CDR2, and CDR3 sequences of the amino acid sequences of SEQ ID NOs: 134, 135 and 116; and the light chain CDR1, CDR2, and CDR3 sequences of the second binding domain are selected from: second light chain CDR1, CDR2, and CDR3 sequences of the amino acid sequences of SEQ ID NOs: 95, 96 and 97, second light chain CDR1, CDR2, and CDR3 sequences of the amino acid sequences of SEQ ID NOs: 102, 103 and 97, second light chain CDR1, CDR2, and CDR3 sequences of the amino acid sequences of SEQ ID NOs: 95, 96 and 108, second light chain CDR1, CDR2, and CDR3 sequences of the amino acid sequences of SEQ ID NOs: 95, 111 and 108;

more preferably, the second binding domain comprises second heavy chain CDR1, CDR2, and CDR3 sequences of the amino acid sequences of SEQ ID NOs: 114, 115, 116 and second light chain CDR1, CDR2, and CDR3 sequences of the amino acid sequences of SEQ ID NOs: 95, 96, 97;

more preferably, the second binding domain comprises second heavy chain CDR1, CDR2, and CDR3 sequences of the amino acid sequences of SEQ ID NOs: 114, 115, 116 and second light chain CDR1, CDR2, and CDR3 sequences of the amino acid sequences of SEQ ID NOs: 102, 103, 97;

more preferably, the second binding domain comprises second heavy chain CDR1, CDR2, and CDR3 sequences of the amino acid sequences of SEQ ID NOs: 114, 115, 116 and second light chain CDR1, CDR2, and CDR3 sequences of the amino acid sequences of SEQ ID NOs: 95, 96, and 108;

more preferably, the second binding domain comprises second heavy chain CDR1, CDR2, and CDR3 sequences of the amino acid sequences of SEQ ID NOs: 114, 115, 116 and second light chain CDR1, CDR2, and CDR3 sequences of the amino acid sequences of SEQ ID NOs: 95, 111, and 108;

preferably, the second binding domain comprises a second heavy chain variable region selected from the amino acid sequences of SEQ ID NOs: 112, 117, 120, 123, 126, 129, 132, 136, 138, 140 or any variant thereof, and/or comprises a second light chain variable region selected from the amino acid sequences of SEQ ID NOs: 93, 98, 100, 104, 106, 109 or any variant thereof;

more preferably, the second binding domain comprises a second heavy chain variable region of the amino acid sequence of SEQ ID NO: 136 or any variant thereof, and a second light chain variable region of the amino acid sequence of SEQ ID NO: 93 or any variant thereof;

more preferably, the second binding domain comprises a second heavy chain variable region of the amino acid sequence of SEQ ID NO: 138 or any variant thereof, and a second light chain variable region of the amino acid sequence of SEQ ID NO: 93 or any variant thereof;

more preferably, the second binding domain comprises a second heavy chain variable region of the amino acid sequence of SEQ ID NO: 138 or any variant thereof, and a second light chain variable region of the amino acid sequence of SEQ ID NO: 98 or any variant thereof;

more preferably, the second binding domain comprises a second heavy chain variable region of the amino acid sequence of SEQ ID NO: 138 or any variant thereof, and a second light chain variable region of the amino acid sequence of SEQ ID NO: 100 or any variant thereof;

more preferably, the second binding domain comprises a second heavy chain variable region of the amino acid sequence of SEQ ID NO: 112 or any variant thereof, and a second light chain variable region of the amino acid sequence of SEQ ID NO: 106 or any variant thereof;

more preferably, the second binding domain comprises a second heavy chain variable region of the amino acid sequence of SEQ ID NO: 136 or any variant thereof, and a second light chain variable region of the amino acid sequence of SEQ ID NO: 106 or any variant thereof;

more preferably, the second binding domain comprises a second heavy chain variable region of the amino acid sequence of SEQ ID NO: 138 or any variant thereof, and a second light chain variable region of the amino acid sequence of SEQ ID NO: 106 or any variant thereof.

6. The bispecific antibody or antigen-binding portion thereof of any of claims 1 to 5, wherein, the first light chain of the first antigen-binding portion is a $\kappa$ type light chain, the second light chain of the second antigen-binding portion is a $\lambda$ type light chain,

preferably, the second light chain variable region of the second antigen-binding portion has a $Gln_{40}Glu$ mutation ($V\lambda_{CD3}$:$Gln_{40}Glu$); the second heavy chain variable region of the second antigen-binding portion has a $Gln_{39}Lys$ mutation ($VH_{CD3}$:$Gln_{39}Lys$);

the first light chain variable region of the first antigen-binding portion has a $Gln_{42}Lys$ mutation ($V\kappa_{BCMA}$: $Gln_{42}Lys$);

more preferably, the first heavy chain variable region of the first antigen-binding portion has a $Gln_{39}Glu$ mutation ($VH_{BCMA}$: $Gln_{39}Glu$);

preferably, the Fc portion of the first antigen-binding portion and the second antigen-binding portion of the bispecific antibody adopts a knob-into-hole structure; preferably, a human IgG4 knob-into-hole structure is adopted.

7. The bispecific antibody or antigen-binding portion thereof of any of claims 1 to 6, wherein the first heavy chain comprises a heavy chain selected from SEQ ID NOs: 174, 178 or any variant thereof, the first light chain comprises a light chain selected from SEQ ID NOs: 172, 176 or any variant thereof;

preferably, the first heavy chain comprises a heavy chain selected from SEQ ID NO: 174 or any variant thereof, the first light chain comprises a light chain selected from SEQ ID NO: 172 or any variant thereof;

preferably, the first heavy chain comprises a heavy chain selected from SEQ ID NO: 174 or any variant thereof, the first light chain comprises a light chain selected from SEQ ID NO: 176 or any variant thereof;

preferably, the first heavy chain comprises a heavy chain selected from SEQ ID NO: 178 or any variant thereof, the first light chain comprises a light chain selected from SEQ ID NO: 172 or any variant thereof;
preferably, the first heavy chain comprises a heavy chain selected from SEQ ID NO: 178 or any variant thereof, the first light chain comprises a light chain selected from SEQ ID NO: 176 or any variant thereof.

8. The bispecific antibody or antigen-binding portion thereof of any of claims 1 to 7, wherein the second binding domain comprises a second heavy chain variable region of the amino acid sequence of SEQ ID NO: 138 or any variant thereof, and a second light chain variable region of the amino acid sequence of SEQ ID NO: 106 or any variant thereof;

preferably, the second heavy chain comprises a heavy chain of SEQ ID NO: 182 or any variant thereof, the second light chain comprises a light chain of SEQ ID NO: 180 or any variant thereof;
preferably, the first binding domain of the bispecific antibody comprises a first heavy chain variable region of the amino acid sequence of SEQ ID NO: 145 or any variant thereof, and a first light chain variable region of the amino acid sequence of SEQ ID NO: 142 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof;
preferably, the first binding domain of the bispecific antibody comprises a first heavy chain variable region of the amino acid sequence of SEQ ID NO: 150 or any variant thereof, and a first light chain variable region of the amino acid sequence of SEQ ID NO: 148 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof;
preferably, the first binding domain of the bispecific antibody comprises a first heavy chain variable region of the amino acid sequence of SEQ ID NO: 154 or any variant thereof, and a first light chain variable region of the amino acid sequence of SEQ ID NO: 152 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof;
preferably, the first binding domain of the bispecific antibody comprises a first heavy chain variable region of the amino acid sequence of SEQ ID NO: 158 or any variant thereof, and a first light chain variable region of the amino acid sequence of SEQ ID NO: 156 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof;
preferably, the first binding domain of the bispecific antibody comprises a first heavy chain variable region of the amino acid sequence of SEQ ID NO: 162 or any variant thereof, and a first light chain variable region of the amino acid sequence of SEQ ID NO: 160 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof;
preferably, the first binding domain of the bispecific antibody comprises a first heavy chain variable region of the amino acid sequence of SEQ ID NO: 166 or any variant thereof, and a first light chain variable region of the amino acid sequence of SEQ ID NO: 164 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof;
preferably, the first binding domain of the bispecific antibody comprises a first heavy chain variable region of the amino acid sequence of SEQ ID NO: 170 or any variant thereof, and a first light chain variable region of the amino acid sequence of SEQ ID NO: 168 or any variant thereof; the second binding domain comprises a second heavy chain variable region of the amino acid sequence of SEQ ID NO: 138 or any variant thereof, and a second light chain variable region of the amino acid sequence of SEQ ID NO: 106 or any variant thereof;
more preferably, the first heavy chain of the bispecific antibody comprises a heavy chain of SEQ ID NO: 174 or any variant thereof, the first light chain comprises a light chain of SEQ ID NO: 172 or any variant thereof; the second heavy chain comprises a heavy chain of SEQ ID NO: 182 or any variant thereof, the second light chain comprises a light chain of SEQ ID NO: 180 or any variant thereof;
more preferably, the first heavy chain of the bispecific antibody comprises a heavy chain of SEQ ID NO: 174 or any variant thereof, the first light chain comprises a light chain of SEQ ID NO: 176 or any variant thereof; the second heavy chain comprises a heavy chain of SEQ ID NO: 182 or any variant thereof, the second light chain comprises a light chain of SEQ ID NO: 180 or any variant thereof;
more preferably, the first heavy chain of the bispecific antibody comprises a heavy chain of SEQ ID NO: 178 or any variant thereof, the first light chain comprises a light chain of SEQ ID NO: 172 or any variant thereof; the second heavy chain comprises a heavy chain of SEQ ID NO: 182 or any variant thereof, the second light chain comprises a light chain of SEQ ID NO: 180 or any variant thereof;

more preferably, the first heavy chain of the bispecific antibody comprises a heavy chain of SEQ ID NO: 178 or any variant thereof, the first light chain comprises a light chain of SEQ ID NO: 176 or any variant thereof; the second heavy chain comprises a heavy chain of SEQ ID NO: 182 or any variant thereof, the second light chain comprises a light chain of SEQ ID NO: 180 or any variant thereof.

9. A BCMA-binding antibody or antigen-binding portion thereof, the antibody comprises the first antigen-binding portion of any of claims 1 to 8.

10. A nucleic acid encoding the bispecific antibody or antigen-binding portion thereof of any of claims 1 to 8 or the BCMA-binding antibody or antigen-binding portion thereof of claim 9;

preferably, the nucleic acid encoding the first heavy chain variable region of the first antigen-binding portion is selected from the nucleotide sequences of SEQ ID NOs: 11, 25, 35, 42, 49, 54, 64, 70, 80, 146, 151, 155, 159, 163, 167, 171 or any variant thereof; and/or the nucleic acid encoding the first light chain variable region of the first antigen-binding portion is selected from the nucleotide sequences of SEQ ID NOs: 16, 21, 30, 39, 46, 52, 59, 68, 75, 82, 86, 91, 143, 149, 153, 157, 161, 165, 169 or any variant thereof;

more preferably, the nucleic acid encoding the first heavy chain variable region of the first antigen-binding portion is the nucleotide sequence of SEQ ID NO: 11; and the nucleic acid encoding the first light chain variable region is the nucleotide sequence of SEQ ID NO: 16;

more preferably, the nucleic acid encoding the first heavy chain variable region of the first antigen-binding portion is the nucleotide sequence of SEQ ID NO: 11; and the nucleic acid encoding the first light chain variable region is the nucleotide sequence of SEQ ID NO: 21;

more preferably, the nucleic acid encoding the first heavy chain variable region of the first antigen-binding portion is the nucleotide sequence of SEQ ID NO: 25; and the nucleic acid encoding the first light chain variable region is the nucleotide sequence of SEQ ID NO: 30;

more preferably, the nucleic acid encoding the first heavy chain variable region of the first antigen-binding portion is the nucleotide sequence of SEQ ID NO: 35; and the nucleic acid encoding the first light chain variable region is the nucleotide sequence of SEQ ID NO: 39;

more preferably, the nucleic acid encoding the first heavy chain variable region of the first antigen-binding portion is the nucleotide sequence of SEQ ID NO: 42; and the nucleic acid encoding the first light chain variable region is the nucleotide sequence of SEQ ID NO: 46;

more preferably, the nucleic acid encoding the first heavy chain variable region of the first antigen-binding portion is the nucleotide sequence of SEQ ID NO: 49; and the nucleic acid encoding the first light chain variable region is the nucleotide sequence of SEQ ID NO: 52;

more preferably, the nucleic acid encoding the first heavy chain variable region of the first antigen-binding portion is the nucleotide sequence of SEQ ID NO: 54; and the nucleic acid encoding the first light chain variable region is the nucleotide sequence of SEQ ID NO: 59;

more preferably, the nucleic acid encoding the first heavy chain variable region of the first antigen-binding portion is the nucleotide sequence of SEQ ID NO: 11; and the nucleic acid encoding the first light chain variable region is the nucleotide sequence of SEQ ID NO: 39;

more preferably, the nucleic acid encoding the first heavy chain variable region of the first antigen-binding portion is the nucleotide sequence of SEQ ID NO: 64; and the nucleic acid encoding the first light chain variable region is the nucleotide sequence of SEQ ID NO: 68;

more preferably, the nucleic acid encoding the first heavy chain variable region of the first antigen-binding portion is the nucleotide sequence of SEQ ID NO: 70; and the nucleic acid encoding the first light chain variable region is the nucleotide sequence of SEQ ID NO: 75;

more preferably, the nucleic acid encoding the first heavy chain variable region of the first antigen-binding portion is the nucleotide sequence of SEQ ID NO: 80; and the nucleic acid encoding the first light chain variable region is the nucleotide sequence of SEQ ID NO: 82;

more preferably, the nucleic acid encoding the first heavy chain variable region of the first antigen-binding portion is the nucleotide sequence of SEQ ID NO: 11; and the nucleic acid encoding the first light chain variable region is the nucleotide sequence of SEQ ID NO: 86;

more preferably, the nucleic acid encoding the first heavy chain variable region of the first antigen-binding portion is the nucleotide sequence of SEQ ID NO: 11; and the nucleic acid encoding the first light chain variable region is the nucleotide sequence of SEQ ID NO: 91;

more preferably, the nucleic acid encoding the first heavy chain variable region of the first antigen-binding portion is the nucleotide sequence of SEQ ID NO: 146; and the nucleic acid encoding the first light chain variable region is the nucleotide sequence of SEQ ID NO: 143;

more preferably, the nucleic acid encoding the first heavy chain variable region of the first antigen-binding portion is the nucleotide sequence of SEQ ID NO: 151; and the nucleic acid encoding the first light chain variable region is the nucleotide sequence of SEQ ID NO: 149;

more preferably, the nucleic acid encoding the first heavy chain variable region of the first antigen-binding portion is the nucleotide sequence of SEQ ID NO: 155; and the nucleic acid encoding the first light chain variable region is the nucleotide sequence of SEQ ID NO: 153;

more preferably, the nucleic acid encoding the first heavy chain variable region of the first antigen-binding portion is the nucleotide sequence of SEQ ID NO: 159; and the nucleic acid encoding the first light chain variable region is the nucleotide sequence of SEQ ID NO: 157;

more preferably, the nucleic acid encoding the first heavy chain variable region of the first antigen-binding portion is the nucleotide sequence of SEQ ID NO: 163; and the nucleic acid encoding the first light chain variable region is the nucleotide sequence of SEQ ID NO: 161;

more preferably, the nucleic acid encoding the first heavy chain variable region of the first antigen-binding portion is the nucleotide sequence of SEQ ID NO: 167; and the nucleic acid encoding the first light chain variable region is the nucleotide sequence of SEQ ID NO: 165;

more preferably, the nucleic acid encoding the first heavy chain variable region of the first antigen-binding portion is the nucleotide sequence of SEQ ID NO: 171; and the nucleic acid encoding the first light chain variable region is the nucleotide sequence of SEQ ID NO: 169;

preferably, the nucleic acid encoding the first heavy chain of the first antigen-binding portion is selected from the nucleotide sequences of SEQ ID NOs: 175, 179 or any variant thereof; and/or the nucleic acid encoding the first light chain of the first antigen-binding portion is selected from the nucleotide sequences of SEQ ID NOs: 173, 177 or any variant thereof;

more preferably, the nucleic acid encoding the first heavy chain of the first antigen-binding portion is the nucleotide sequence of SEQ ID NO: 175, and the nucleic acid encoding the first light chain of the first antigen-binding portion is the nucleotide sequence of SEQ ID NO: 173;

more preferably, the nucleic acid encoding the first heavy chain of the first antigen-binding portion is the nucleotide sequence of SEQ ID NO: 175, and the nucleic acid encoding the first light chain of the first antigen-binding portion is the nucleotide sequence of SEQ ID NO: 177;

more preferably, the nucleic acid encoding the first heavy chain of the first antigen-binding portion is the nucleotide sequence of SEQ ID NO: 179, and the nucleic acid encoding the first light chain of the first antigen-binding portion is the nucleotide sequence of SEQ ID NO: 173;

more preferably, the nucleic acid encoding the first heavy chain of the first antigen-binding portion is the nucleotide sequence of SEQ ID NO: 179, and the nucleic acid encoding the first light chain of the first antigen-binding portion is the nucleotide sequence of SEQ ID NO: 177;

preferably, the nucleic acid encoding the second heavy chain variable region of the second antigen-binding portion is selected from the nucleotide sequences of SEQ ID NOs: 113, 118, 121, 124, 127, 130, 133, 137, 139, 141 or any variant thereof; and/or the nucleic acid encoding the second light chain variable region of the second antigen-binding portion is selected from the nucleotide sequences of SEQ ID NOs: 94, 99, 101, 105, 107, 110 or any variant thereof;

more preferably, the nucleic acid encoding the second heavy chain variable region of the second antigen-binding portion is the nucleotide sequence of SEQ ID NO: 137; and the nucleic acid encoding the second light chain variable region is the nucleotide sequence of SEQ ID NO: 94;

more preferably, the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 94;

more preferably, the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 99;

more preferably, the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 101;

more preferably, the nucleic acid encoding the second heavy chain variable region of the second antigen-binding portion is the nucleotide sequence of SEQ ID NO: 113; and the nucleic acid encoding the second light chain variable region is the nucleotide sequence of SEQ ID NO: 107;

more preferably, the nucleic acid encoding the second heavy chain variable region of the second antigen-binding portion is the nucleotide sequence of SEQ ID NO: 137; and the nucleic acid encoding the second light chain variable region is the nucleotide sequence of SEQ ID NO: 107;

more preferably, the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107;

preferably, the nucleic acid encoding the second heavy chain of the second antigen-binding portion is the nucleotide sequence of SEQ ID NO: 183 or any variant thereof; and/or the nucleic acid encoding the second light chain of the second antigen-binding portion is the nucleotide sequence of SEQ ID NO: 181 or any variant thereof;

more preferably, the nucleic acid encoding the second heavy chain of the second antigen-binding portion is the nucleotide sequence of SEQ ID NO: 183, and the nucleic acid encoding the second light chain of the second antigen-binding portion is the nucleotide sequence of SEQ ID NO: 181;

more preferably, the nucleic acid encoding the first heavy chain variable region of the first antigen-binding portion of the bispecific antibody is the nucleotide sequence of SEQ ID NO: 146; and the nucleic acid encoding the first light chain variable region is the nucleotide sequence of SEQ ID NO: 143; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107;

more preferably, the nucleic acid encoding the first heavy chain variable region of the first antigen-binding portion of the bispecific antibody is the nucleotide sequence of SEQ ID NO: 151; and the nucleic acid encoding the first light chain variable region is the nucleotide sequence of SEQ ID NO: 149; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107;

more preferably, the nucleic acid encoding the first heavy chain variable region of the first antigen-binding portion of the bispecific antibody is the nucleotide sequence of SEQ ID NO: 155; and the nucleic acid encoding the first light chain variable region is the nucleotide sequence of SEQ ID NO: 153; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107;

more preferably, the nucleic acid encoding the first heavy chain variable region of the first antigen-binding portion of the bispecific antibody is the nucleotide sequence of SEQ ID NO: 159; and the nucleic acid encoding the first light chain variable region is the nucleotide sequence of SEQ ID NO: 157; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107;

more preferably, the nucleic acid encoding the first heavy chain variable region of the first antigen-binding portion of the bispecific antibody is the nucleotide sequence of SEQ ID NO: 163; and the nucleic acid encoding the first light chain variable region is the nucleotide sequence of SEQ ID NO: 161; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107;

more preferably, the nucleic acid encoding the first heavy chain variable region of the first antigen-binding portion of the bispecific antibody is the nucleotide sequence of SEQ ID NO: 167; and the nucleic acid encoding the first light chain variable region is the nucleotide sequence of SEQ ID NO: 165; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107;

more preferably, the nucleic acid encoding the first heavy chain variable region of the first antigen-binding portion of the bispecific antibody is the nucleotide sequence of SEQ ID NO: 171; and the nucleic acid encoding the first light chain variable region is the nucleotide sequence of SEQ ID NO: 169; the nucleic acid encoding the second heavy chain variable region of the second antigen-binding portion is the nucleotide sequence of SEQ ID NO: 139; and the nucleic acid encoding the second light chain variable region is the nucleotide sequence of SEQ ID NO: 107;

preferably, the nucleic acid encoding the first heavy chain of the first antigen-binding portion of the bispecific antibody is the nucleotide sequence of SEQ ID NO: 175, and the nucleic acid encoding the first light chain of the first antigen-binding portion is the nucleotide sequence of SEQ ID NO: 173, the nucleic acid encoding the second heavy chain of the second antigen-binding portion is the nucleotide sequence of SEQ ID NO: 183, and the nucleic acid encoding the second light chain of the second antigen-binding portion is the nucleotide sequence of SEQ ID NO: 181;

preferably, the nucleic acid encoding the first heavy chain of the first antigen-binding portion of the bispecific antibody is the nucleotide sequence of SEQ ID NO: 175, and the nucleic acid encoding the first light chain of the first antigen-binding portion is the nucleotide sequence of SEQ ID NO: 177, the nucleic acid encoding the second heavy chain of the second antigen-binding portion is the nucleotide sequence of SEQ ID NO: 183, and the nucleic acid encoding the second light chain of the second antigen-binding portion is the nucleotide sequence of SEQ ID NO: 181;

preferably, the nucleic acid encoding the first heavy chain of the first antigen-binding portion of the bispecific antibody is the nucleotide sequence of SEQ ID NO: 179, and the nucleic acid encoding the first light chain of the first antigen-binding portion is the nucleotide sequence of SEQ ID NO: 173, the nucleic acid encoding the second heavy chain of the second antigen-binding portion is the nucleotide sequence of SEQ ID NO: 183, and the nucleic acid encoding the second light chain of the second antigen-binding portion is the nucleotide sequence of SEQ ID NO: 181;

preferably, the nucleic acid encoding the first heavy chain of the first antigen-binding portion of the bispecific antibody is the nucleotide sequence of SEQ ID NO: 179, and the nucleic acid encoding the first light chain of the first antigen-binding portion is the nucleotide sequence of SEQ ID NO: 177, the nucleic acid encoding the second heavy chain of the second antigen-binding portion is the nucleotide sequence of SEQ ID NO: 183, and the nucleic acid encoding the second light chain of the second antigen-binding portion is the nucleotide sequence of SEQ ID NO: 181.

11. A vector comprising the nucleic acid of claim 10.

12. A cell comprising the nucleic acid of claim 9 or the vector of claim 11.

13. A composition comprising the bispecific antibody or antigen-binding portion thereof of any of claims 1 to 8, the BCMA-binding antibody or antigen-binding portion thereof of claim 9, the nucleic acid of claim 10, the vector of claim 11 and/or the cell of claim 12.

14. An antibody-drug conjugate comprising the bispecific antibody or antigen-binding portion thereof of any of claims 1 to 8 or the BCMA-binding antibody or antigen-binding portion thereof of claim 9 covalently attached to a therapeutic moiety;

preferably, the therapeutic moiety is selected from a cytotoxic moiety, a chemotherapeutic agent, a cytokine, an immunosuppressant, an immunostimulant, a lytic peptide, or a radioisotope;

preferably, the cytotoxic moiety is selected from paclitaxel; cytochalasin B; gramicidin D; ethidium bromide; emetine; mitomycin; etoposide; teniposide; vincristine; vinblastine; colchicine; doxorubicin; daunorubicin; dihydroxy anthracenedione; tubulin inhibitors such as maytansine or analogs or derivatives thereof; antimitotic agents such as monomethyl auristatin E or F or analogues or derivatives thereof; dolastatin 10 or 15 or an analogue thereof; irinotecan or an analog thereof; mitoxantrone; mithramycin; actinomycin D; 1-dehydrotestosterone; glucocorticoids; procaine; tetracaine; lidocaine; propranolol; puromycin; calicheamicin or an analogue or derivative thereof; antimetabolites such as methotrexate, 6 mercaptopurine, 6 thioguanine, cytarabine, fludarabine, 5 fluorouracil, decadiazine, hydroxyurea, asparaginase, gemcitabine or cladribine; alkylating agents such as mechlorethamine, thiopurine, chlorambucil, melphalan, carmustine (BSNU), lomustine (CCNU), cyclophosphamide, busulfan, dibromomannitol, streptozotocin, dacarbazine (DTIC), procarbazine, mitomycin C; platinum derivatives such as cisplatin or carboplatin; duocarmycin A, duocarmycin SA, rachelmycin (CC-1065) or analogs or derivatives thereof; antibiotics such as actinomycin, bleomycin, daunorubicin, doxorubicin, idarubicin, mithramycin, mitomycin, mitoxantrone, plicomycin, anthramycin (AMC); pyrrolo [2,1-c] [1,4]-benzodiazepine (PDB); diphtheria toxin and related molecules such as diphtheria A chain and active fragments thereof and hybrid molecules, ricin toxin such as ricin A or deglycosylated ricin A chain toxin, cholera toxin, Shiga-like toxin such as SLT I, SLT II, SLT IIV, LT toxin, C3 toxin, Shiga toxin, pertussis toxin, tetanus toxin, soybean Bowman-Birk protease inhibitor, pseudomonas exotoxin, azlocillin, saporin, modeccin, gelsolin, abrin A chain, modeccin A chain, $\alpha$-sarcin, aleurites fordii proteins, dianthin proteins, Phytolacca americana proteins such as PAPI, PAPII, and PAP-S, momordica charantia inhibitor, curcin, crotin, sapaonaria officinalis inhibitor, gelonin, mitomycin, restrictocin, phenomycin, and enomycin toxins; ribonuclease (RNase); DNase I, staphylococcal endotoxin A; pokeweed antiviral protein; diphtheria toxin and pseudomonas endotoxin;

preferably, the cytokine is selected from IL-2, IL-4, IL-6, IL-7, IL-10, IL-12, IL-13, IL-15, IL-18, IL-23, IL-24, IL-27, IL-28a, IL-28b, IL-29, KGF, IFN$\alpha$, IFN$\beta$, IFN$\gamma$, GM-CSF, CD40L, Flt3 ligand, stem cell factor, ancestim, and TNF$\alpha$;

preferably, the radioisotope is selected from $^{3}$H, $^{14}$C, $^{15}$N, $^{35}$S, $^{67}$Cu, $^{90}$Y, $^{99}$Tc, $^{125}$I, $^{131}$I, $^{186}$Re, $^{188}$Re, $^{211}$At, $^{212}$Bi, $^{212}$Pb, $^{213}$Bi, $^{225}$Ac, and $^{227}$Th.

**15.** A kit comprising the specific antibody or antigen-binding portion thereof of any of claims 1 to 8, the BCMA-binding antibody or antigen-binding portion thereof of claim 9, the nucleic acid molecule of claim 10, the vector of claim 11, the cell of claim 12, the composition of claim 13 and/or the antibody-drug conjugate of claim 14.

**16.** Use of the specific antibody or antigen-binding portion thereof of any of claims 1 to 8, the BCMA-binding antibody or antigen-binding portion thereof of claim 9, the nucleic acid molecule of claim 10, the vector of claim 11, the cell of claim 12, the composition of claim 13 and/or the antibody-drug conjugate of claim 14 in the manufacture of a drug or kit for the diagnosis, treatment or prevention of a disease associated with BCMA; preferably, the disease associated with BCMA comprises B cell disease;

preferably, the disease is cancer; more preferably, the cancer is a B-cell related cancer selected from the group consisting of multiple myeloma, malignant plasmacytoma, hodgkin lymphoma, Nodular lymphocyte predominant Hodgkin lymphoma, kahler's disease and myeloid leukemia, plasma cell leukemia, plasmacytoma, B-cell prolymphocytic leukemia, hairy cell leukemia, B-cell non-Hodgkin lymphoma (NHL), acute myelogenous leukemia (AML), chronic lymphocytic leukemia (CLL), acute lymphocytic leukemia (ALL), chronic myelogenous leukemia (CML), follicular lymphoma, burkitt lymphoma, marginal zone lymphoma, mantle cell lymphoma, large cell lymphoma, precursor B-lymphocyte lymphoma, myeloid leukemia, waldenstrom's macroglobulinaemia, diffuse large B cell lymphoma, follicular lymphoma, marginal zone lymphoma, mucosa-associated lymphoid tissue lymphoma, small cell lymphocytic lymphoma, mantle cell lymphoma, burkitt lymphoma, primary mediastinal (thymic) large B cell lymphoma, lymphoplasmacytic lymphoma, waldenstrom' s macroglobulinaemia, lymph node marginal zone B cell lymphoma, splenic marginal zone lymphoma, intravascular large B-cell lymphoma, primary effusion lymphoma, lymphomatoid granulomatosis, large B-cell lymphoma rich in T cells/histiocytes, primary central nervous system lymphoma, primary cutaneous diffuse large B-cell lymphoma (leg type), EBV-positive diffuse large B-cell lymphoma of the elderly, diffuse large B-cell lymphoma, intravascular large B-cell lymphoma associated with inflammation, ALK-positive large B-cell lymphoma, plasmablastic lymphoma, large B-cell lymphoma arising in HHV8-associated multicenter Castleman's disease, unclassified B-cell lymphoma with intermediate features between diffuse large B-cell lymphoma and Burkitt' s lymphoma, unclassified B-cell lymphoma with intermediate features between diffuse large B-cell lymphoma and classic Hodgkin lymphoma, and other B-cell-related lymphomas;
more preferably, the B cell disease is a B cell disorder; preferably, the plasma cell disorder is selected from multiple myeloma, plasmacytoma, plasma cell leukemia, macroglobulinaemia, amyloidosis, waldenstrom's macroglobulinaemia, solitary plasmacytoma of the bone, extramedullary plasmacytoma, osteosclerotic myeloma, heavy chain disease, monoclonal gammopathy of indetermined significance, and multiple myeloma of stasis type;
the disease is an autoimmune disorder, such as systemic lupus erythematosus or rheumatoid arthritis.

FIG. 1

FIG. 2

FIG. 3

FIG. 4

FIG. 5

FIG. 6

## NCI-H929

## RPMI 8226

FIG. 7

FIG. 8

FIG. 9

FIG. 10

FIG. 11

FIG. 12

FIG. 13

FIG. 14

* BCMA×CD3 κλ003
* BCMA×CD3 κλ004
* BCMA×CD3 κλ005
* BCMA×CD3 κλ006
* REGN5458
* KLH×CD3

FIG. 15

* BCMA×CD3 κλ005  0.6mg/kg
* BCMA×CD3 κλ005  3.0mg/kg
* BCMA×CD3 κλ006  0.6mg/kg
* BCMA×CD3 κλ006  3.0mg/kg
* REGN5458 0.6mg/kg
* REGN5458 3.0mg/kg
* KLH×CD3  3.0mg/kg

* BCMA×CD3 κλ005 0.6mg/kg
* BCMA×CD3 κλ005 3.0mg/kg
* BCMA×CD3 κλ006 0.6mg/kg
* BCMA×CD3 κλ006 3.0mg/kg
* REGN5458 0.6mg/kg
* REGN5458 3.0mg/kg
* KLH×CD3 3.0mg/kg

FIG. 16

## INTERNATIONAL SEARCH REPORT

| International application No. |
|---|
| **PCT/CN2021/135121** |

**A. CLASSIFICATION OF SUBJECT MATTER**

C07K 16/28(2006.01)i; A61K 39/395(2006.01)i; A61P 35/02(2006.01)i; A61P 37/00(2006.01)i

According to International Patent Classification (IPC) or to both national classification and IPC

**B. FIELDS SEARCHED**

Minimum documentation searched (classification system followed by classification symbols)

C07K16;A61K39

Documentation searched other than minimum documentation to the extent that such documents are included in the fields searched

Electronic data base consulted during the international search (name of data base and, where practicable, search terms used)

CNTXT; WPABSC; OETXT; ENTXT; DWPI; ENTXTC; CNKI; PubMed; ISI web of knowledge; 中国专利生物序列检索系统; Chinese Patent Biological Sequence Retrieval System; Genbank; EMBL-EBI; STN: BCMA, CD3, 双抗, 双特异性抗体, bispecific antibody, SEQ ID NO: 10, 12-15, 17-19, 22, 23, 26-28, 31-33, 36-37, 40, 43-44, 47, 50, 56, 57, 60-62, 65-66, 71-73, 76-78, 83-84, 87-89, 92-93, 95-97, 102-103, 106, 108, 111-112, 114-116, 119, 122, 125, 128, 131, 134, 135, 136, 138, 144, 147

**C. DOCUMENTS CONSIDERED TO BE RELEVANT**

| Category* | Citation of document, with indication, where appropriate, of the relevant passages | Relevant to claim No. |
|---|---|---|
| X | CN 104114578 A (AMGEN RESEARCH (MUNICH) GMBH et al.) 22 October 2014 (2014-10-22) claims 1-6 and 15-24, and description, paragraphs 739-741 | 1-2, 4-16 |
| Y | CN 104114578 A (AMGEN RESEARCH (MUNICH) GMBH et al.) 22 October 2014 (2014-10-22) claims 1-6 and 15-24, and description, paragraphs 739-741 | 5-8, 10-16 |
| X | WO 2020018820 A1 (REGENERON PHARMACEUTICALS INC) 23 January 2020 (2020-01-23) claims 1-56, and description, embodiments 1-2, and table 1, SEQ ID NO: 10 and 16 | 1-2, 4, 6-7, 9-16 |
| Y | WO 2020018820 A1 (REGENERON PHARMACEUTICALS INC) 23 January 2020 (2020-01-23) claims 1-56, and description, embodiments 1-2, and table 1, SEQ ID NO: 10 and 16 | 5-8, 10-16 |
| Y | CN 103703024 A (MACROGENICS INC.) 02 April 2014 (2014-04-02) claim 1 and sequence table | 5-8, 10-16 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 January 2022** | **07 February 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/135121**

**C.    DOCUMENTS CONSIDERED TO BE RELEVANT**

| Category* | Citation of document, with indication, where appropriate, of the relevant passages | Relevant to claim No. |
|---|---|---|
| Y | WO 2007042261 A2 (MICROMET AG) 19 April 2007 (2007-04-19) description, sequence table, SEQ ID NO: 110-118 | 5-8, 10-16 |
| A | 解志刚等 (XIE, Zhigang et al.). "基因工程双特异性抗体构建模式研究进展 (Construction Formats of Engineered Bispecific Antibodies)" 军事医学科学院院刊 *(Bulletin of the Academy of Military Medical Sciences),* Vol. 28, No. 4, 31 August 2004 (2004-08-31), ISSN: 1674-9960, pp. 375-378 | 1-16 |

Form PCT/ISA/210 (second sheet) (January 2015)

## INTERNATIONAL SEARCH REPORT

International application No.

**PCT/CN2021/135121**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

## INTERNATIONAL SEARCH REPORT

| International application No. |
|---|
| **PCT/CN2021/135121** |

**Box No. III     Observations where unity of invention is lacking (Continuation of item 3 of first sheet)**

This International Searching Authority found multiple inventions in this international application, as follows:

[1]    Claims 1-16 relate o a large amount of BCMAxCD3 bispecific antibodies or antigen-binding portions of different amino acid sequences, a correspondingly contained BCMA antibody, and encoding nucleic acids, vectors, cells, compositions, antibody-drug conjugates, kits of bispecific antibodies and uses thereof. The same or corresponding technical feature between the described parallel technical solutions is a bispecific antibody that specifically binds BCMA and CD3, and the same or corresponding technical feature is disclosed in the prior art. Therefore, the described same or corresponding technical feature makes no contribution over the prior art. Therefore, the described parallel technical solutions are not technical related, do not belong to a single general inventive concept, and thus do not comply with the requirement of unity of invention as defined in PCT Rule 13.1.

1.  ☐   As all required additional search fees were timely paid by the applicant, this international search report covers all searchable claims.

2.  ☑   As all searchable claims could be searched without effort justifying additional fees, this Authority did not invite payment of additional fees.

3.  ☐   As only some of the required additional search fees were timely paid by the applicant, this international search report covers only those claims for which fees were paid, specifically claims Nos.:

4.  ☐   No required additional search fees were timely paid by the applicant. Consequently, this international search report is restricted to the invention first mentioned in the claims; it is covered by claims Nos.:

**Remark on Protest**   ☐   The additional search fees were accompanied by the applicant's protest and, where applicable, the payment of a protest fee.

☐   The additional search fees were accompanied by the applicant's protest but the applicable protest fee was not paid within the time limit specified in the invitation.

☐   No protest accompanied the payment of additional search fees.

Form PCT/ISA/210 (continuation of first sheet) (January 2015)

**INTERNATIONAL SEARCH REPORT**
Information on patent family members

International application No.

**PCT/CN2021/135121**

| Patent document cited in search report | | | Publication date (day/month/year) | Patent family member(s) | | | Publication date (day/month/year) |
|---|---|---|---|---|---|---|---|
| CN | 104114578 | A | 22 October 2014 | PT | 2780375 | T | 12 November 2019 |
| | | | | MX | 2014005851 | A | 19 January 2015 |
| | | | | US | 2013156769 | A1 | 20 June 2013 |
| | | | | US | 9340621 | B2 | 17 May 2016 |
| | | | | EA | 201490931 | A1 | 29 August 2014 |
| | | | | EA | 028162 | B1 | 31 October 2017 |
| | | | | BR | 112014010940 | A2 | 16 May 2017 |
| | | | | ES | 2749451 | T3 | 20 March 2020 |
| | | | | JP | 2020202838 | A | 24 December 2020 |
| | | | | LT | 2780375 | T | 11 November 2019 |
| | | | | HR | P20191697 | T1 | 13 December 2019 |
| | | | | JP | 2017195889 | A | 02 November 2017 |
| | | | | UA | 116766 | C2 | 10 May 2018 |
| | | | | JP | 2018050627 | A | 05 April 2018 |
| | | | | JP | 6738314 | B2 | 12 August 2020 |
| | | | | JP | 2014534242 | A | 18 December 2014 |
| | | | | JP | 6231007 | B2 | 15 November 2017 |
| | | | | EC | SP14004893 | A | 30 June 2015 |
| | | | | AP | 201407529 | A0 | 31 March 2014 |
| | | | | AP | 2014007529 | A0 | 31 March 2014 |
| | | | | JP | 2015504306 | A | 12 February 2015 |
| | | | | JP | 6378087 | B2 | 22 August 2018 |
| | | | | SG | 11201400671 Y | A | 28 April 2014 |
| | | | | RS | 59373 | B1 | 29 November 2019 |
| | | | | IL | 260189 | D0 | 31 July 2018 |
| | | | | CN | 104169300 | A | 26 November 2014 |
| | | | | MA | 35449 | B1 | 01 September 2014 |
| | | | | CN | 109485729 | A | 19 March 2019 |
| | | | | IL | 232636 | D0 | 30 June 2014 |
| | | | | ES | 2751996 | T3 | 02 April 2020 |
| | | | | SG | 11201401729 P | A | 26 September 2014 |
| | | | | WO | 2013072406 | A1 | 23 May 2013 |
| | | | | AR | 088883 | A1 | 16 July 2014 |
| | | | | CA | 2850591 | A1 | 23 May 2013 |
| | | | | AU | 2012327203 | A1 | 30 May 2013 |
| | | | | TN | 2014000097 | A1 | 01 July 2015 |
| | | | | BR | 112014010630 | A2 | 25 April 2017 |
| | | | | SG | 10201704483 R | A | 28 July 2017 |
| | | | | UY | 34453 | A | 28 June 2013 |
| | | | | SG | 10201606484 S | A | 28 October 2016 |
| | | | | MX | 2014005852 | A | 22 September 2014 |
| | | | | MX | 349396 | B | 26 July 2017 |
| | | | | NZ | 622087 | A | 24 June 2016 |
| | | | | PH | 12014501091 | A1 | 28 July 2014 |
| | | | | EA | 201490932 | A1 | 30 September 2014 |
| | | | | PL | 2780375 | T3 | 31 January 2020 |
| | | | | ZA | 201401615 | B | 30 August 2017 |
| | | | | PE | 20141564 | A1 | 29 November 2014 |
| | | | | DK | 2780375 | T3 | 18 November 2019 |
| | | | | KR | 20140105757 | | 02 September 2014 |

Form PCT/ISA/210 (patent family annex) (January 2015)

**INTERNATIONAL SEARCH REPORT**
Information on patent family members

International application No.

**PCT/CN2021/135121**

| Patent document cited in search report | | | Publication date (day/month/year) | Patent family member(s) | | | Publication date (day/month/year) |
|---|---|---|---|---|---|---|---|
| | | | | TW | I679212 | B | 11 December 2019 |
| | | | | AU | 2012327200 | B | 18 February 2016 |
| | | | | IL | 232603 | A | 31 July 2018 |
| | | | | GE | 201806928 | B | 26 November 2018 |
| | | | | KR | 20200003934 | A | 10 January 2020 |
| | | | | VN | 39296 | A | 25 September 2014 |
| | | | | AU | 2012327200 | B | 21 January 2016 |
| | | | | CN | 104114578 | B | 16 October 2018 |
| | | | | US | 2013156770 | A1 | 20 June 2013 |
| | | | | TW | 201326214 | A | 01 July 2013 |
| | | | | US | 2015023967 | A1 | 22 January 2015 |
| | | | | HK | 1198040 | A1 | 29 May 2020 |
| | | | | KR | 102229469 | B | 18 March 2021 |
| | | | | VN | 10027129 | B | 25 January 2021 |
| | | | | ID | 201503394 | A | 07 August 2015 |
| | | | | ID | 201603353 | A | 13 May 2016 |
| | | | | ZA | 201401615 | A | 30 August 2017 |
| | | | | KR | 20210032012 | A | 23 March 2021 |
| | | | | PH | 12014501076 | A1 | 23 June 2014 |
| | | | | IN | 201402860 | P1 | 31 August 2016 |
| | | | | EP | 3611193 | A1 | 19 February 2020 |
| | | | | AU | 2012327200 | A1 | 30 May 2013 |
| | | | | MX | 2014005852 | A | 30 September 2014 |
| | | | | SG | 10201606484 | B | 12 June 2017 |
| | | | | KR | 102062231 | B | 03 January 2020 |
| | | | | US | 10766969 | B2 | 08 September 2020 |
| | | | | CA | 2849196 | C | 20 April 2021 |
| | | | | CA | 2849196 | A1 | 23 May 2013 |
| | | | | EP | 2780375 | A1 | 24 September 2014 |
| | | | | HK | 1198040 | A0 | 06 March 2015 |
| | | | | US | 9150664 | B2 | 06 October 2015 |
| | | | | IL | 232603 | A1 | 30 June 2014 |
| | | | | SG | 10201704483 | A1 | 28 July 2017 |
| | | | | EP | 2780375 | B1 | 11 September 2019 |
| WO | 2020018820 | A1 | 23 January 2020 | CN | 112423785 | A | 26 February 2021 |
| | | | | CA | 3107126 | A1 | 23 January 2020 |
| | | | | CL | 2021000131 | A1 | 02 July 2021 |
| | | | | BR | 112021000186 | A2 | 10 August 2021 |
| | | | | IL | 279974 | D0 | 01 March 2021 |
| | | | | AU | 2019307928 | A1 | 11 February 2021 |
| | | | | SG | 11202100252 S | A | 25 February 2021 |
| | | | | US | 2020024356 | A1 | 23 January 2020 |
| | | | | MA | 53168 | A | 26 May 2021 |
| | | | | CO | 2021000188 | A2 | 18 January 2021 |
| | | | | EP | 3823664 | A1 | 26 May 2021 |
| | | | | JP | 2021531005 | A | 18 November 2021 |
| | | | | KR | 20210034032 | A | 29 March 2021 |
| | | | | TW | 202019966 | A | 01 June 2020 |
| | | | | PH | 12021550031 | A1 | 20 September 2021 |
| | | | | IN | 202147006015 | | 19 February 2021 |

Form PCT/ISA/210 (patent family annex) (January 2015)

**INTERNATIONAL SEARCH REPORT**
Information on patent family members

International application No.

**PCT/CN2021/135121**

| Patent document cited in search report | | | Publication date (day/month/year) | Patent family member(s) | | | Publication date (day/month/year) |
|---|---|---|---|---|---|---|---|
| CN | 103703024 | A | 02 April 2014 | UA | 117072 | C2 | 11 June 2018 |
| | | | | BR | 112013029893 | A2 | 30 May 2017 |
| | | | | HU | E044209 | T2 | 28 October 2019 |
| | | | | ME | 03440 | B | 20 January 2020 |
| | | | | UA | 115122 | C2 | 25 September 2017 |
| | | | | JP | 2017206505 | A | 24 November 2017 |
| | | | | JP | 6496349 | B2 | 03 April 2019 |
| | | | | PT | 2714733 | T | 23 May 2019 |
| | | | | RS | 58765 | B1 | 28 June 2019 |
| | | | | ES | 2732213 | T3 | 21 November 2019 |
| | | | | JP | 2019142866 | A | 29 August 2019 |
| | | | | US | 2014099318 | A1 | 10 April 2014 |
| | | | | US | 9587021 | B2 | 07 March 2017 |
| | | | | UA | 115402 | C2 | 25 October 2017 |
| | | | | EP | 2714733 | A2 | 09 April 2014 |
| | | | | EP | 2714733 | A4 | 14 October 2015 |
| | | | | EP | 2714733 | B1 | 23 January 2019 |
| | | | | US | 2017137519 | A1 | 18 May 2017 |
| | | | | US | 10150812 | B2 | 11 December 2018 |
| | | | | JP | 2014517844 | A | 24 July 2014 |
| | | | | JP | 6141831 | B2 | 07 June 2017 |
| | | | | US | 2019040135 | A1 | 07 February 2019 |
| | | | | US | 11111299 | B2 | 07 September 2021 |
| | | | | IL | 259187 | D0 | 31 July 2018 |
| | | | | IL | 259187 | A | 28 February 2019 |
| | | | | CL | 2016001254 | A1 | 02 December 2016 |
| | | | | CN | 107827985 | A | 23 March 2018 |
| | | | | CN | 107722124 | A | 23 February 2018 |
| | | | | CL | 2013003329 | A1 | 04 July 2014 |
| | | | | CA | 2836857 | A1 | 29 November 2012 |
| | | | | CA | 2836857 | C | 03 December 2019 |
| | | | | NZ | 618021 | A | 27 February 2015 |
| | | | | AU | 2019200159 | A1 | 31 January 2019 |
| | | | | AU | 2019200159 | B2 | 12 March 2020 |
| | | | | SG | 10201509588 T | A | 30 December 2015 |
| | | | | DK | 2714733 | T3 | 06 May 2019 |
| | | | | ZA | 201308602 | B | 30 July 2014 |
| | | | | SG | 195072 | A1 | 30 December 2013 |
| | | | | AU | 2017204545 | A1 | 20 July 2017 |
| | | | | AU | 2017204545 | B2 | 15 November 2018 |
| | | | | EP | 3492494 | A1 | 05 June 2019 |
| | | | | SG | 10201703425 R | A | 30 May 2017 |
| | | | | KR | 20190105112 | A | 11 September 2019 |
| | | | | KR | 20140033107 | A | 17 March 2014 |
| | | | | KR | 102060389 | B1 | 31 December 2019 |
| | | | | SI | 2714733 | T1 | 28 June 2019 |
| | | | | WO | 2012162067 | A2 | 29 November 2012 |
| | | | | WO | 2012162067 | A3 | 31 January 2013 |
| | | | | EC | SP13013101 | A | 31 January 2014 |
| | | | | EA | 033677 | B1 | 15 November 2019 |

Form PCT/ISA/210 (patent family annex) (January 2015)

**INTERNATIONAL SEARCH REPORT**
Information on patent family members

International application No.

**PCT/CN2021/135121**

| Patent document cited in search report | | | Publication date (day/month/year) | Patent family member(s) | | | Publication date (day/month/year) |
|---|---|---|---|---|---|---|---|
| | | | | IL | 264293 | A | 30 September 2020 |
| | | | | IN | 377006 | B | 17 September 2021 |
| | | | | AU | 2012259161 | A1 | 12 December 2013 |
| | | | | MY | 173899 | A2 | 06 February 2012 |
| | | | | MX | 2013013584 | A | 30 September 2014 |
| | | | | IN | 201310078 | P1 | 24 June 2016 |
| | | | | SG | 195072 | B | 30 May 2017 |
| | | | | HK | 1190725 | A1 | 13 March 2020 |
| | | | | HK | 40009624 | A0 | 26 June 2020 |
| | | | | NZ | 703939 | A | 29 January 2016 |
| | | | | MX | 369220 | B | 31 October 2019 |
| | | | | CN | 103703024 | A | 02 April 2014 |
| | | | | BR | 122016016837 | A2 | 27 August 2019 |
| | | | | CN | 103703024 | B | 14 November 2017 |
| | | | | HK | 1190725 | A0 | 11 July 2014 |
| | | | | IL | 229575 | A | 31 May 2018 |
| | | | | AU | 2012259161 | B | 06 April 2017 |
| WO | 2007042261 | A2 | 19 April 2007 | EP | 1940881 | A2 | 09 July 2008 |
| | | | | EP | 1940881 | B1 | 30 November 2016 |
| | | | | CA | 2625440 | A1 | 19 April 2007 |
| | | | | US | 2009252683 | A1 | 08 October 2009 |
| | | | | US | 8236308 | B2 | 07 August 2012 |
| | | | | EP | 3178850 | A1 | 14 June 2017 |
| | | | | EP | 3178850 | B1 | 13 January 2021 |
| | | | | DK | 1940881 | T3 | 20 February 2017 |
| | | | | EP | 3770174 | A1 | 27 January 2021 |
| | | | | ES | 2856451 | T3 | 27 September 2021 |
| | | | | WO | 2007042261 | A3 | 21 December 2007 |
| | | | | WO | 2007042261 | A9 | 07 May 2020 |
| | | | | AU | 2006301492 | A1 | 19 April 2007 |
| | | | | AU | 2006301492 | B2 | 09 June 2011 |
| | | | | JP | 2009511521 | A | 19 March 2009 |
| | | | | JP | 5686953 | B2 | 18 March 2015 |
| | | | | US | 2013129729 | A1 | 23 May 2013 |
| | | | | US | 2019070290 | A1 | 07 March 2019 |
| | | | | ES | 2616316 | T3 | 12 June 2017 |
| | | | | SG | 141561 | A1 | 28 May 2008 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7229619 B
- US 5635483 A
- US 5780588 A
- US 6214345 B
- WO 02088172 A
- WO 2004010957 A
- WO 2005081711 A
- WO 2005084390 A
- WO 2006132670 A
- WO 03026577 A
- WO 200700860 A
- WO 207011968 A
- WO 205082023 A
- WO 2012059882 A
- US 201213678247 A

### Non-patent literature cited in the description

- **ARMOUR KL et al.** Recombinant human IgG molecules lacking Fc gamma receptor I binding and monocyte triggering activities. *Eur J Immunol.,* August 1999, vol. 29 (8), 2613-24
- **R. et al.** Modification of the Fc region of a primatized IgG antibody to human CD4 retains its ability to modulate CD4 receptors but does not deplete CD4(+) T cells in chimpanzees. *Clin Immunol.,* February 2001, vol. 98 (2), 164-74
- **IDUSOGIE EE et al.** Mapping of the C1q Binding Site on Rituxan, a Chimeric Antibody with a Human IgG1 Fc. *J Immunol,* 15 April 2000, vol. 164 (8), 4178-4184
- **CARRILLO, H ; LIPMAN, D.** *SIAM J Applied Math,* 1988, vol. 48, 1073
- **SMITH et al.** *J. Clin. Pathol,* 2004, vol. 57, 912-917
- **NELSON et al.** *J Clin Pathol,* 2000, vol. 53, 111-117
- **RICH ; MYSZKA.** *Curr. Opin. Biotechnol,* 2000, vol. 11, 54
- **ENGLEBIENNE.** *Analyst,* 1998, vol. 123, 1599
- **MALMQVIST.** *Biochem.Soc.Trans,* 2000, vol. 27, 335
- **BRUMMELL et al.** *Biochem,* 1993, vol. 32, 1180-1187
- **KOBAYASHI et al.** *Protein Eng,* 1999, vol. 12 (10), 879-884
- **BURKS et al.** *Proc. Natl. Acad. Sci. USA,* 1997, vol. 94, 412-417
- **HARTLEY J.A et al.** *Cancer Res,* 2010, vol. 70 (17), 6849-6858
- **ANTONOW D et al.** *Cancer J,* 2008, vol. 14 (3), 154-169
- **HOWARD P.W et al.** *Bioorg Med Chem Lett,* 2009, vol. 19, 6463-6466
- **SAGNOU et al.** *Bioorg Med Chem Lett,* 2000, vol. 10 (18), 2083-2086
- Remington's Pharmaceutical Sciences. Mack Publishing Company, 1975
- ELISA: Theory and Practice: Methods in Molecular Biology. Human Press, 1995, vol. 42
- **E. DIAMANDIS ; T. CHRISTOPOULUS.** Immunoassay. Academic Press, Inc, 1996
- **P. TIJSSEN.** Practice and Theory of Enzyme Immunoassays. Elsevier Science Publishers, 1985
- Remington's Pharmaceutical Sciences: The Science And Practice Of Pharmacy. Mack Pub. Co, 1995
- Drug Absorption Enhancement: Concepts, Possibilities, Limitations, And Trends. Harwood Academic Publishers, 1994
- Peptide And Protein Drug Delivery (Advances In Parenteral Sciences. M. Dekker, 1991, vol. 4
- **MARASCO et al.** *Proc. Natl. Acad. Sci. USA,* 1993, vol. 90, 7889-7893
- *EMBO J,* 1985, vol. 4 (2), 337-344
- *J. Immunol,* 1986, vol. 137 (4), 1097-100
- *J.Exp.Med.,* 1991, vol. 174, 319-326
- *J. Immunol.,* 1991, vol. 147 (9), 3047-52